# EUROPEAN PATENT APPLICATION

(11) **EP 4 596 535 A1**
(43) Date of publication of application: **06.08.2025**
(21) Application number: 23869706.4
(22) Date of filing: 06.06.2023
(51) Int. Cl.: C07D 221/18, C07D 401/14, A61K 31/435, A61K 47/55, A61P 35/00

(54) **DRUG FOR TREATING HUMAN TUMOR BY ERF3A TARGETED PROTEIN DEGRADATION MECHANISM**

(30) Priority: 30.09.2022 CN 202211216359
(71) Applicant: Suzhou Degen Bio-Medical Co, Ltd., Suzhou, Jiangsu 215000 (CN)
(72) Inventor: YU, Guangliang, Suzhou, Jiangsu 215000 (CN); ZHONG, Kai, Suzhou, Jiangsu 215000 (CN); CHEN, Yao, Suzhou, Jiangsu 215000 (CN); HAN, Liuquan, Suzhou, Jiangsu 215000 (CN); TIAN, Jiahui, Suzhou, Jiangsu 215000 (CN); ZHANG, Changzhou, Suzhou, Jiangsu 215000 (CN); HU, Zhenxing, Suzhou, Jiangsu 215000 (CN); XU, Ping, Suzhou, Jiangsu 215000 (CN); MAO, Shujun, Suzhou, Jiangsu 215000 (CN)
(74) Representative: RGTH
(86) International application number: PCT/CN2023/098569
(87) International publication number: WO 2024/066454

(57) **Abstract**

The present disclosure provides a proteolysis-targeting compound TPB-L-E3B, a method for synthesizing the same, and use thereof. The compound can treat human tumor diseases through the eRF3a-targeting proteolysis mechanism, and exhibits great potential in treating such diseases in *in-vitro* studies, particularly, in treating diseases such as prostate cancer, ovarian cancer, liver cancer, cervical cancer, leukemia, breast cancer, and the like.

## Description

### FIELD OF THE INVENTION

The present disclosure relates to the field of pharmaceutical chemistry, and in particular, to a proteolysis-targeting compound, a method for synthesizing the same, and use thereof in preparing a medicament.

### DESCRIPTION OF THE RELATED ART

Proteolysis-targeting chimeras (PROTACs) are bifunctional molecules that use the cell's own ubiquitin-proteasome system to label a target protein and facilitate the degradation of same. They generally consist of an E3 ligase (i.e., E3 ubiquitin ligase)-binding moiety, a linker, and a target protein-binding moiety. Over 20 years of PROTAC development has promoted a number of pharmaceuticals into the clinical stage, which have exhibited certain clinical advantages.

Molecular glues (MGs) are a class of small molecules that can induce the interaction between E3 ligase and the target protein, thereby helping degrade the target protein through the ubiquitin-proteasome system of the cells. Representative drugs are, for example, thalidomide, pomalidomide, lenalidomide, etc.

Although both PROTAC and MG rely on the ubiquitin-proteasome system to degrade target proteins, there are some differences between the two mechanisms. The target protein degradation ability of the PROTAC molecules depends more on the binding ability of the molecule itself to E3 ligase and the target protein, while the target protein degradation ability of MG molecules depends more on the binding ability of the allosteric E3 ligase to target protein after the MG molecule is added. The PRORAC molecules generally contain a linear or rigid loop structure with a greater number of atoms as the linker, while MGs contain a linker with fewer atoms or even no linking moiety. PROTACs target a variety of proteins, which is related to their specific mechanism, while MGs have a narrow spectrum of target proteins, of which commonly known ones are Ikaros, Aiolos, eRF3a (GSPT1), CK1α, and the like.

GSPT1 (G1 to S phase transition 1) gene is located in region 13 of the long arm of human chromosome 16. It has 7141 base pairs in the genome and consists of 15 exons, and the protein-coding region (bp 237-2150) codes 637 amino acids. GSPT1 (eukaryotic peptide chain release factor 3, eRF3a), as a key member of peptide chain release factor, is involved in a variety of important biological processes, such as the termination of protein translation, apoptosis, cell cycle regulation, cytoskeleton assembly, tumorigenesis, etc. Many studies have revealed that the eRF3a is closely related to the development and progression of various human tumors. Currently, there're targeted eRF3a therapies in the clinical stage, but none of them are approved. More and more studies have shown that targeted degradation of eRF3a has a good therapeutic effect on many human tumors.

Steroid 5α-reductases (SRD5As) are one of the important enzyme systems in the human body, including three isozymes, SRD5A1, SRD5A2, and SRD5A3, which are reduced coenzyme II (NADPH)-dependent membrane proteases located on endoplasmic reticulum and widely distributed in various tissues of the human body at different expression levels. SRD5A1 and SRD5A2 primarily serve to reduce specific steroid substrates, such as testosterone to dihydrotestosterone. SRD5A3 not only reduces specific steroid substrates, but also plays an important role in the N-glycosylation modification of certain proteins.

Various articles reported that the steroid 5α-reductase is abnormally overexpressed or has high activity in many diseases in human, in particular human tumor diseases, such as breast cancer, testicular cancer, ovarian cancer, thyroid cancer, lung cancer, esophageal cancer, liver cancer, prostate cancer, and the like. Many researchers believe that SRD5A1, SRD5A2, and SRD5A3 may serve as potential biomarkers of some tumors and are related to the progression and prognosis of the tumors. Thus, the direct or indirect targeted inhibition or downregulation of the biofunctionality of steroid 5α-reductase may be a potential mechanism for the treatment of tumors.

Finasteride and dutasteride, known as steroid 5α-reductase inhibitors, have been clinically used as inhibitors of specific SRD5A2 in treating human diseases with abnormal SRD5A2 expression such as prostatic hyperplasia, androgen-dependent alopecia, etc., but there are no effective treatments in inhibiting the biofunctionality of SRD5A1 and SRD5A3 to treating human diseases with abnormal SRD5A1 or SRD5A3 expression at present.

Prostate cancer is one of the most common tumors in the male urinary system. Androgen receptor (AR) plays an important role in the development and progression of prostate cancer, and can regulate the expression of downstream target genes after being activated by binding to androgen, thereby promoting the progression and metastasis of prostate cancer. Androgen deprivation therapy (ADT) is the primary treatment for advanced prostate cancer, but in most patients, the disease may develop resistance and progress to castration-resistant prostate cancer (CRPC) after 18 to 24 months of treatment. In the CRPC stage, the AR signaling pathway still plays a crucial role in CRPC despite the suppressed androgen levels. Currently, various studies have demonstrated that the drug resistance mechanism against abiraterone and enzalutamide is mainly caused by AR gene variation, including AR amplifications, AR mutations, AR variants (AR-Vs), and the like. AR-Vs are truncated variants of AR, and more than 20 AR-Vs have been reported, of which AR-V7 is one of the most extensively studied splice variants among CRPCs, with the highest frequency of detection. Recent studies have shown that AR-V7 plays an important role in the CRPC and drug resistance development processes and can be used as a molecular marker for guiding treatment options of CRPC patients.

The present disclosure provides a class of compounds that, as an MG to degrade eRF3a, show great potential in treating various human tumors such as prostate cancer, ovarian cancer, liver cancer, breast cancer, pancreatic cancer, cerebral glioma, cervical cancer, leukemia, gastric cancer, and colon cancer, in particular, most human prostate cancers, in *in-vitro* studies. The compounds have the activity of inhibiting SRD5A1 and SRD5A3 in a part of prostate cancers, or have the activity of degrading eRF3a while reducing the expression of SRD5A1 and SRD5A3 proteins, and can influence the expression of some proteins (such as AR, AR-V7, etc.) on which the survival and proliferation of tumor cells depend, thereby achieving the treatment of human prostate cancer.

### SUMMARY OF THE INVENTION

The present disclosure provides a pharmaceutical proteolysis-targeting compound, including a composition comprising the compound, a method for preparing the same, and use thereof.

The present disclosure is intended to provide a compound having the following structure: TPB-L-E3B, wherein TPB is a target protein-binding moiety, L is a linker, and E3B is an E3 ligase-binding moiety.

Specifically, in compound TPB-L-E3B, TPB is shown in structure (I): 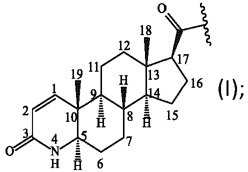
L is selected from one of structures (IIA) and (IIB): 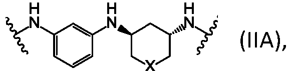 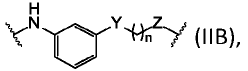 X is CH₂, O, NH, or CF₂, n is an integer of 2-3, Y is NH or O, and Z is NH or O;
E3B is selected from one of structures (IIIA), (IIIB), and (IIIC): 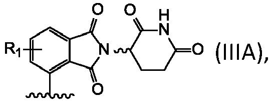 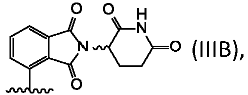 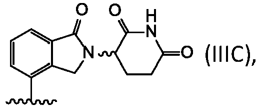 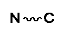 denotes that C may have R configuration, S configuration, or a mixture of R and S configurations, and R₁ denotes that the H at any one of the three remaining positions of the benzene ring is substituted by F.

Preferably, L is selected from one of structures (IIA) and (IIB): 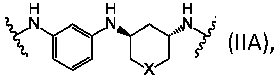 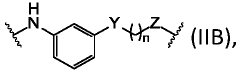 X is CH₂, O, NH, or CF₂, n is an integer of 2-3, Y is NH or O, and Z is NH;
E3B is selected from one of structures (IIIB) and (IIIC): 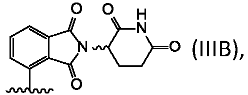 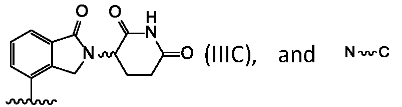 denotes that C may have R configuration, S configuration, or a mixture of R and S configurations.

More preferably, L is selected from one of structures (IIA) and (IIB): 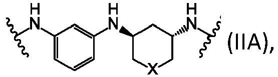 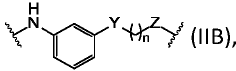 X is CH₂, n is 3, Y is NH, and Z is NH;

E3B is shown in structure (IIIB): 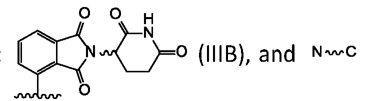 denotes that C may have R configuration, S configuration, or a mixture of R and S configurations.

Furthermore, preferred compounds of the present disclosure include, but are not limited to, the following structures: 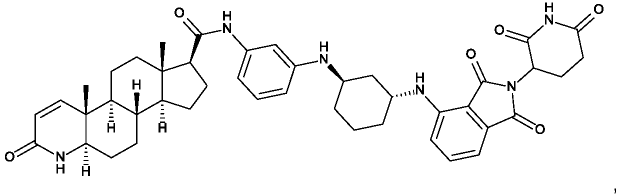 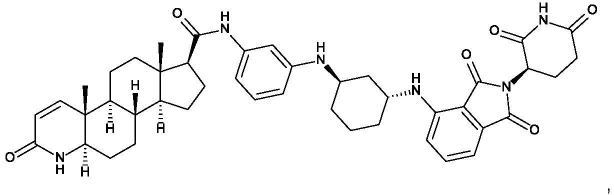 and

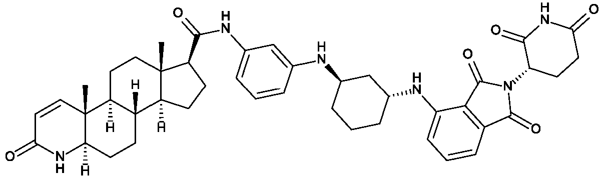

The present disclosure is also intended to provide a method for synthesizing compound TPB-L-E3B, and the compound related to the present disclosure may be synthesized by the following methods:
1. When Z is NH,
   (1) E3B is 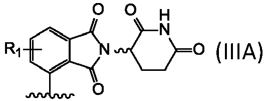 or 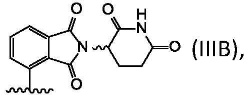 and the compound TPB-L-E3B can be represented by structure CA: 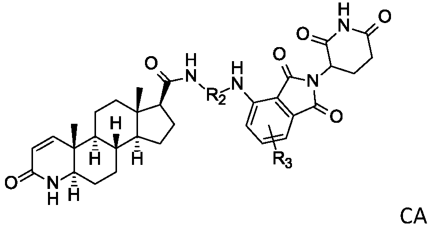 wherein R₂ represents 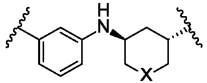 and 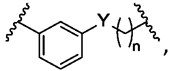 X is CH₂, O, NH, or CF₂, n is an integer of 2-3, Y is NH or O, R₃ is hydrogen or R₁, and R₁ denotes that the H at any one of the three remaining positions of the benzene ring is substituted by F; in this case, the compound TPB-L-E3B can be synthesized by method 1.

### Method 1:

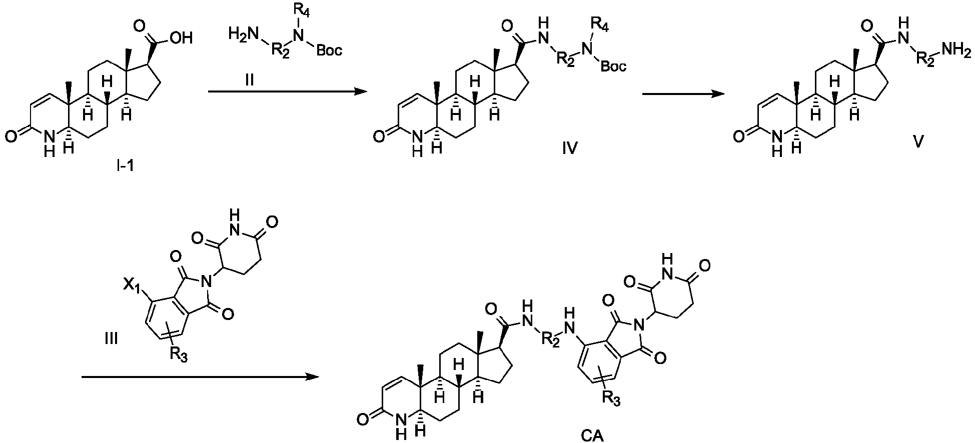

wherein R₂ represents 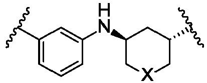 and 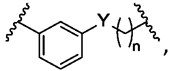 X is CH₂, O, NH, or CF₂, n is an integer of 2-3, Y is NH or O, R₃ is hydrogen or R₁, R₁ denotes that the H at any one of the three remaining positions of the benzene ring is substituted by F, R₄ represents H or Boc group, and X₁ is a halogen.

(2) E3B is 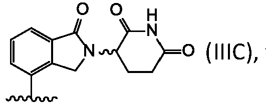 the compound TPB-L-E3B can be represented by structure CA1: 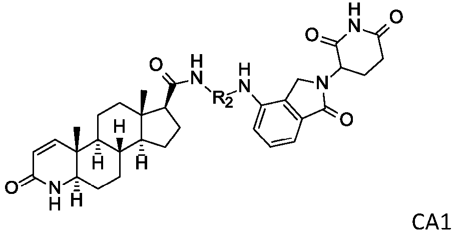 wherein R₂ represents 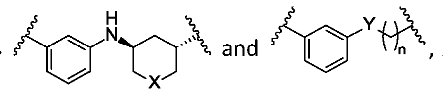 X is CH₂, O, NH, or CF₂, n is an integer of 2-3, and Y is NH or O; in this case, the compound TPB-L-E3B can be synthesized by method 2.

### Method 2:

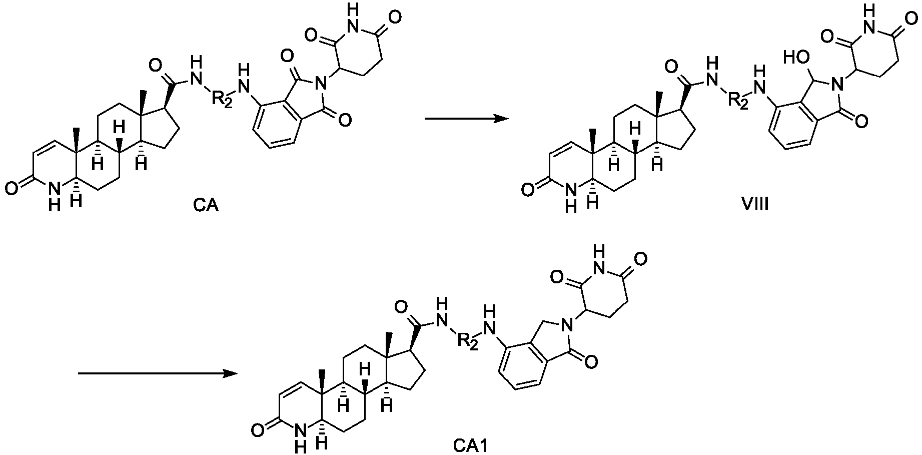

**wherein** R₂ represents 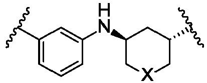 and 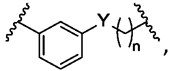 X is CH₂, O, NH, or CF₂, n is an integer of 2-3, and Y is NH or O.

2. When Z is O,
(1) E3B is 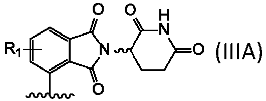 or 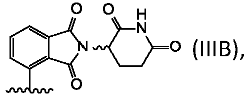 and the compound TPB-L-E3B can be represented by structure CA2: 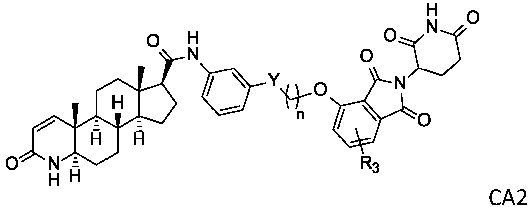 wherein Y is NH or O, n is an integer of 2-3, R₃ is hydrogen or R₁, and R₁ denotes that the H at any one of the three remaining positions of the benzene ring is substituted by F; in this case, the compound TPB-L-E3B can be synthesized by method 3.

### Method 3:

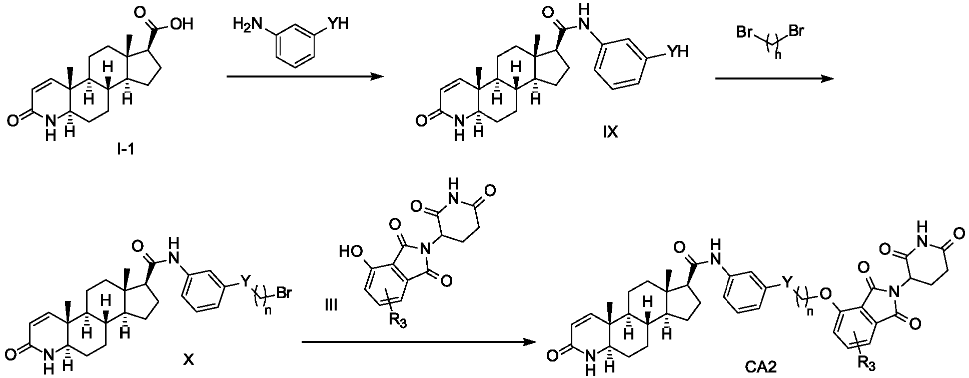

wherein Y is NH or O, n is an integer of 2-3, R₃ is hydrogen or R₁, and R₁ denotes that the H at any one of the three remaining positions of the benzene ring is substituted by F.

(2) E3B is 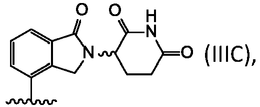 the compound TPB-L-E3B can be represented by structure CA3: 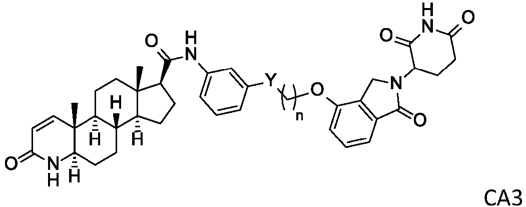 wherein Y is NH or O, and n is an integer of 2-3; in this case, the compound TPB-L-E3B can be synthesized by method 4.

### Method 4:

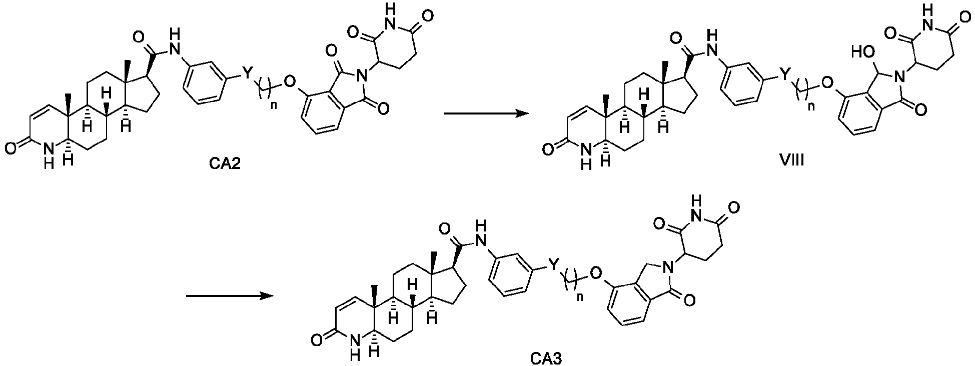

Y is NH or O, and n is an integer of 2-3.

In another aspect, the present disclosure provides use of compound TPB-L-E3B in preparing a medicament for treating a tumor. The medicament comprising TPB-L-E3B can treat human tumor diseases through the eRF3a-targeting proteolysis mechanism. The tumor includes, but is not limited to: prostate cancer, ovarian cancer, liver cancer, esophageal squamous cancer, breast cancer, pancreatic cancer, cervical cancer, lung cancer, leukemia, gastric cancer, colon cancer, or the like; preferably, the tumor is prostate cancer, ovarian cancer, liver cancer, breast cancer, pancreatic cancer, cervical cancer or lung cancer; more preferably, the tumor is prostate cancer.

### BRIEF DESCRIPTION OF THE DRAWINGS

FIG. 1 shows the body weight of mice receiving C29 intraperitoneal (IP) injection;
FIG. 2 shows the tumor volume in mice receiving C29 intraperitoneal (IP) injection;
FIG. 3 shows the body weight of mice receiving C59 intraperitoneal (IP) or oral gavage (PO) ;
FIG. 4 shows the tumor volume in mice receiving C59 intraperitoneal (IP) or oral gavage (PO) ;
FIG. 5 shows the body weight of mice receiving C63 oral gavage (PO) ;
FIG. 6 shows the tumor volume in mice receiving C63 oral gavage (PO) ;
FIG. 7 is a WB pattern illustrating the downregulation of related proteins in 22Rv1 cells at 24 h by C59 at different concentrations;
FIG. 8 is a WB pattern illustrating the downregulation of related proteins in 22Rv1 cells at different time points by C59 at 3 nM;
FIG. 9 is a WB pattern illustrating the downregulation of related proteins in PC-3 cells at 24 h by C59 at different concentrations; and
FIG. 10 is a WB pattern illustrating the downregulation of related proteins in HepG2 cells at 24 h by C59 at different concentrations.

### DETAILED DESCRIPTION OF THE PREFERRED EMBODIMENTS

The present disclosure will be further illustrated by the following specific examples. It will be appreciated that the following examples are illustrative of the present disclosure only and are not intended to limit the present disclosure in any way.

The starting materials used in the present disclosure are commercially available, or may be synthesized by methods known in the art or according to the methods described herein.

The abbreviations used in the present disclosure are listed as follows:
Boc: *tert*-butyloxycarbonyl
pyBoP: benzotriazol-1-yl-oxytripyrrolidino-phosphonium hexafluorophosphate
DIPEA: *N,N*-diisopropylethylamine
DMF: *N,N*-dimethylformamide
STAB: sodium triacetoxyborohydride
DME: 1,2-dichloroethane
XPhoS: 2-dicyclohexylphosphino-2',4',6'-triisopropylbiphenyl
Cbz: benzyloxycarbonyl
V/V: volume ratio
SPF: specific pathogen free
IVC system: individually ventilated cage system
CCK8: cell viability detection reagent
L-Glutamine: L-glutamine
PBS buffer: phosphate-buffered saline
RIPA lysis buffer: radio immunoprecipitation assay lysis buffer
PVDF membrane: polyvinylidene fluoride membrane
TBST solution: tris(hydroxymethyl)aminomethane-hydrochloride buffered saline containing 2% Tween

Abbreviations that are used herein but not included in the above list have the meaning generally accepted by those skilled in the art.

### Example 1: Synthesis of N-(2-((4-(2-(3-(2,6-dioxopiperidine))-isoindole-1,3-dione))amino)ethyl)-3-oxo-4-aza-5α-androst-1-ene-17β-carboxamide (C1)

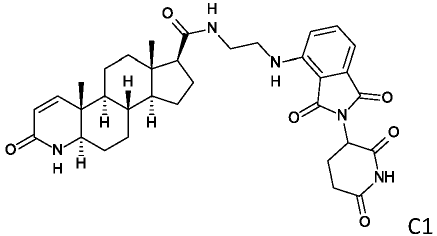

### Step 1: Synthesis of N-(2-(Boc-amino)ethyl)-3-oxo-4-aza-5α-androst-1-ene-17β-carboxamide (IV-1)

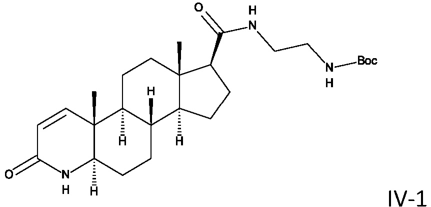

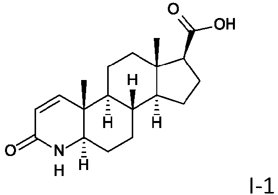

At room temperature, 1.00 g of 3-oxo-4-aza-5α-androst-1-ene-17β-carboxylic acid (I-1), 0.74 g of N-Boc-ethylenediamine hydrochloride, 2.00 g of pyBoP, and 20 mL of DMF were sequentially added into a 100-mL three-necked flask, and the mixture was stirred for 5 min. 1.22 g of DIPEA was added dropwise to the three-necked flask, and the reaction system was incubated overnight. The reaction was quenched with 50 mL of water to give a reaction solution. The reaction solution was extracted with ethyl acetate (50 mL × 3). The organic phases were combined, sequentially washed with 50 mL of 1 mol/L aqueous hydrochloric acid, 50 mL of saturated aqueous sodium carbonate, and 50 mL of saturated aqueous sodium chloride, dried over anhydrous sodium sulfate, and concentrated to give a black oily substance (2.56 g). The oily substance was purified by column chromatography (eluent: dichloromethane:methanol = 20:1) to give an off-white solid (compound IV-1, 0.70 g).

Step 2: Synthesis of *N*-(2-aminoethyl)-3-oxo-4-aza-5α-androst-1-ene-17β-carboxamide (V-1)

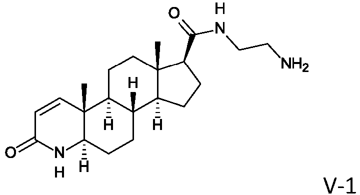

At room temperature, 0.70 g of compound IV-1 and 10 mL of dichloromethane were sequentially added into a 50 mL three-necked flask, before 10 mL of trifluoroacetic acid was dropwise added. The reaction system was incubated for 10 min. The reaction solution was concentrated to give an oily substance (crude compound V-1), which was directly used in the next reaction without purification.

### Step 3: Synthesis of N-(2-((4-(2-(3-(2,6-dioxopiperidine))-isoindole-1,3-dione))amino)ethyl)-3-oxo-4-aza-5α-androst-1-ene-17β-carboxamide (C1)

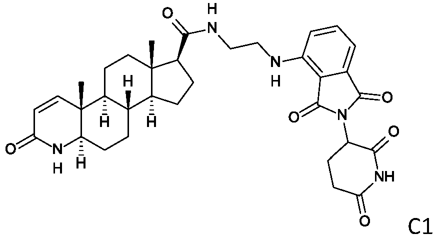

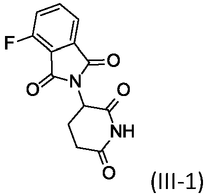

At room temperature, the oily substance obtained in step 2 (crude compound V-1), 0.15 g of 4-fluoro-2-(3-(2,6-dioxopiperidine))-isoindole-1,3-dione (III-1), 0.20 g of DIPEA, and 5 mL of DMF were sequentially added into a reaction flask. The reaction flask was treated in a microwave reactor for 10 min of reaction at 50 Hz. 20 mL of water was added into the reaction flask, and the mixture was extracted with dichloromethane (20 mL × 3). The organic phases were combined, washed with 20 mL of water, dried over anhydrous sodium sulfate, and concentrated to give an oily substance. The oily substance was purified by column chromatography (eluent: dichloromethane:methanol = 10:1) to give compound C1 (40 mg).

The mass spectral data for compound C1 are, MS (ESI): (M+H)⁺, 616.3

The nuclear magnetic resonance data for compound C1 are as follows:
H NMR (400 MHz, CDCl₃) δ: 8.45-8.47 (m, 1H), 7.48-7.50 (m, 1H), 7.00-7.03 (m, 1H), 6.78-6.81 (m, 1H), 6.62-6.65 (m, 1H), 5.73-5.76 (m, 2H), 5.57-5.61 (m, 1H), 4.89-4.92 (m, 1H), 3.10-3.22 (m, 5H), 2.65-2.80 (m, 3H), 2.01-2.15 (m, 2H), 1.5-1.73 (m, 7H), 1.15-1.47 (m, 6H), 0.99-1.05 (m, 2H), 0.93 (s, 3H), 0.70 (s, 3H)

### Example 2: Synthesis of N-(2-((5-(2-(3-(2,6-dioxopiperidine))-isoindole-1,3-dione))amino)ethyl)-3-oxo-4-aza-5α-androst-1-ene-17β-carboxamide (C2)

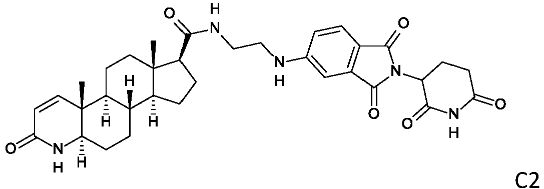

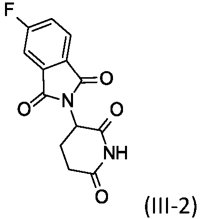

The synthesis was similar to that in Example 1, except that 4-fluoro-2-(3-(2,6-dioxopiperidine))-isoindole-1,3-dione in step 3 of Example 1 was replaced with 5-fluoro-2-(3-(2,6-dioxopiperidine))-isoindole-1,3-dione (compound III-2).

The mass spectral data for compound C2 are, MS (ESI): (M+H)⁺, 616.3

The nuclear magnetic resonance data for compound C2 are as follows:
H NMR (400 MHz, CDCl₃) δ: 8.85 (s, 1H), 7.45-7.48 (m, 1H), 6.91 (s, 1H), 6.69-6.71 (m, 2H), 5.73-5.77 (m, 2H), 5.41-5.45 (m, 1H), 4.92-4.97 (m, 1H), 3.08-3.27 (m, 5H), 2.65-2.75 (m, 3H), 2.01-2.15 (m, 2H), 1.58-1.93 (m, 7H), 1.15-1.47 (m, 6H), 0.95-1.01 (m, 2H), 0.94 (s, 3H), 0.71 (s, 3H)

### Example 3: Synthesis of N-(3-((4-(2-(3-(2,6-dioxopiperidine))-isoindole-1,3-dione))amino)propyl)-3-oxo-4-aza-5α-androst-1-ene-17β-carboxamide (C3)

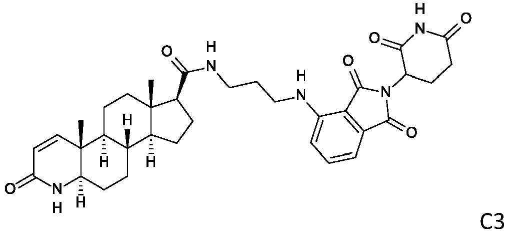

The synthesis was similar to that in Example 1, except that *N*-Boc-ethylenediamine hydrochloride in step 1 of Example 1 was replaced with *N*-Boc-1,3-propanediamine hydrochloride.
The mass spectral data for compound C3 are, MS (ESI): (M+H)⁺, 630.3

The nuclear magnetic resonance data for compound C3 are as follows:
H NMR (400 MHz, CDCl₃) δ: 8.47-8.49 (m, 1H), 7.46-7.48 (m, 1H), 7.01-7.03 (m, 1H), 6.78-6.83 (m, 1H), 6.62-6.65 (m, 1H), 5.72-5.76 (m, 2H), 5.57-5.60 (m, 1H), 4.89-4.92 (m, 1H), 3.10-3.22 (m, 5H), 2.65-2.80 (m, 3H), 2.01-2.15 (m, 2H), 1.73-1.98 (m, 9H), 1.15-1.47 (m, 6H), 0.99-1.05 (m, 2H), 0.92 (s, 3H), 0.68 (s, 3H)

### Example 4: Synthesis of N-(3-((5-(2-(3-(2,6-dioxopiperidine))-isoindole-1,3-dione))amino)propyl)-3-oxo-4-aza-5α-androst-1-ene-17β-carboxamide (C4)

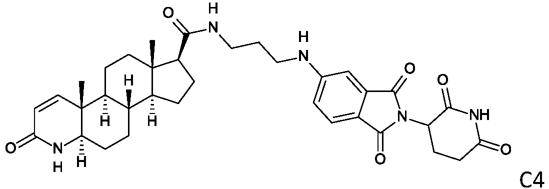

The synthesis was similar to that in Example 1, except that *N*-Boc-ethylenediamine hydrochloride in step 1 of Example 1 was replaced with *N*-Boc-1,3-propanediamine hydrochloride, and 4-fluoro-2-(3-(2,6-dioxopiperidine))-isoindole-1,3-dione in step 3 was replaced with 5-fluoro-2-(3-(2,6-dioxopiperidine))-isoindole-1,3-dione.
The mass spectral data for compound C4 are, MS (ESI): (M+H)⁺, 630.3

The nuclear magnetic resonance data for compound C4 are as follows:
H NMR (400 MHz, CDCl₃) δ: 8.65-8.68 (m, 1H), 7.42-7.46 (m, 1H), 6.88 (s, 1H), 6.65-6.68 (m, 2H), 5.73-5.77 (m, 2H), 5.39-5.42 (m, 1H), 4.85-4.90 (m, 1H), 3.25-3.27 (m, 2H), 3.10-3.15 (m, 3H), 2.63-2.75 (m, 3H), 1.91-2.10 (m, 5H), 1.89-1.73 (m, 6H), 1.15-1.47 (m, 6H), 0.95-1.01 (m, 2H), 0.91 (s, 3H), 0.68 (s, 3H)

### Example 5: Synthesis of N-(4-((4-(2-(3-(2,6-dioxopiperidine))-isoindole-1,3-dione))amino)butyl)-3-oxo-4-aza-5α-androst-1-ene-17β-carboxamide (C5)

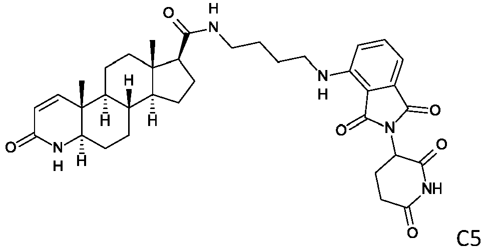

The synthesis was similar to that in Example 1, except that *N*-Boc-ethylenediamine hydrochloride in step 1 of Example 1 was replaced with *N*-Boc-1,4-butanediamine hydrochloride.
The mass spectral data for compound C5 are, MS (ESI): (M+H)⁺, 644.3

The nuclear magnetic resonance data for compound C5 are as follows:
H NMR (400 MHz, CDCl₃) δ: 8.77-8.79 (m, 1H), 7.42-7.45 (m, 1H), 7.02-7.06 (m, 1H), 6.77-6.83 (m, 1H), 6.62-6.68 (m, 1H), 5.72-5.76 (m, 2H), 5.58-5.60 (m, 1H), 4.89-4.92 (m, 1H), 3.11-3.28 (m, 5H), 2.64-2.80 (m, 3H), 2.01-2.15 (m, 3H), 1.89-1.97 (m, 2H), 1.51-1.73 (m, 7H), 1.15-1.47 (m, 7H), 0.93-1.08 (m, 2H), 0.90 (s, 3H), 0.65 (s, 3H)

### Example 6: Synthesis of N-(4-((5-(2-(3-(2,6-dioxopiperidine))-isoindole-1,3-dione))amino)butyl)-3-oxo-4-aza-5α-androst-1-ene-17β-carboxamide (C6)

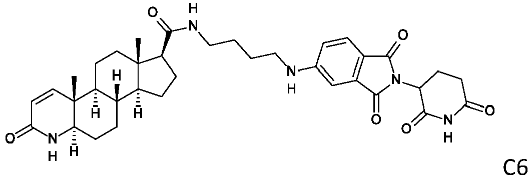

The synthesis was similar to that in Example 1, except that *N*-Boc-ethylenediamine hydrochloride in step 1 of Example 1 was replaced with *N*-Boc-1,4-butanediamine hydrochloride, and 4-fluoro-2-(3-(2,6-dioxopiperidine))-isoindole-1,3-dione in step 3 was replaced with 5-fluoro-2-(3-(2,6-dioxopiperidine))-isoindole-1,3-dione.
The mass spectral data for compound C6 are, MS (ESI): (M+H)⁺, 644.3

The nuclear magnetic resonance data for compound C6 are as follows:
H NMR (400 MHz, CDCl₃) δ: 8.71-8.74 (m, 1H), 7.42-7.48 (m, 1H), 6.85 (s, 1H), 6.62-6.68 (m, 2H), 5.73-5.76 (m, 2H), 5.38-5.42 (m, 1H), 4.83-4.85 (m, 1H), 3.24-3.27 (m, 2H), 3.10-3.15 (m, 3H), 2.63-2.79 (m, 3H), 2.05-2.17 (m, 3H), 1.89-1.97 (m, 2H), 1.17-1.75 (m, 14H), 0.95-1.01 (m, 2H), 0.91 (s, 3H), 0.68 (s, 3H)

### Example 7: Synthesis of N-(5-((4-(2-(3-(2,6-dioxopiperidine))-isoindole-1,3-dione))amino)pentyl)-3-oxo-4-aza-5α-androst-1-ene-17β-carboxamide (C7)

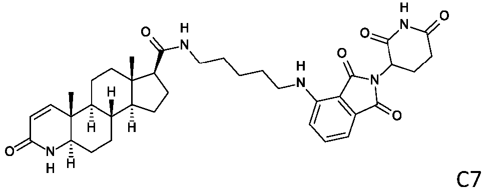

The synthesis was similar to that in Example 1, except that *N*-Boc-ethylenediamine hydrochloride in step 1 of Example 1 was replaced with *N*-Boc-1,5-pentanediamine hydrochloride.
The mass spectral data for compound C7 are, MS (ESI): (M+H)⁺, 658.4

The nuclear magnetic resonance data for compound C7 are as follows:

H NMR (400 MHz, CDCl₃) δ: 8.60-8.62 (m, 1H), 7.44-7.48 (m, 1H), 7.03-7.06 (m, 1H), 6.77-6.82 (m, 1H), 6.62-6.68 (m, 1H), 5.71-5.76 (m, 2H), 5.54-5.56 (m, 1H), 4.85-4.88 (m, 1H), 3.29-3.31 (m, 2H), 3.11-3.16 (m, 3H), 2.64-2.80 (m, 3H), 2.01-2.15 (m, 5H), 1.89-1.97 (m, 2H), 1.51-1.73 (m, 5H), 1.15-1.47 (m, 9H), 0.93-1.08 (m, 2H), 0.90 (s, 3H), 0.65 (s, 3H)

### Example 8: Synthesis of N-(5-((5-(2-(3-(2,6-dioxopiperidine))-isoindole-1,3-dione))amino)pentyl)-3-oxo-4-aza-5α-androst-1-ene-17β-carboxamide (C8)

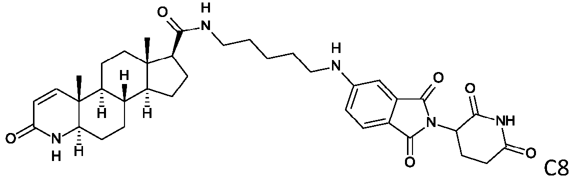

The synthesis was similar to that in Example 1, except that *N*-Boc-ethylenediamine hydrochloride in step 1 of Example 1 was replaced with *N*-Boc-1,5-pentanediamine hydrochloride, and 4-fluoro-2-(3-(2,6-dioxopiperidine))-isoindole-1,3-dione in step 3 was replaced with 5-fluoro-2-(3-(2,6-dioxopiperidine))-isoindole-1,3-dione.
The mass spectral data for compound C8 are, MS (ESI): (M+H)⁺, 658.4

The nuclear magnetic resonance data for compound C8 are as follows:
H NMR (400 MHz, CDCl₃) δ: 8.65-8.67 (m, 1H), 7.46-7.49 (m, 1H), 6.84 (s, 1H), 6.62-6.68 (m, 2H), 5.71-5.76 (m, 2H), 5.38-5.43 (m, 1H), 4.81-4.85 (m, 1H), 3.24-3.27 (m, 2H), 3.10-3.15 (m, 3H), 2.63-2.79 (m, 3H), 2.02-2.17 (m, 5H), 1.89-1.97 (m, 2H), 1.51-1.75 (m, 5H), 1.17-1.47 (m, 9H), 0.93-1.08 (m, 2H), 0.90 (s, 3H), 0.67 (s, 3H)

### Example 9: Synthesis of N-(6-((4-(2-(3-(2,6-dioxopiperidine))-isoindole-1,3-dione))amino)hexyl)-3-oxo-4-aza-5α-androst-1-ene-17β-carboxamide (C9)

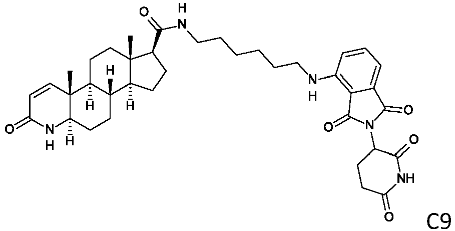

The synthesis was similar to that in Example 1, except that *N*-Boc-ethylenediamine hydrochloride in step 1 of Example 1 was replaced with *N*-Boc-1,6-hexanediamine hydrochloride.
The mass spectral data for compound C9 are, MS (ESI): (M+H)⁺, 672.4

The nuclear magnetic resonance data for compound C9 are as follows:
H NMR (400 MHz, CDCl₃) δ: 8.56-8.57 (m, 1H), 7.42-7.48 (m, 1H), 7.02-7.05 (m, 1H), 6.75-6.82 (m, 1H), 6.62-6.68 (m, 1H), 5.70-5.76 (m, 2H), 5.54-5.56 (m, 1H), 4.88-4.91 (m, 1H), 3.28-3.33 (m, 2H), 3.11-3.17 (m, 3H), 2.64-2.81 (m, 3H), 2.05-2.17 (m, 5H), 1.89-1.97 (m, 2H), 1.51-1.73 (m, 5H), 1.15-1.47 (m, 10H), 0.93-1.08 (m, 3H), 0.89 (s, 3H), 0.65 (s, 3H)

### Example 10: Synthesis of N-(6-((5-(2-(3-(2,6-dioxopiperidine))-isoindole-1,3-dione))amino)hexyl)-3-oxo-4-aza-5α-androst-1-ene-17β-carboxamide (C10)

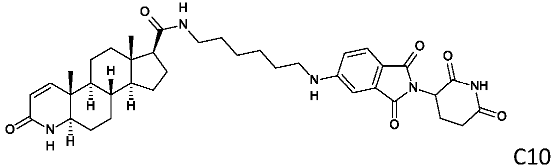

### Step 1: Synthesis of N-(6-(Boc-amino)hexyl)-3-oxo-4-aza-5α-androst-1-ene-17β-carboxamide (IV-10)

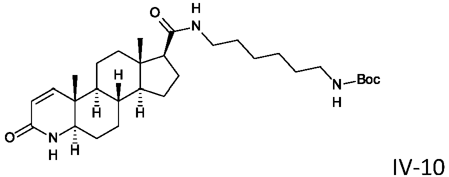

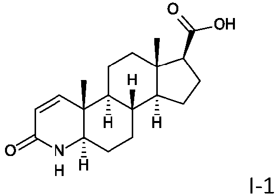

At room temperature, 2.00 g of compound I-1, 1.92 g of *N*-Boc-1,6-hexanediamine hydrochloride, 4.00 g of pyBoP, and 30 mL of DMF were sequentially added into a reaction flask, and the mixture was stirred for 5 min. 2.44 g of DIPEA was added dropwise into the three-necked flask, and the reaction system was incubated overnight. The next day, 70 mL of water was added to give a reaction solution. The reaction solution was extracted with ethyl acetate (70 mL × 3). The organic phases were combined, sequentially washed with 70 mL of 1 mol/L aqueous hydrochloric acid, 70 mL of saturated aqueous sodium carbonate, and 70 mL of saturated aqueous sodium chloride, dried over anhydrous sodium sulfate, and concentrated to give a black oily substance (5.29 g). The oily substance was purified by column chromatography (eluent: dichloromethane:methanol = 20:1) to give compound IV-10 (1.48 g).

Step 2: Synthesis of *N*-(6-aminohexyl)-3-oxo-4-aza-5α-androst-1-ene-17β-carboxamide (V-10)

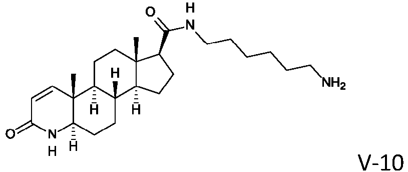

At room temperature, 1.40 g of compound IV-10 and 15 mL of dichloromethane were sequentially added into a reaction flask, before 15 mL of trifluoroacetic acid was dropwise added. The reaction system was incubated for 10 min. The reaction solution was concentrated to give an oily substance (crude compound V-10), which was directly used in the next reaction without purification.

### Step 3: Synthesis of N-(6-((5-(2-(3-(2,6-dioxopiperidine))-isoindole-1,3-dione))amino)hexyl)-3-oxo-4-aza-5α-androst-1-ene-17β-carboxamide (C10)

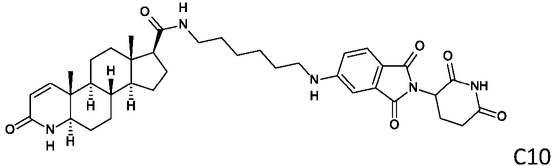

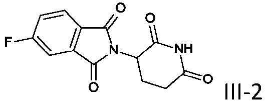

At room temperature, the oily substance obtained in step 2 (crude compound V-10), 0.66 g of compound 5-fluoro-2-(3-(2,6-dioxopiperidine))-isoindole-1,3-dione (III-2), 0.86 g of DIPEA, and 20 mL of DMF were sequentially added into a reaction flask. The reaction flask was treated in a microwave reactor for 10 min of reaction at 50 Hz. 30 mL of water was added into the reaction flask, and the mixture was extracted with dichloromethane (30 mL × 3). The organic phases were combined, washed with 30 mL of water, dried over anhydrous sodium sulfate, and concentrated to give an oily substance. The oily substance was purified by column chromatography (eluent: dichloromethane:methanol = 10:1) to give compound C10 (0.46 g).
The mass spectral data for compound C10 are, MS (ESI): (M+H)⁺, 672.4

The nuclear magnetic resonance data for compound C10 are as follows:
H NMR (400 MHz, CDCl₃) δ: 8.66-8.67 (m, 1H), 7.48-7.50 (m, 1H), 6.84 (s, 1H), 6.62-6.68 (m, 2H), 5.70-5.76 (m, 2H), 5.38-5.41 (m, 1H), 4.82-4.86 (m, 1H), 3.24-3.28 (m, 2H), 3.11-3.18 (m, 3H), 2.63-2.79 (m, 3H), 2.01-2.17 (m, 5H), 1.89-1.97 (m, 2H), 1.51-1.70 (m, 5H), 1.20-1.47 (m, 10H), 0.93-1.08 (m, 3H), 0.88 (s, 3H), 0.64 (s, 3H)

### Example 11: Synthesis of N-(7-((4-(2-(3-(2,6-dioxopiperidine))-isoindole-1,3-dione))amino)heptyl)-3-oxo-4-aza-5α-androst-1-ene-17β-carboxamide (C11)

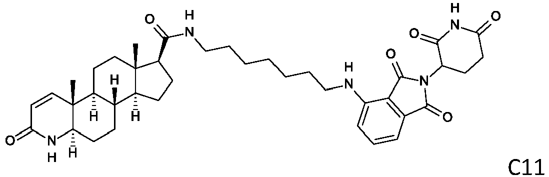

The synthesis was similar to that in Example 10, except that N-Boc-1,6-hexylenediamine hydrochloride in step 1 of Example 10 was replaced with N-Boc-1,7-heptanediamine hydrochloride, and 5-fluoro-2-(3-(2,6-dioxopiperidine))-isoindole-1,3-dione in step 3 was replaced with 4-fluoro-2-(3-(2,6-dioxopiperidine))-isoindole-1,3-dione.
The mass spectral data for compound C11 are, MS (ESI): (M+H)⁺, 686.4

The nuclear magnetic resonance data for compound C11 are as follows:
H NMR (400 MHz, CDCl₃) δ: 8.55-8.57 (m, 1H), 7.44-7.48 (m, 1H), 7.06-7.08 (m, 1H), 6.75-6.82 (m, 1H), 6.62-6.68 (m, 1H), 5.69-5.73 (m, 2H), 5.53-5.56 (m, 1H), 4.85-4.91 (m, 1H), 3.25-3.33 (m, 2H), 3.11-3.17 (m, 3H), 2.64-2.81 (m, 3H), 2.05-2.17 (m, 4H), 1.89-1.97 (m, 2H), 1.51-1.83 (m, 8H), 1.17-1.47 (m, 10H), 0.99-1.08 (m, 3H), 0.88 (s, 3H), 0.66 (s, 3H)

### Example 12: Synthesis of N-(7-((5-(2-(3-(2,6-dioxopiperidine))-isoindole-1,3-dione))amino)heptyl)-3-oxo-4-aza-5α-androst-1-ene-17β-carboxamide (C12)

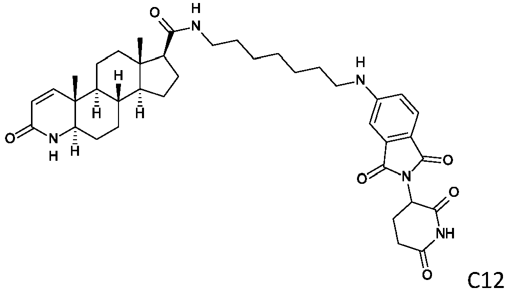

The synthesis was similar to that in Example 10, except that *N*-Boc-1,6-hexylenediamine hydrochloride in step 1 of Example 10 was replaced with *N*-Boc-1,7-heptanediamine hydrochloride.
The mass spectral data for compound C12 are, MS (ESI): (M+H)⁺, 686.4

The nuclear magnetic resonance data for compound C12 are as follows: H NMR (400 MHz, CDCl₃) δ: 8.09-8.38 (m, 1H), 7.52-7.55 (m, 1H), 6.84 (s, 1H), 6.63-6.75 (m, 2H), 5.65-5.74 (m, 2H), 5.27-5.30 (m, 1H), 4.71-4.86 (m, 1H), 3.20-3.28 (m, 2H), 3.10-3.16 (m, 3H), 2.61-2.82 (m, 3H), 2.02-2.16 (m, 5H), 1.90-1.96 (m, 2H), 1.54-1.72 (m, 7H), 1.17-1.43 (m, 10H), 0.94-1.10 (m, 3H), 0.97 (s, 3H), 0.61 (s, 3H)

### Example 13: Synthesis of 1-(4-(2-(3-(2,6-dioxopiperidine))-isoindole-1,3-dione))-4-(4-(1-(3-oxo-4-aza-5α-androst-1-ene-17β-formyl)piperidine)methyl)-piperazine (C13)

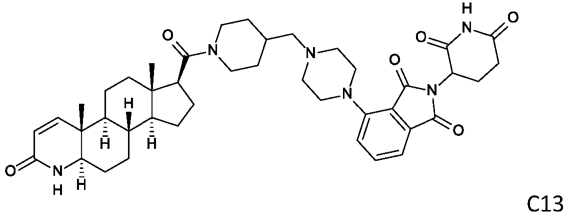

### Step 1: Synthesis of 4-(piperidin-4-yl-methyl)-1-Boc-piperazine (II-13)

### a. Synthesis of 4-(4-(1-Cbz-piperidine)methyl)-1-Boc-piperazine (II-13-1)

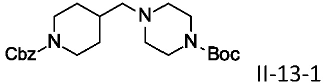

At room temperature, 3.96 g of 1-Cbz-4-formylpiperidine, 3.16 g of *N*-Boc-piperazine, 40 mL of DME, and 0.72 g of glacial acetic acid were sequentially added to a 100-mL three-necked flask, before 6.90 g of STAB was added with stirring. The system was heated to 40 °C and incubated for 3 h. The reaction solution was concentrated to give an oily substance, and the oily substance was purified by column chromatography (eluent: petroleum ether:ethyl acetate = 10:1) to give compound II-13-1 (2.71 g).

### b. Synthesis of 4-(piperidin-4-yl-methyl)-1-Boc-piperazine (II-13)

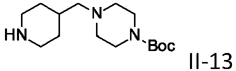

At room temperature, 2.71 g of compound II-13-1, 40 mL of methanol, 270 mg of 5% palladium on carbon, and 0.77 g of glacial acetic acid were sequentially added into a 100-mL hydrogenation reactor for 4 h of hydrogenation reaction at 60 °C. When the reaction was completed, the reaction solution was concentrated to give a black oily substance. The oily substance was purified by column chromatography (eluent: dichloromethane:methanol = 10:1) to give a gray oily substance (1.39 g, crude compound II-13).

### Step 2: Synthesis of 4-(4-(1-(3-oxo-4-aza-5α-androst-1-ene-17β-formyl)piperidine)methyl)-1-Boc-piperazine (IV-13)

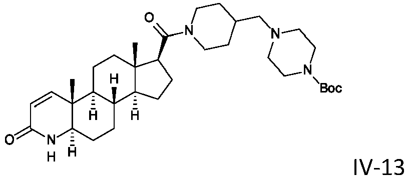

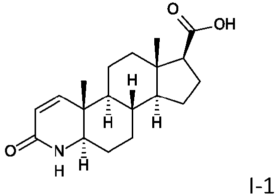

At room temperature, 1.11 g of compound I-1, 1.39 g of crude compound II-13, 6.55 g of pyBoP, and 50 mL of DMF were sequentially added into a 100-mL three-necked flask, and the mixture was stirred for 5 min. 2.44 g of DIPEA was added dropwise, and the reaction system was incubated overnight. The next day, 50 mL of water was added. The reaction solution was extracted with ethyl acetate (50 mL × 3). The organic phases were combined, sequentially washed once with 50 mL of 1 mol/L aqueous hydrochloric acid, 50 mL of saturated aqueous sodium carbonate, and 50 mL of saturated aqueous sodium chloride, dried over anhydrous sodium sulfate, and concentrated to give a black oily substance (2.56 g). The oily substance was purified by column chromatography (eluent: dichloromethane:methanol = 20:1) to give compound IV-13 (1.52 g).

### Step 3: Synthesis of 4-(4-(1-(3-oxo-4-aza-5α-androst-1-ene-17β-formyl)piperidine)methyl)-piperazine (V-13)

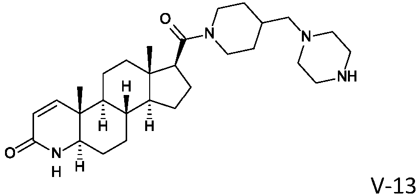

At room temperature, 1.52 g of compound IV-13 and 15 mL of dichloromethane were added into a 50-mL three-necked flask, before 15 mL of trifluoroacetic acid was dropwise added. The reaction system was incubated for 10 min. The reaction solution was concentrated to give an oily substance (crude compound V-13), which was directly used in the next reaction without purification.

### Step 4: Synthesis of 1-(4-(2-(3-(2,6-dioxopiperidine))-isoindole-1,3-dione))-4-(4-(1-(3-oxo-4-aza-5α-androst-1-ene-17β-formyl)piperidine)methyl)-piperazine (C13)

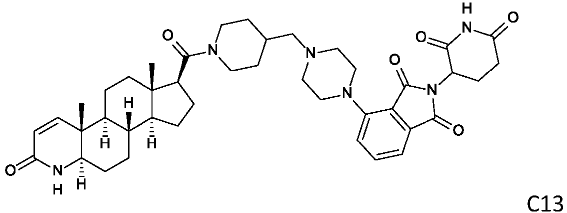

At room temperature, the oily substance obtained in step 3 (crude compound V-13), 0.17 g of 4-fluoro-2-(3-(2,6-dioxopiperidine))-isoindole-1,3-dione, 0.16 g of DIPEA, and 15 mL of DMF were sequentially added into a reaction flask. The reaction flask was treated in a microwave reactor for 10 min of reaction at 50 Hz. 20 mL of water was added into the reaction flask, and the mixture was extracted with dichloromethane (20 mL × 3). The organic phases were combined, washed once with 20 mL of water, dried over anhydrous sodium sulfate, and concentrated to give a crude product. The crude product was purified by column chromatography (eluent: dichloromethane:methanol = 10:1) to give compound C13 (40 mg).
The mass spectral data for compound C13 are, MS (ESI): (M+H)⁺, 739.4

The nuclear magnetic resonance data for compound C13 are as follows:
H NMR (400 MHz, CDCl₃) δ: 8.57-8.59 (m, 1H), 7.55-7.57 (m, 1H), 7.09-7.13 (m, 1H), 6.87-6.89 (m, 1H), 6.65-6.67 (m, 1H), 5.73-5.76 (m, 2H), 4.89-4.92 (m, 1H), 3.53-3.59 (m, 1H), 3.33-3.52 (m, 3H), 3.11-3.28 (m, 5H), 2.51-2.80 (m, 5H), 2.28-2.38 (m, 4H), 2.07-2.21 (m, 2H), 1.50-1.93 (m, 10H), 1.15-1.47 (m, 8H), 0.99-1.05 (m, 2H), 0.93 (s, 3H), 0.70 (s, 3H)

### Example 14: Synthesis of 1-(5-(2-(3-(2,6-dioxopiperidine))-isoindole-1,3-dione))-4-(4-(1-(3-oxo-4-aza-5α-androst-1-ene-17β-formyl)piperidine)methyl)-piperazine (C14)

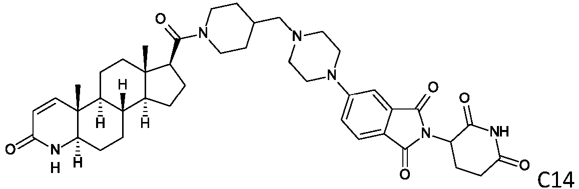

The synthesis was similar to that in Example 13, except that 4-fluoro-2-(3-(2,6-dioxopiperidine))-isoindole-1,3-dione in step 4 of Example 13 was replaced with 5-fluoro-2-(3-(2,6-dioxopiperidine))-isoindole-1,3-dione.
The mass spectral data for compound C14 are, MS (ESI): (M+H)⁺, 739.4

The nuclear magnetic resonance data for compound C14 are as follows:
H NMR (400 MHz, CDCl₃) δ: 8.77 (s, 1H), 7.49-7.50 (m, 1H), 6.96 (s, 1H), 6.71-6.72 (m, 1H), 5.88 (s, 1H), 5.73-5.75 (m, 1H), 5.41-5.45 (m, 1H), 4.92-4.97 (m, 1H), 3.15-3.49 (m, 9H), 2.59-2.75 (m, 5H), 2.07-2.35 (m, 6H), 1.58-1.99 (m, 10H), 1.11-1.44 (m, 8H), 0.95-1.01 (m, 2H), 0.94 (s, 3H), 0.71 (s, 3H)

### Example 15: Synthesis of N-(4-((2-((4-(2-(3-(2,6-dioxopiperidine))-isoindole-1,3-dione))amino)ethyl)oxy)phenyl)-3-oxo-4-aza-5α-androst-1-ene-17β-carboxamide (C15)

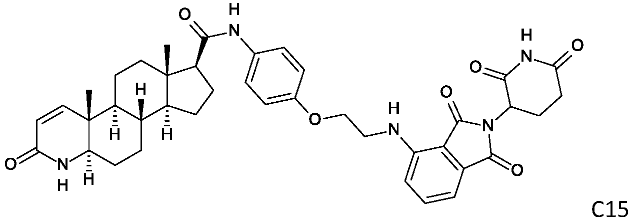

The synthesis was similar to that in Example 17, except that 1,3-dibromopropane in step 1 of Example 17 was replaced with 1,2-dibromoethane.
The mass spectral data for compound C15 are, MS (ESI): (M+H)⁺, 708.3

The nuclear magnetic resonance data for compound C15 are as follows:
H NMR (400 MHz, CDCl₃) δ: 8.65 (s, 1H), 7.53-7.57 (m, 1H), 7.36-7.40 (m, 2H), 7.17-7.23 (m, 3H), 7.02-7.03 (m, 1H), 6.79-6.82 (d, 1H, J=12Hz), 6.69-6.73 (m, 1H), 6.61-6.64 (m, 1H), 5.75 (s, 1H), 5.65-5.68 (d, 1H, J=12Hz), 5.05-5.09 (m, 1H), 4.02-4.06 (q, 2H, J=8Hz), 3.44-3.48 (m, 2H), 3.18-3.22 (m, 1H), 2.80-2.92 (m, 1H), 2.50-2.57 (m, 1H), 2.41-2.44 (m, 1H), 2.01-2.09 (m, 2H), 1.58-1.93 (m, 6H), 1.01-1.48 (m, 8H), 0.97 (s, 3H), 0.80 (s, 3H)

### Example 16: Synthesis of N-(4-((2-((5-(2-(3-(2,6-dioxopiperidine))-isoindole-1,3-dione))amino)ethyl)oxy)phenyl)-3-oxo-4-aza-5α-androst-1-ene-17β-carboxamide (C16)

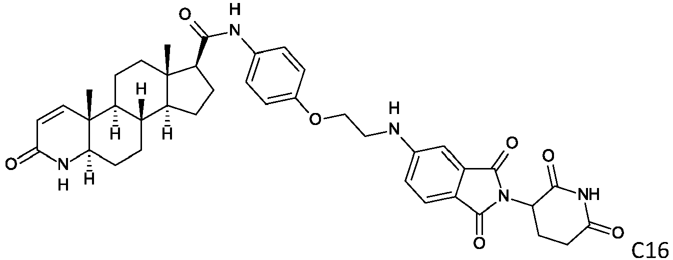

The synthesis was similar to that in Example 17, except that 1,3-dibromopropane in step 1 of Example 17 was replaced with 1,2-dibromoethane, and 4-fluoro-2-(3-(2,6-dioxopiperidine))-isoindole-1,3-dione in step 4 was replaced with 5-fluoro-2-(3-(2,6-dioxopiperidine))-isoindole-1,3-dione.
The mass spectral data for compound C16 are, MS (ESI): (M+H)⁺, 708.3

The nuclear magnetic resonance data for compound C16 are as follows:
H NMR (400 MHz, CDCl₃) δ: 8.85 (s, 1H), 7.45-7.48 (m, 1H), 7.36-7.40 (m, 1H), 7.17-7.23 (m, 2H), 7.02-7.03 (m, 2H), 6.91 (s, 1H), 6.69-6.71 (m, 2H), 5.73-5.77 (m, 1H), 5.55 (s, 1H), 4.92-4.97 (m, 1H), 3.99-4.03 (m, 2H), 3.19-3.27 (m, 3H), 2.65-2.75 (m, 3H), 2.01-2.15 (m, 2H), 1.57-1.93 (m, 7H), 1.15-1.47 (m, 6H), 0.95-1.01 (m, 2H), 0.97 (s, 3H), 0.72 (s, 3H)

### Example 17: Synthesis of N-(4-((3-((4-(2-(3-(2,6-dioxopiperidine))-isoindole-1,3-dione))amino)propyl)oxy)phenyl)-3-oxo-4-aza-5α-androst-1-ene-17β-carboxamide (C17)

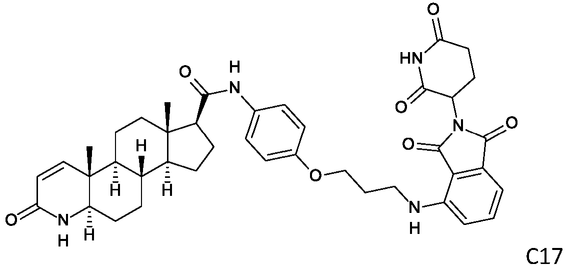

### Step 1: Synthesis of 4-((3-(N,N-di-Boc-amino)propyl)oxy)aniline (II-17)

### a. Synthesis of (3-bromopropyl)-N,N-di-Boc-amine (II-17-1)

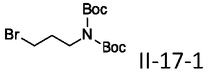

At room temperature, 4.65 g of 1,3-dibromopropane, 1.00 g of *N,N*-di-Boc-amine, 3.18 g of potassium carbonate, and 50 mL of DMF were sequentially added to a 250-mL three-necked flask, and the reaction system was heated to 60 °C and incubated for 3 h. The reaction solution was extracted with dichloromethane (40 mL × 3) and washed with 40 mL of water once. The organic phases were combined, dried over anhydrous sodium sulfate, and concentrated to give a crude product. The crude product was purified by column chromatography (eluent: petroleum ether:ethyl acetate = 15:1) to give a colorless transparent oily substance (compound II-17-1, 0.82 g).

### b. Synthesis of 4-((3-(N,N-di-Boc-amino)propyl)oxy)nitrobenzene (II-17-2)

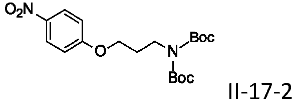

At room temperature, 0.50 g of compound II-17-1, 0.20 g of 4-nitrophenol, 0.40 g of potassium carbonate, and 30 mL of DMF were sequentially added to a 100-mL three-necked flask, and the reaction system was heated to 60 °C and incubated for 3 h. The reaction solution was extracted with dichloromethane (40 mL × 3) and washed with 40 mL of water once. The organic phases were combined, dried over anhydrous sodium sulfate, and concentrated to give a crude product. The crude product was purified by column chromatography (eluent: petroleum ether:ethyl acetate = 5:1) to give a colorless transparent oily substance (compound II-17-2, 0.30 g).

### c. Synthesis of 4-((3-(N,N-di-Boc-amino)propyl)oxy)aniline (II-17)

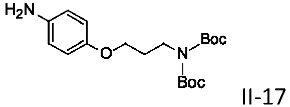

At room temperature, 0.30 g of compound II-17-2, 15 mL of methanol, and 30 mg of 5% palladium on carbon were sequentially added into a 100-mL hydrogenation reactor for 1 h of hydrogenation reaction at 25 °C. When the reaction was completed, the reaction solution was concentrated to give a dark gray oily substance (crude compound II-17, 0.28 g).

### Step 2: Synthesis of N-(4-((3-(N,N-di-Boc-amino)propyl)oxy)phenyl)-3-oxo-4-aza-5α-androst-1-ene-17β-carboxamide (IV-17)

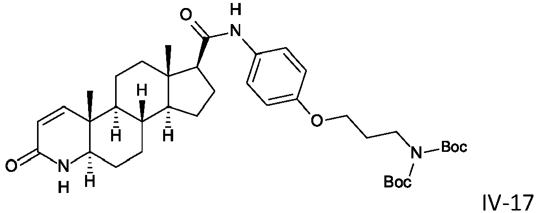

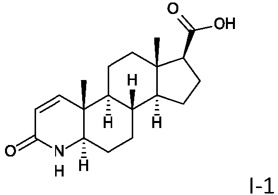

At room temperature, 0.26 g of compound I-1, 0.28 g of crude compound II-17, 0.79 g of pyBoP, and 20 mL of DMF were sequentially added into a 50-mL three-necked flask, and the mixture was stirred for 5 min. 0.49 g of DIPEA was added dropwise to the reaction system, and the reaction system was incubated overnight. The next day, 50 mL of water was added to the reaction solution, and the mixture was extracted with ethyl acetate (50 mL × 3). The organic phases were combined, sequentially washed once with 50 mL of 1 mol/L aqueous hydrochloric acid, 50 mL of saturated aqueous sodium carbonate, and 50 mL of saturated aqueous sodium chloride, dried over anhydrous sodium sulfate, and concentrated to give a black oily substance (2.56 g). The oily substance was purified by column chromatography (eluent: dichloromethane:methanol = 20:1) to give compound IV-17 (0.21 g).

### Step 3: Synthesis of N-(4-((3-aminopropyl)oxy)phenyl)-3-oxo-4-aza-5α-androst-1-ene-17β-carboxamide (V-17)

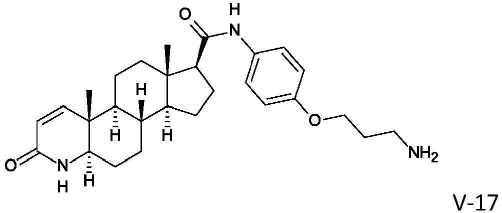

At room temperature, 0.21 g of compound IV-17 and 10 mL of dichloromethane were sequentially added into a 50 mL three-necked flask, before 10 mL of trifluoroacetic acid was dropwise added. The reaction system was incubated for 10 min. The reaction solution was concentrated to give an oily substance (crude compound V-17), which was directly used in the next reaction without purification.

### Step 4: Synthesis of N-(4-((3-((4-(2-(3-(2,6-dioxopiperidine))-isoindole-1,3-dione))amino)propyl)oxy)phenyl)-3-oxo-4-aza-5α-androst-1-ene-17β-carboxamide (C17)

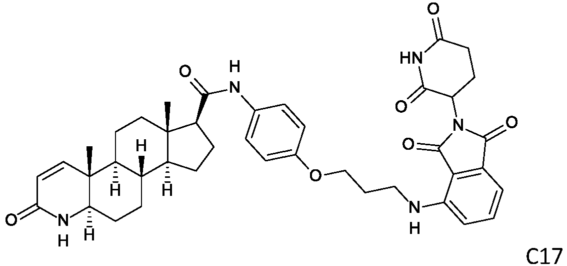

At room temperature, the oily substance obtained in step 3 (crude compound V-17), 0.09 g of 4-fluoro-2-(3-(2,6-dioxopiperidine))-isoindole-1,3-dione, 0.14 g of DIPEA, and 3 mL of DMF were sequentially added into a reaction flask. The reaction flask was treated in a microwave reactor for 10 min of reaction at 50 Hz. 15 mL of water was added into the reaction flask, and the mixture was extracted with dichloromethane (15 mL × 3). The organic phases were combined, washed once with 15 mL of water, dried over anhydrous sodium sulfate, and concentrated to give a crude product. The crude product was purified by column chromatography (eluent: dichloromethane:methanol = 10:1) to give compound C17 (35 mg).
The mass spectral data for compound C17 are, MS (ESI): (M+H)⁺, 722.3

The nuclear magnetic resonance data for compound C17 are as follows:
H NMR (400 MHz, CDCl₃) δ: 8.49 (s, 1H), 7.52-7.56 (m, 1H), 7.35-7.39 (m, 2H), 7.16-7.22 (m, 3H), 7.02-7.03 (m, 1H), 6.79-6.82 (d, 1H, J=12Hz), 6.69-6.73 (m, 1H), 6.61-6.64 (m, 1H), 5.75 (s, 1H), 5.65-5.68 (d, 1H, J=12Hz), 5.05-5.09 (m, 1H), 4.02-4.06 (q, 2H, J=8Hz), 3.44-3.48 (m, 2H), 3.18-3.22 (m, 1H), 2.80-2.92 (m, 1H), 2.50-2.57 (m, 1H), 2.41-2.44 (m, 1H), 2.01-2.09 (m, 4H), 1.58-1.79 (m, 6H), 0.97-1.48 (m, 8H), 0.93 (s, 3H), 0.81 (s, 3H)

### Example 18: Synthesis of N-(4-((3-((5-(2-(3-(2,6-dioxopiperidine))-isoindole-1,3-dione))amino)propyl)oxy)phenyl)-3-oxo-4-aza-5α-androst-1-ene-17β-carboxamide (C18)

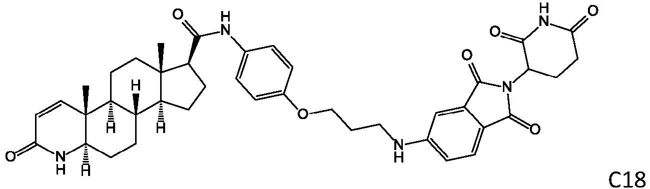

The synthesis was similar to that in Example 17, except that 4-fluoro-2-(3-(2,6-dioxopiperidine))-isoindole-1,3-dione in step 4 of Example 17 was replaced with 5-fluoro-2-(3-(2,6-dioxopiperidine))-isoindole-1,3-dione.
The mass spectral data for compound C18 are, MS (ESI): (M+H)⁺, 722.3

The nuclear magnetic resonance data for compound C18 are as follows:
H NMR (400 MHz, CDCl₃) δ: 8.55 (s, 1H), 7.44-7.45 (m, 1H), 7.37-7.40 (m, 1H), 7.17-7.22 (m, 2H), 7.01-7.03 (m, 2H), 6.95 (s, 1H), 6.70-6.75 (m, 2H), 5.75-5.77 (m, 1H), 5.57 (s, 1H), 4.92-4.97 (m, 1H), 4.01-4.04 (m, 2H), 3.25-3.33 (m, 3H), 2.65-2.75 (m, 3H), 2.01-2.15 (m, 2H), 1.78-1.97 (m, 4H), 1.57-1.73 (m, 5H), 1.15-1.47 (m, 6H), 0.95-1.01 (m, 2H), 0.97 (s, 3H), 0.72 (s, 3H)

### Example 19: Synthesis of N-(4-((5-((4-(2-(3-(2,6-dioxopiperidine))-isoindole-1,3-dione))amino)pentyl)oxy)phenyl)-3-oxo-4-aza-5α-androst-1-ene-17β-carboxamide (C19)

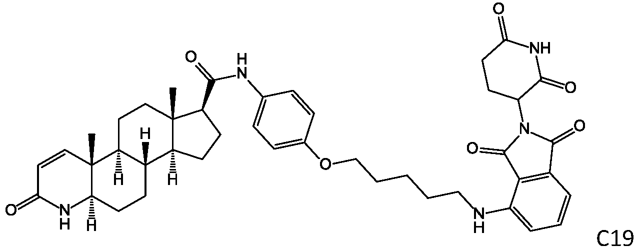

The synthesis was similar to that in Example 17, except that 1,3-dibromopropane in step 1 of Example 17 was replaced with 1,5-dibromopentane.
The mass spectral data for compound C19 are, MS (ESI): (M+H)⁺, 750.4

The nuclear magnetic resonance data for compound C19:
H NMR (400 MHz, CDCl₃) δ: 8.65 (s, 1H), 7.53-7.56 (m, 1H), 7.36-7.43 (m, 2H), 7.15-7.22 (m, 3H), 7.02-7.03 (m, 1H), 6.79-6.82 (d, 1H, J=12Hz), 6.69-6.73 (m, 1H), 6.65-6.67 (m, 1H), 5.75 (s, 1H), 5.65-5.68 (d, 1H, J=12Hz), 5.05-5.09 (m, 1H), 4.02-4.06 (q, 2H, J=8Hz), 3.44-3.48 (m, 2H), 3.18-3.22 (m, 1H), 2.80-2.92 (m, 1H), 2.50-2.57 (m, 1H), 2.41-2.44 (m, 1H), 2.01-2.09 (m, 4H), 1.58-1.91 (m, 10H), 0.97-1.11 (m, 2H), 1.15-1.48 (m, 6H), 0.91 (s, 3H), 0.77 (s, 3H)

### Example 20: Synthesis of N-(4-((5-((5-(2-(3-(2,6-dioxopiperidine))-isoindole-1,3-dione))amino)pentyl)oxy)phenyl)-3-oxo-4-aza-5α-androst-1-ene-17β-carboxamide (C20)

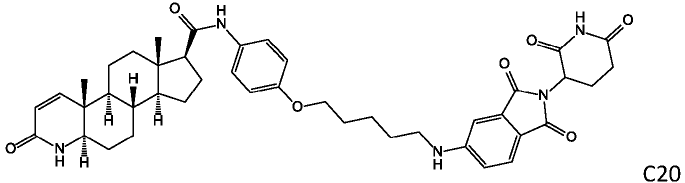

The synthesis was similar to that in Example 17, except that 1,3-dibromopropane in step 1 of Example 17 was replaced with 1,5-dibromopentane, and 4-fluoro-2-(3-(2,6-dioxopiperidine))-isoindole-1,3-dione in step 4 was replaced with 5-fluoro-2-(3-(2,6-dioxopiperidine))-isoindole-1,3-dione.
The mass spectral data for compound C20 are, MS (ESI): (M+H)⁺, 750.4

The nuclear magnetic resonance data for compound C20 are as follows:
H NMR (400 MHz, CDCl₃) δ: 8.57 (s, 1H), 7.43-7.45 (m, 1H), 7.37-7.41 (m, 1H), 7.17-7.22 (m, 2H), 7.02-7.05 (m, 2H), 6.95 (s, 1H), 6.71-6.75 (m, 2H), 5.75-5.77 (m, 1H), 5.58 (s, 1H), 4.92-4.97 (m, 1H), 4.00-4.03 (m, 2H), 3.18-3.31 (m, 3H), 2.61-2.75 (m, 3H), 2.05-2.15 (m, 2H), 1.61-1.99 (m, 12H), 1.15-1.55 (m, 7H), 0.92-1.01 (m, 2H), 0.96 (s, 3H), 0.72 (s, 3H)

### Example 21: Synthesis of N-(4-((6-((4-(2-(3-(2,6-dioxopiperidine))-isoindole-1,3-dione))amino)hexyl)oxy)phenyl)-3-oxo-4-aza-5α-androst-1-ene-17β-carboxamide (C21)

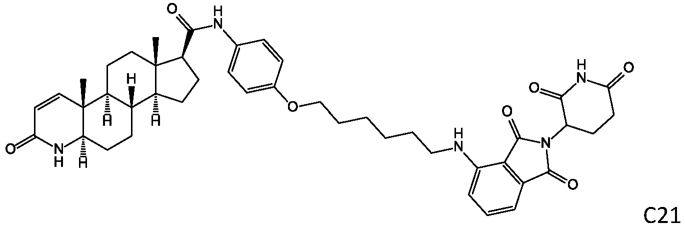

The synthesis was similar to that in Example 17, except that 1,3-dibromopropane in step 1 of Example 17 was replaced with 1,6-dibromohexane.
The mass spectral data for compound C21 are, MS (ESI): (M+H)⁺, 764.4

The nuclear magnetic resonance data for compound C21 are as follows:
H NMR (400 MHz, CDCl₃) δ: 8.65 (s, 1H), 7.53-7.56 (m, 1H), 7.36-7.43 (m, 2H), 7.15-7.22 (m, 3H), 7.02-7.03 (m, 1H), 6.79-6.82 (d, 1H, J=12Hz), 6.69-6.73 (m, 1H), 6.65-6.67 (m, 1H), 5.75 (s, 1H), 5.65-5.68 (d, 1H, J=12Hz), 5.05-5.09 (m, 1H), 4.02-4.06 (q, 2H, J=8Hz), 3.44-3.48 (m, 2H), 3.18-3.22 (m, 1H), 2.80-2.92 (m, 1H), 2.50-2.57 (m, 1H), 2.41-2.44 (m, 1H), 2.01-2.09 (m, 4H), 1.85-1.99 (m, 2H), 1.58-1.79 (m, 10H), 0.97-1.11 (m, 2H), 1.15-1.48 (m, 6H), 0.91 (s, 3H), 0.76 (s, 3H)

### Example 22: Synthesis of N-(4-((6-((5-(2-(3-(2,6-dioxopiperidine))-isoindole-1,3-dione))amino)hexyl)oxy)phenyl)-3-oxo-4-aza-5α-androst-1-ene-17β-carboxamide (C22)

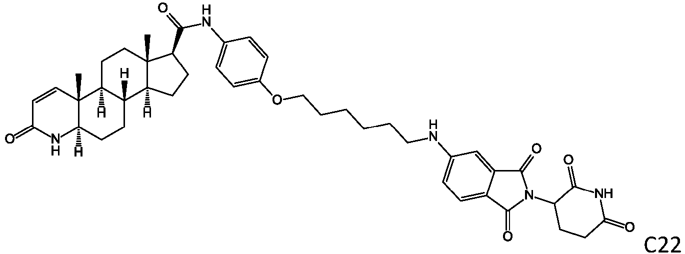

The synthesis was similar to that in Example 17, except that 1,3-dibromopropane in step 1 of Example 17 was replaced with 1,6-dibromohexane, and 4-fluoro-2-(3-(2,6-dioxopiperidine))-isoindole-1,3-dione in step 4 was replaced with 5-fluoro-2-(3-(2,6-dioxopiperidine))-isoindole-1,3-dione.
The mass spectral data for compound C22 are, MS (ESI): (M+H)⁺, 764.4

The nuclear magnetic resonance data for compound C22 are as follows:
H NMR (400 MHz, CDCl₃) δ: 8.65 (s, 1H), 7.41-7.45 (m, 1H), 7.37-7.39 (m, 1H), 7.18-7.22 (m, 2H), 7.02-7.05 (m, 2H), 7.01 (s, 1H), 6.71-6.75 (m, 2H), 5.74-5.77 (m, 1H), 5.45 (s, 1H), 4.88-4.91 (m, 1H), 3.99-4.03 (m, 2H), 3.21-3.35 (m, 3H), 2.61-2.90 (m, 3H), 2.01-2.08 (m, 2H), 1.71-1.99 (m, 12H), 1.11-1.65 (m, 11H), 0.98-1.08 (m, 2H), 0.93 (s, 3H), 0.70 (s, 3H)

### Example 23: Synthesis of N-(4-((7-((4-(2-(3-(2,6-dioxopiperidine))-isoindole-1,3-dione))amino)heptyl)oxy)phenyl)-3-oxo-4-aza-5α-androst-1-ene-17β-carboxamide (C23)

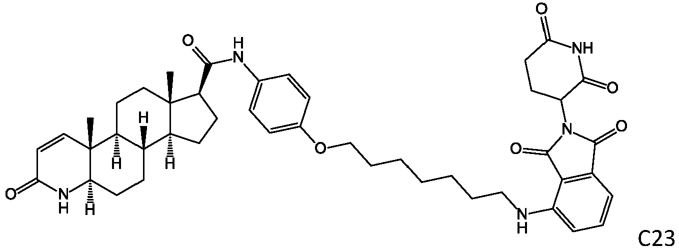

The synthesis was similar to that in Example 17, except that 1,3-dibromopropane in step 1 of Example 17 was replaced with 1,7-dibromoheptane.
The mass spectral data for compound C23 are, MS (ESI): (M+H)⁺, 778.4

The nuclear magnetic resonance data for compound C23 are as follows:
H NMR (400 MHz, CDCl₃) δ: 8.55 (s, 1H), 7.53-7.57 (m, 1H), 7.36-7.43 (m, 2H), 7.16-7.22 (m, 3H), 7.01-7.03 (m, 1H), 6.80-6.82 (d, 1H, J=12Hz), 6.69-6.74 (m, 1H), 6.65-6.67 (m, 1H), 5.77 (s, 1H), 5.70-5.72 (d, 1H, J=12Hz), 5.05-5.09 (m, 1H), 4.04-4.06 (q, 2H, J=8Hz), 3.45-3.48 (m, 2H), 2.98-3.15 (m, 3H), 2.44-2.55 (m, 2H), 2.01-2.09 (m, 5H), 1.85-1.99 (m, 3H), 1.58-1.79 (m, 10H), 0.97-1.11 (m, 2H), 1.15-1.48 (m, 6H), 0.89 (s, 3H), 0.75 (s, 3H)

### Example 24: Synthesis of N-(4-((7-((5-(2-(3-(2,6-dioxopiperidine))-isoindole-1,3-dione))amino)heptyl)oxy)phenyl)-3-oxo-4-aza-5α-androst-1-ene-17β-carboxamide (C24)

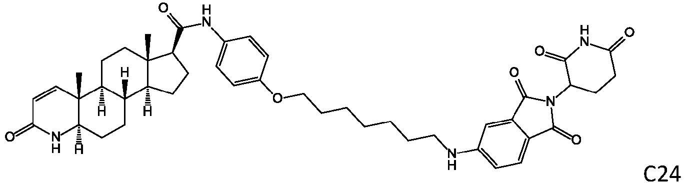

The synthesis was similar to that in Example 17, except that 1,3-dibromopropane in step 1 of Example 17 was replaced with 1,7-dibromoheptane, and 4-fluoro-2-(3-(2,6-dioxopiperidine))-isoindole-1,3-dione in step 4 was replaced with 5-fluoro-2-(3-(2,6-dioxopiperidine))-isoindole-1,3-dione.
The mass spectral data for compound C24 are, MS (ESI): (M+H)⁺, 778.4

The nuclear magnetic resonance data for compound C24 are as follows:
H NMR (400 MHz, CDCl₃) δ: 8.68 (s, 1H), 7.39-7.42 (m, 1H), 7.37-7.39 (m, 1H), 7.18-7.25 (m, 2H), 7.01-7.05 (m, 2H), 6.99 (s, 1H), 6.71-6.74 (m, 2H), 5.74-5.77 (m, 1H), 5.66 (s, 1H), 4.89-4.95 (m, 1H), 3.99-4.03 (m, 2H), 3.23-3.41 (m, 3H), 2.61-2.77 (m, 3H), 1.71-2.11 (m, 16H), 1.11-1.65 (m, 11H), 0.98-1.08 (m, 2H), 0.89 (s, 3H), 0.69 (s, 3H)

### Example 25: Synthesis of N-(4-((8-((4-(2-(3-(2,6-dioxopiperidine))-isoindole-1,3-dione))amino)octyl)oxy)phenyl)-3-oxo-4-aza-5α-androst-1-ene-17β-carboxamide (C25)

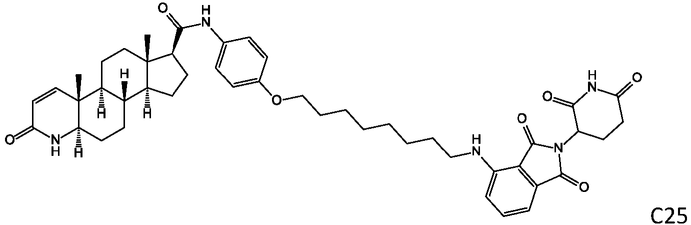

The synthesis was similar to that in Example 17, except that 1,3-dibromopropane in step 1 of Example 17 was replaced with 1,8-dibromooctane.
The mass spectral data for compound C25 are, MS (ESI): (M+H)⁺, 792.4

The nuclear magnetic resonance data for compound C25 are as follows:
H NMR (400 MHz, CDCl₃) δ: 8.55 (s, 1H), 7.53-7.57 (m, 1H), 7.36-7.43 (m, 2H), 7.16-7.22 (m, 3H), 7.01-7.03 (m, 1H), 6.80-6.82 (d, 1H, J=12Hz), 6.69-6.74 (m, 1H), 6.65-6.67 (m, 1H), 5.77 (s, 1H), 5.70-5.72 (d, 1H, J=12Hz), 5.05-5.09 (m, 1H), 4.04-4.06 (q, 2H, J=8Hz), 3.45-3.48 (m, 2H), 2.98-3.15 (m, 3H), 2.44-2.55 (m, 2H), 2.01-2.09 (m, 5H), 1.58-1.97 (m, 15H), 0.97-1.11 (m, 2H), 1.15-1.48 (m, 6H), 0.90 (s, 3H), 0.71 (s, 3H)

### Example 26: Synthesis of N-(4-((8-((5-(2-(3-(2,6-dioxopiperidine))-isoindole-1,3-dione))amino)octyl)oxy)phenyl)-3-oxo-4-aza-5α-androst-1-ene-17β-carboxamide (C26)

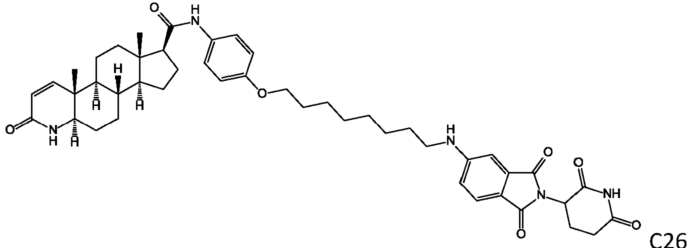

The synthesis was similar to that in Example 17, except that 1,3-dibromopropane in step 1 of Example 17 was replaced with 1,8-dibromooctane, and 4-fluoro-2-(3-(2,6-dioxopiperidine))-isoindole-1,3-dione in step 4 was replaced with 5-fluoro-2-(3-(2,6-dioxopiperidine))-isoindole-1,3-dione.
The mass spectral data for compound C26 are, MS (ESI): (M+H)⁺, 792.4

The nuclear magnetic resonance data for compound C26 are as follows:
H NMR (400 MHz, CDCl₃) δ: 8.69 (s, 1H), 7.39-7.42 (m, 1H), 7.35-7.39 (m, 1H), 7.19-7.25 (m, 2H), 7.01-7.04 (m, 2H), 6.95 (s, 1H), 6.71-6.74 (m, 2H), 5.74-5.77 (m, 1H), 5.72 (s, 1H), 4.85-4.93 (m, 1H), 4.00-4.03 (m, 2H), 3.21-3.38 (m, 3H), 2.60-2.78 (m, 3H), 1.55-2.11 (m, 20H), 1.11-1.47 (m, 11H), 0.86 (s, 3H), 0.68 (s, 3H)

### Example 27: Synthesis of N-(3-((2-((4-(2-(3-(2,6-dioxopiperidine))-isoindole-1,3-dione))amino)ethyl)oxy)phenyl)-3-oxo-4-aza-5α-androst-1-ene-17β-carboxamide (C27)

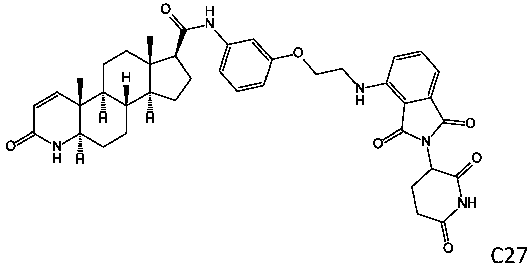

### Step 1: Synthesis of 3-((2-(N,N-di-Boc-amino)ethyl)oxy)aniline (II-27)

### a. Synthesis of (2-bromoethyl)-N,N-di-Boc-amine (II-27-1)

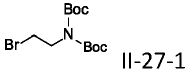

At room temperature, 4.32 g of 1,2-dibromoethane, 1.00 g of *N,N*-di-Boc-amine, 3.18 g of potassium carbonate, and 50 mL of DMF were sequentially added to a 250-mL three-necked flask, and the reaction system was heated to 60 °C and incubated for 3 h. The reaction solution was extracted with dichloromethane (40 mL × 3) and washed with 40 mL of water once. The organic phases were combined, dried over anhydrous sodium sulfate, and concentrated to give a crude product. The crude product was purified by column chromatography (eluent: petroleum ether:ethyl acetate = 15:1) to give a colorless transparent oily substance (compound II-27-1, 0.76 g).

### b. Synthesis of 3-((2-(N,N-di-Boc-amino)ethyl)oxy)nitrobenzene (II-27-2)

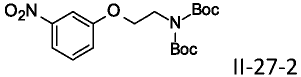

At room temperature, 0.70 g of compound II-27-1, 0.29 g of 3-nitrophenol, 0.58 g of potassium carbonate, and 30 mL of DMF were sequentially added to a 100-mL three-necked flask, and the reaction system was heated to 60 °C and incubated for 3 h. The reaction solution was extracted with dichloromethane (40 mL × 3) and washed with 40 mL of water once. The organic phases were combined, dried over anhydrous sodium sulfate, and concentrated to give a crude product. The crude product was purified by column chromatography (eluent: petroleum ether:ethyl acetate = 5:1) to give a colorless transparent oily substance (compound II-27-2, 0.46 g).

### c. Synthesis of 3-((2-(N,N-di-Boc-amino)ethyl)oxy)aniline (II-27)

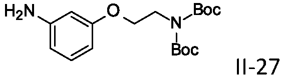

At room temperature, 0.46 g of compound II-27-2, 15 mL of methanol, and 46 mg of 5% palladium on carbon were sequentially added into a 100-mL hydrogenation reactor for 1 h of hydrogenation reaction at 25 °C. When the reaction was completed, the reaction solution was concentrated to give a dark gray oily substance (crude compound II-27, 0.44 g).

### Step 2: Synthesis of N-(3-((2-(N,N-di-Boc-amino)ethyl)oxy)phenyl)-3-oxo-4-aza-5α-androst-1-ene-17β-carboxamide (IV-27)

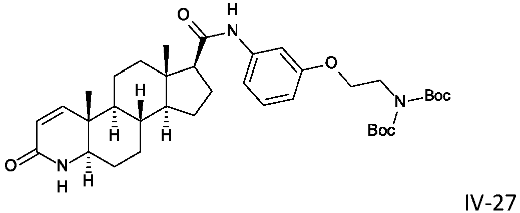

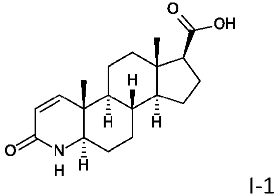

At room temperature, 0.38 g of compound I-1, 0.44 g of crude compound II-27, 1.25 g of pyBoP, and 25 mL of DMF were sequentially added into a reaction flask, and the mixture was stirred for 5 min. 0.70 g of DIPEA was added dropwise to the reaction system, and the reaction system was incubated overnight. The next day, 50 mL of water was added to the reaction solution, and the mixture was extracted with ethyl acetate (50 mL × 3). The organic phases were combined, sequentially washed once with 50 mL of 1 mol/L aqueous hydrochloric acid, 50 mL of saturated aqueous sodium carbonate, and 50 mL of saturated aqueous sodium chloride, dried over anhydrous sodium sulfate, and concentrated to give a black oily substance (2.49 g). The oily substance was purified by column chromatography (eluent: dichloromethane:methanol = 20:1) to give compound IV-27 (0.40 g).

### Step 3: Synthesis of N-(3-((2-aminoethyl)oxy)phenyl)-3-oxo-4-aza-5α-androst-1-ene-17β-carboxamide (V-27)

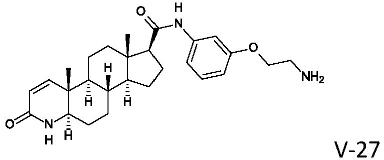

At room temperature, 0.40 g of compound IV-27 and 15 mL of dichloromethane were sequentially added into a reaction flask, before 15 mL of trifluoroacetic acid was dropwise added. The reaction system was incubated for 10 min. The reaction solution was concentrated to give an oily substance (crude compound V-27), which was directly used in the next reaction without purification.

### Step 4: Synthesis of N-(3-((2-((4-(2-(3-(2,6-dioxopiperidine))-isoindole-1,3-dione))amino)ethyl)oxy)phenyl)-3-oxo-4-aza-5α-androst-1-ene-17β-carboxamide (C27)

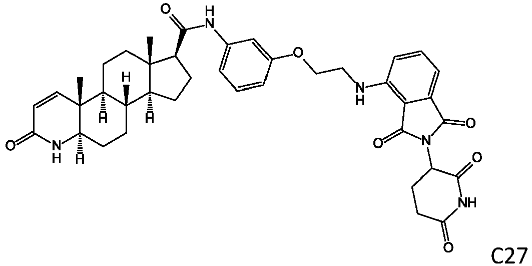

At room temperature, the oily substance obtained in step 3 (crude compound V-27), 0.23 g of 4-fluoro-2-(3-(2,6-dioxopiperidine))-isoindole-1,3-dione, 0.36 g of DIPEA, and 6 mL of DMF were sequentially added into a reaction flask. The reaction flask was treated in a microwave reactor for 10 min of reaction at 50 Hz. 20 mL of water was added into the reaction flask, and the mixture was extracted with dichloromethane (20 mL × 3). The organic phases were combined, washed once with 20 mL of water, dried over anhydrous sodium sulfate, and concentrated to give a crude product. The crude product was purified by column chromatography (eluent: dichloromethane:methanol = 10:1) to give compound C27 (0.13 g).
The mass spectral data for compound C27 are, MS (ESI): (M+H)⁺, 708.3

The nuclear magnetic resonance data for compound C27 are as follows:
H NMR (400 MHz, CDCl₃) δ: 8.95-8.96 (m, 1H), 7.51-7.52 (m, 1H), 7.35-7.37 (m, 1H), 7.09-7.12 (m, 1H), 6.95-7.02 (m, 2H), 6.82-6.86 (m, 2H), 6.70-6.74 (m, 1H), 6.59-6.61 (m, 1H), 6.38-6.42 (m, 1H), 5.75 (s, 1H), 5.71-5.73 (m, 1H), 4.75-4.83 (m, 1H), 4.01-4.03 (m, 2H), 3.41-3.44 (m, 2H), 3.25-3.28 (m, 1H), 2.60-2.79 (m, 3H), 2.18-2.25 (m, 2H), 1.65-2.10 (m, 6H), 1.48-1.53 (m, 2H), 1.16-1.37 (m, 5H), 0.92-0.95 (m, 2H), 0.91 (s, 3H), 0.78 (s, 3H)

### Example 28: Synthesis of N-(3-((2-((5-(2-(3-(2,6-dioxopiperidine))-isoindole-1,3-dione))amino)ethyl)oxy)phenyl)-3-oxo-4-aza-5α-androst-1-ene-17β-carboxamide (C28)

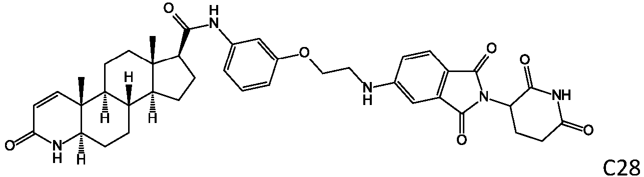

The synthesis was similar to that in Example 27, except that 4-fluoro-2-(3-(2,6-dioxopiperidine))-isoindole-1,3-dione in step 4 of Example 27 was replaced with 5-fluoro-2-(3-(2,6-dioxopiperidine))-isoindole-1,3-dione.
The mass spectral data for compound C28 are, MS (ESI): (M+H)⁺, 708.3

The nuclear magnetic resonance data for compound C28 are as follows:
H NMR (400 MHz, CDCl₃) δ: 8.88-8.90 (m, 1H), 7.45-7.47 (m, 1H), 7.28-7.29 (m, 1H), 7.09-7.12 (m, 1H), 7.00-7.02 (m, 2H), 6.91 (s, 1H), 6.79-6.83 (m, 2H), 6.70-6.72 (m, 1H), 6.55-6.57 (m, 1H), 6.38-6.42 (m, 1H), 5.75 (s, 1H), 5.71-5.73 (m, 1H), 4.73-4.83 (m, 1H), 4.01-4.03 (m, 2H), 3.41-3.44 (m, 2H), 3.25-3.28 (m, 1H), 2.60-2.79 (m, 3H), 2.10-2.25 (m, 2H), 1.65-2.01 (m, 6H), 1.48-1.55 (m, 2H), 1.11-1.37 (m, 5H), 0.92-0.95 (m, 2H), 0.91 (s, 3H), 0.76 (s, 3H)

### Example 29: Synthesis of N-(3-((3-((4-(2-(3-(2,6-dioxopiperidine))-isoindole-1,3-dione))amino)propyl)oxy)phenyl)-3-oxo-4-aza-5α-androst-1-ene-17β-carboxamide (C29)

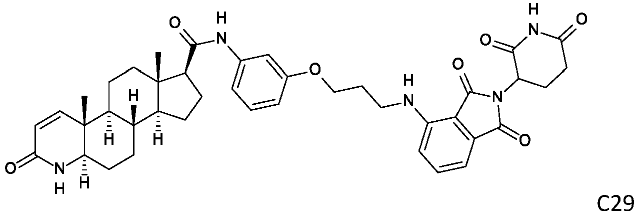

### Step 1: Synthesis of 3-((3-(N,N-di-Boc-amino)propyl)oxy)aniline (II-29)

### a. Synthesis of 3-((3-(N,N-di-Boc-amino)propyl)oxy)nitrobenzene (II-29-1)

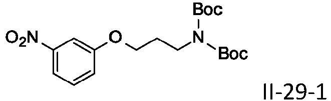

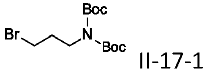

At room temperature, 0.80 g of compound II-17-1, 0.31 g of 3-nitrophenol, 0.64 g of potassium carbonate, and 30 mL of DMF were sequentially added to a 100-mL three-necked flask, and the reaction system was heated to 60 °C and incubated for 3 h. The reaction solution was extracted with dichloromethane (40 mL × 3) and washed with 40 mL of water once. The organic phases were combined, dried over anhydrous sodium sulfate, and concentrated to give a crude product. The crude product was purified by column chromatography (eluent: petroleum ether:ethyl acetate = 5:1) to give a colorless transparent oily substance (compound II-29-1, 0.52 g).

### b. Synthesis of 3-((3-(N,N-di-Boc-amino)propyl)oxy)aniline (II-29)

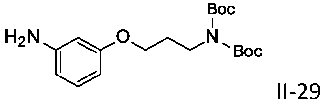

At room temperature, 0.52 g of compound II-29-1, 15 mL of methanol, and 52 mg of 5% palladium on carbon were sequentially added into a 100-mL hydrogenation reactor for 1 h of hydrogenation reaction at 25 °C. When the reaction was completed, the reaction solution was concentrated to give a dark gray oily substance (crude compound II-29, 0.46 g).

### Step 2: Synthesis of N-(3-((3-(N,N-di-Boc-amino)propyl)oxy)phenyl)-3-oxo-4-aza-5α-androst-1-ene-17β-carboxamide (IV-29)

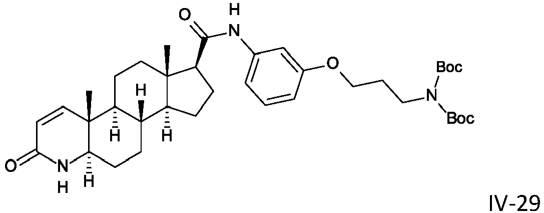

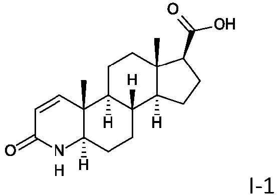

At room temperature, 0.41 g of compound I-1, 0.46 g of crude compound II-29, 1.30 g of pyBoP, and 25 mL of DMF were sequentially added into a reaction flask, and the mixture was stirred for 5 min. 0.72 g of DIPEA was added dropwise to the reaction system, and the reaction system was incubated overnight. The next day, 50 mL of water was added to the reaction solution, and the mixture was extracted with ethyl acetate (50 mL × 3). The organic phases were combined, sequentially washed once with 50 mL of 1 mol/L aqueous hydrochloric acid, 50 mL of saturated aqueous sodium carbonate, and 50 mL of saturated aqueous sodium chloride, dried over anhydrous sodium sulfate, and concentrated to give a black oily substance (2.56 g). The oily substance was purified by column chromatography (eluent: dichloromethane:methanol = 20:1) to give compound IV-29 (0.52 g).

### Step 3: Synthesis of N-(3-((3-aminopropyl)oxy)phenyl)-3-oxo-4-aza-5α-androst-1-ene-17β-carboxamide (V-29)

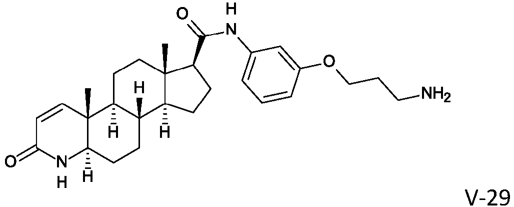

At room temperature, 0.52 g of compound IV-29 and 15 mL of dichloromethane were sequentially added into a reaction flask, before 15 mL of trifluoroacetic acid was dropwise added. The reaction system was incubated for 10 min. The reaction solution was concentrated to give an oily substance (crude compound V-29), which was directly used in the next reaction without purification.

### Step 4: Synthesis of N-(3-((3-((4-(2-(3-(2,6-dioxopiperidine))-isoindole-1,3-dione))amino)propyl)oxy)phenyl)-3-oxo-4-aza-5α-androst-1-ene-17β-carboxamide (C29)

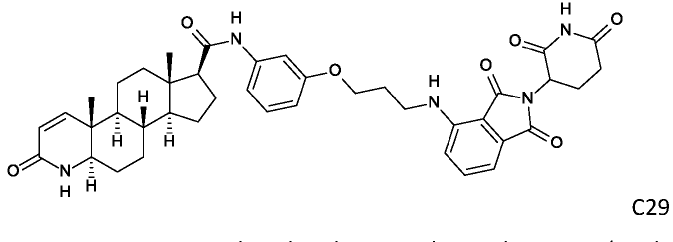

At room temperature, the oily substance obtained in step 3 (crude compound V-29), 0.30 g of 4-fluoro-2-(3-(2,6-dioxopiperidine))-isoindole-1,3-dione, 0.47 g of DIPEA, and 10 mL of DMF were sequentially added into a reaction flask. The reaction flask was treated in a microwave reactor for 10 min of reaction at 50 Hz. 20 mL of water was added into the reaction flask, and the mixture was extracted with dichloromethane (20 mL × 3). The organic phases were combined, washed once with 20 mL of water, dried over anhydrous sodium sulfate, and concentrated to give a crude product. The crude product was purified by column chromatography (eluent: dichloromethane:methanol = 10:1) to give compound C29 (0.18 g). The mass spectral data for compound C29 are, MS (ESI): (M+H)⁺, 722.3

The nuclear magnetic resonance data for compound C29 are as follows:
H NMR (400 MHz, CDCl₃) δ: 8.82-8.94 (m, 1H), 7.38-7.41 (m, 1H), 7.32-7.34 (m, 1H), 7.08-7.12 (m, 1H), 6.98-7.02 (m, 2H), 6.81-6.88 (m, 2H), 6.70-6.75 (m, 1H), 6.59-6.61 (m, 1H), 6.38-6.45 (m, 1H), 5.85 (s, 1H), 5.72-5.75 (m, 1H), 4.74-4.83 (m, 1H), 4.01-4.02 (m, 2H), 3.41-3.44 (m, 2H), 3.22-3.26 (m, 1H), 2.61-2.79 (m, 3H), 2.18-2.22 (m, 2H), 1.80-2.10 (m, 5H), 1.61-1.75 (m, 3H), 1.48-1.53 (m, 2H), 1.16-1.37 (m, 5H), 0.92-0.95 (m, 2H), 0.90 (s, 3H), 0.78 (s, 3H)

### Example 30: Synthesis of N-(3-((3-((5-(2-(3-(2,6-dioxopiperidine))-isoindole-1,3-dione))amino)propyl)oxy)phenyl)-3-oxo-4-aza-5α-androst-1-ene-17β-carboxamide (C30)

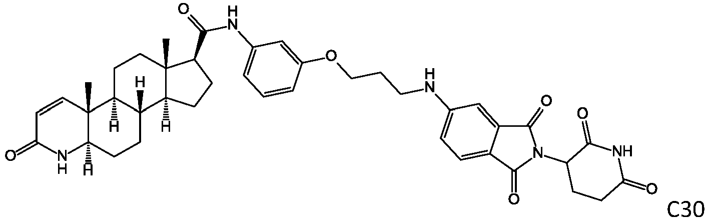

The synthesis was similar to that in Example 29, except that 4-fluoro-2-(3-(2,6-dioxopiperidine))-isoindole-1,3-dione in step 4 of Example 29 was replaced with 5-fluoro-2-(3-(2,6-dioxopiperidine))-isoindole-1,3-dione.
The mass spectral data for compound C30 are, MS (ESI): (M+H)⁺, 722.3

The nuclear magnetic resonance data for compound C30 are as follows:
H NMR (400 MHz, CDCl₃) δ: 8.85 (s, 1H), 7.45-7.47 (m, 1H), 7.28-7.30 (m, 1H), 7.10-7.12 (m, 1H), 6.98-7.02 (m, 2H), 6.89 (s, 1H), 6.79-6.83 (m, 2H), 6.70-6.72 (m, 1H), 6.54-6.57 (m, 1H), 6.38-6.42 (m, 1H), 5.76 (s, 1H), 5.70-5.72 (m, 1H), 4.75-4.81 (m, 1H), 3.99-4.01 (m, 2H), 3.39-3.43 (m, 2H), 3.25-3.28 (m, 1H), 2.65-2.81 (m, 3H), 2.10-2.25 (m, 2H), 1.65-2.01 (m, 8H), 1.48-1.55 (m, 2H), 1.11-1.37 (m, 5H), 0.90-0.95 (m, 2H), 0.90 (s, 3H), 0.76 (s, 3H)

### Example 31: Synthesis of N-(3-((4-((4-(2-(3-(2,6-dioxopiperidine))-isoindole-1,3-dione))amino)butyl)oxy)phenyl)-3-oxo-4-aza-5α-androst-1-ene-17β-carboxamide (C31)

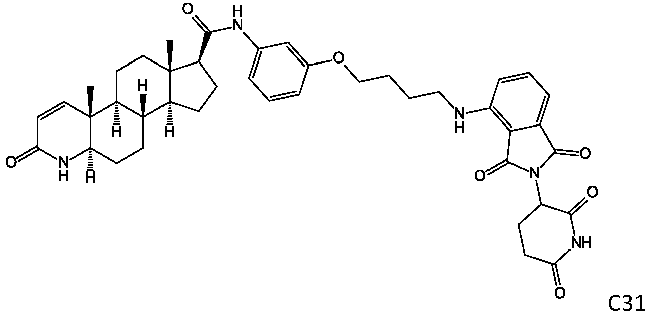

The synthesis was similar to that in Example 29.
The mass spectral data for compound C31 are, MS (ESI): (M+H)⁺, 736.4

The nuclear magnetic resonance data for compound C31 are as follows:
H NMR (400 MHz, CDCl₃) δ: 8.86-8.88 (m, 1H), 7.40-7.42 (m, 1H), 7.34-7.36 (m, 1H), 7.08-7.12 (m, 1H), 6.98-7.02 (m, 2H), 6.81-6.87 (m, 2H), 6.70-6.73 (m, 1H), 6.59-6.61 (m, 1H), 6.38-6.40 (m, 1H), 5.83 (s, 1H), 5.72-5.75 (m, 1H), 4.78-4.83 (m, 1H), 4.00-4.02 (m, 2H), 3.41-3.45 (m, 2H), 3.22-3.26 (m, 1H), 2.61-2.79 (m, 3H), 2.18-2.22 (m, 2H), 2.02-2.10 (m, 2H), 1.80-1.95 (m, 3H), 1.48-1.75 (m, 7H), 1.18-1.45 (m, 5H), 0.92-0.99 (m, 2H), 0.89 (s, 3H), 0.76 (s, 3H)

### Example 32: Synthesis of N-(3-((4-((5-(2-(3-(2,6-dioxopiperidine))-isoindole-1,3-dione))amino)butyl)oxy)phenyl)-3-oxo-4-aza-5α-androst-1-ene-17β-carboxamide (C32)

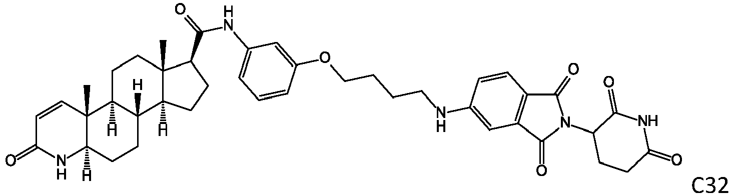

The synthesis was similar to that in Example 29.
The mass spectral data for compound C32 are, MS (ESI): (M+H)⁺, 736.4

The nuclear magnetic resonance data for compound C32 are as follows:
H NMR (400 MHz, CDCl₃) δ: 8.80 (s, 1H), 7.47-7.48 (m, 1H), 7.28-7.30 (m, 1H), 7.10-7.12 (m, 1H), 6.98-7.02 (m, 2H), 6.87 (s, 1H), 6.80-6.83 (m, 2H), 6.70-6.73 (m, 1H), 6.54-6.57 (m, 1H), 6.38-6.42 (m, 1H), 5.77 (s, 1H), 5.70-5.72 (m, 1H), 4.75-4.81 (m, 1H), 3.99-4.01 (m, 2H), 3.39-3.43 (m, 2H), 3.25-3.28 (m, 1H), 2.65-2.81 (m, 3H), 2.05-2.20 (m, 2H), 1.55-1.99 (m, 12H), 1.11-1.37 (m, 5H), 0.95-1.01 (m, 2H), 0.89 (s, 3H), 0.75 (s, 3H)

### Example 33: Synthesis of N-(3-((5-((4-(2-(3-(2,6-dioxopiperidine))-isoindole-1,3-dione))amino)pentyl)oxy)phenyl)-3-oxo-4-aza-5α-androst-1-ene-17β-carboxamide (C33)

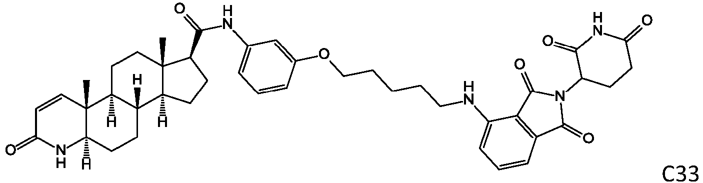

The synthesis was similar to that in Example 29.
The mass spectral data for compound C33 are, MS (ESI): (M+H)⁺, 750.4

The nuclear magnetic resonance data for compound C33 are as follows:
H NMR (400 MHz, CDCl₃) δ: 8.90-8.91 (m, 1H), 7.43-7.45 (m, 1H), 7.34-7.37 (m, 1H), 7.09-7.12 (m, 1H), 6.98-7.02 (m, 2H), 6.83-6.87 (m, 2H), 6.71-6.74 (m, 1H), 6.59-6.62 (m, 1H), 6.38-6.41 (m, 1H), 5.83 (s, 1H), 5.72-5.75 (m, 1H), 4.79-4.83 (m, 1H), 4.00-4.03 (m, 2H), 3.41-3.45 (m, 2H), 3.22-3.26 (m, 1H), 2.61-2.79 (m, 3H), 2.18-2.22 (m, 2H), 2.02-2.10 (m, 2H), 1.57-1.95 (m, 11H), 1.15-1.55 (m, 6H), 0.92-1.00 (m, 2H), 0.87 (s, 3H), 0.75 (s, 3H)

### Example 34: Synthesis of N-(3-((5-((5-(2-(3-(2,6-dioxopiperidine))-isoindole-1,3-dione))amino)pentyl)oxy)phenyl)-3-oxo-4-aza-5α-androst-1-ene-17β-carboxamide (C34)

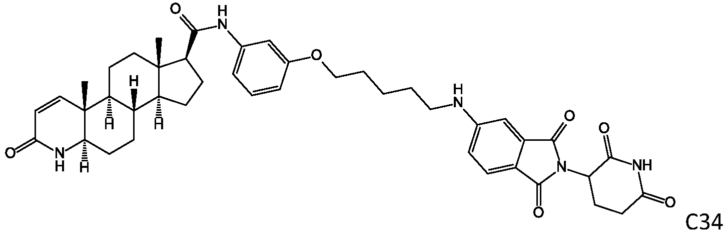

The synthesis was similar to that in Example 29.
The mass spectral data for compound C34 are, MS (ESI): (M+H)⁺, 750.4

The nuclear magnetic resonance data for compound C34 are as follows:
H NMR (400 MHz, CDCl₃) δ: 8.75 (s, 1H), 7.47-7.48 (m, 1H), 7.25-7.28 (m, 1H), 7.10-7.12 (m, 1H), 6.98-7.02 (m, 2H), 6.88 (s, 1H), 6.80-6.83 (m, 2H), 6.71-6.73 (m, 1H), 6.54-6.57 (m, 1H), 6.39-6.42 (m, 1H), 5.93 (s, 1H), 5.70-5.72 (m, 1H), 4.81-4.83 (m, 1H), 4.00-4.03 (m, 2H), 3.38-3.41 (m, 2H), 3.25-3.28 (m, 1H), 2.62-2.78 (m, 3H), 1.71-2.11 (m, 14H), 1.11-1.58 (m, 9H), 0.95-1.03 (m, 2H), 0.88 (s, 3H), 0.74 (s, 3H)

### Example 35: Synthesis of N-(3-((6-((4-(2-(3-(2,6-dioxopiperidine))-isoindole-1,3-dione))amino)hexyl)oxy)phenyl)-3-oxo-4-aza-5α-androst-1-ene-17β-carboxamide (C35)

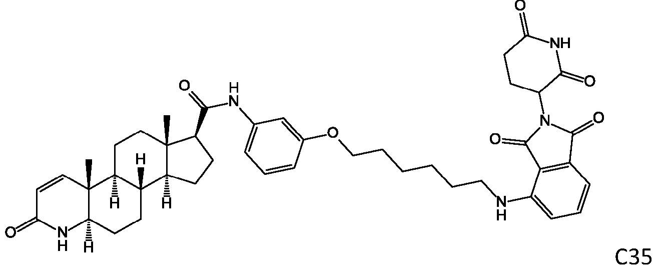

The synthesis was similar to that in Example 29.
The mass spectral data for compound C35 are, MS (ESI): (M+H)⁺, 764.4

The nuclear magnetic resonance data for compound C35 are as follows:
H NMR (400 MHz, CDCl₃) δ: 8.85-8.86 (m, 1H), 7.45-7.47 (m, 1H), 7.35-7.37 (m, 1H), 7.09-7.12 (m, 1H), 6.98-7.02 (m, 2H), 6.84-6.87 (m, 2H), 6.71-6.75 (m, 1H), 6.59-6.62 (m, 1H), 6.39-6.41 (m, 1H), 5.57 (s, 1H), 5.72-5.75 (m, 1H), 4.79-4.82 (m, 1H), 4.03-4.05 (m, 2H), 3.41-3.45 (m, 2H), 3.23-3.26 (m, 1H), 2.65-2.79 (m, 3H), 2.18-2.21 (m, 2H), 2.05-2.10 (m, 2H), 1.71-1.95 (m, 11H), 1.15-1.57 (m, 8H), 0.92-1.01 (m, 2H), 0.89 (s, 3H), 0.75 (s, 3H)

### Example 36: Synthesis of N-(3-((6-((5-(2-(3-(2,6-dioxopiperidine))-isoindole-1,3-dione))amino)hexyl)oxy)phenyl)-3-oxo-4-aza-5α-androst-1-ene-17β-carboxamide (C36)

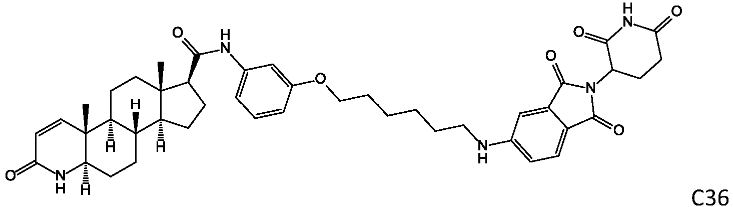

The synthesis was similar to that in Example 29.
The mass spectral data for compound C36 are, MS (ESI): (M+H)⁺, 764.4

The nuclear magnetic resonance data for compound C36 are as follows:
H NMR (400 MHz, CDCl₃) δ: 8.55 (s, 1H), 7.48-7.50 (m, 1H), 7.25-7.28 (m, 1H), 7.12-7.15 (m, 1H), 6.99-7.05 (m, 2H), 6.84 (s, 1H), 6.79-6.83 (m, 2H), 6.71-6.73 (m, 1H), 6.54-6.56 (m, 1H), 6.38-6.40 (m, 1H), 5.72-5.74 (m, 1H), 5.70 (s, 1H), 4.91-4.95 (m, 1H), 4.00-4.02 (m, 2H), 3.35-3.38 (m, 2H), 3.27-3.30 (m, 1H), 2.62-2.78 (m, 3H), 2.00-2.11 (m, 2H), 1.55-1.97 (m, 15H), 1.01-1.45 (m, 10H), 0.87 (s, 3H), 0.74 (s, 3H)

### Example 37: Synthesis of N-(3-((7-((4-(2-(3-(2,6-dioxopiperidine))-isoindole-1,3-dione))amino)heptyl)oxy)phenyl)-3-oxo-4-aza-5α-androst-1-ene-17β-carboxamide (C37)

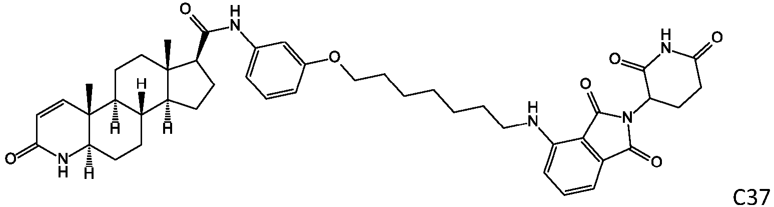

The synthesis was similar to that in Example 29.
The mass spectral data for compound C37 are, MS (ESI): (M+H)⁺, 778.4

The nuclear magnetic resonance data for compound C37 are as follows:
H NMR (400 MHz, CDCl₃) δ: 8.85-8.87 (m, 1H), 7.45-7.47 (m, 1H), 7.34-7.37 (m, 1H), 7.09-7.13 (m, 1H), 6.98-7.02 (m, 2H), 6.84-6.87 (m, 2H), 6.71-6.75 (m, 1H), 6.58-6.63 (m, 1H), 6.39-6.42 (m, 1H), 5.57 (s, 1H), 5.72-5.76 (m, 1H), 4.78-4.82 (m, 1H), 4.03-4.06 (m, 2H), 3.41-3.46 (m, 2H), 3.22-3.26 (m, 1H), 2.65-2.79 (m, 3H), 2.18-2.22 (m, 2H), 1.97-2.10 (m, 5H), 1.55-1.91 (m, 10H), 1.15-1.52 (m, 10H), 0.95-1.01 (m, 2H), 0.87 (s, 3H), 0.71 (s, 3H)

### Example 38: Synthesis of N-(3-((7-((5-(2-(3-(2,6-dioxopiperidine))-isoindole-1,3-dione))amino)heptyl)oxy)phenyl)-3-oxo-4-aza-5α-androst-1-ene-17β-carboxamide (C38)

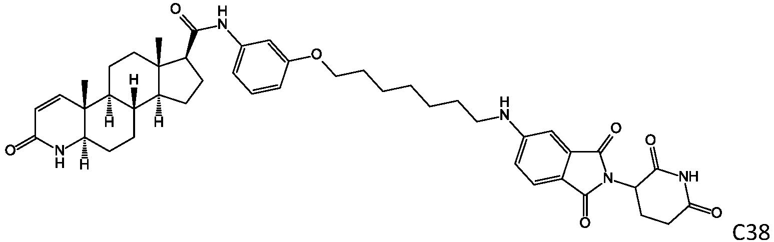

The synthesis was similar to that in Example 29.
The mass spectral data for compound C38 are, MS (ESI): (M+H)⁺, 778.4

The nuclear magnetic resonance data for compound C38 are as follows:
H NMR (400 MHz, CDCl₃) δ: 8.59 (s, 1H), 7.49-7.51 (m, 1H), 7.26-7.28 (m, 1H), 7.13-7.15 (m, 1H), 7.03-7.08 (m, 2H), 6.85 (s, 1H), 6.79-6.84 (m, 2H), 6.71-6.73 (m, 1H), 6.54-6.57 (m, 1H), 6.37-6.40 (m, 1H), 5.79 (s, 1H), 5.72-5.74 (m, 1H), 4.91-4.95 (m, 1H), 4.00-4.02 (m, 2H), 3.22-3.38 (m, 3H), 2.66-2.79 (m, 3H), 1.59-2.15 (m, 17H), 1.15-1.49 (m, 12H), 1.02-1.07 (m, 2H), 0.86 (s, 3H), 0.72 (s, 3H)

### Example 39: Synthesis of N-(3-((8-((4-(2-(3-(2,6-dioxopiperidine))-isoindole-1,3-dione))amino)octyl)oxy)phenyl)-3-oxo-4-aza-5α-androst-1-ene-17β-carboxamide (C39)

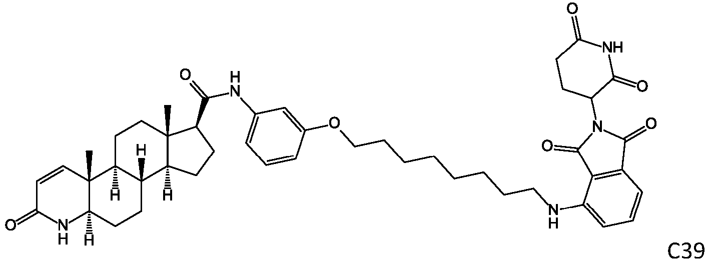

The synthesis was similar to that in Example 29.
The mass spectral data for compound C39 are, MS (ESI): (M+H)⁺, 792.4

The nuclear magnetic resonance data for compound C39 are as follows:
H NMR (400 MHz, CDCl₃) δ: 8.72-8.77 (m, 1H), 7.43-7.45 (m, 1H), 7.34-7.36 (m, 1H), 7.09-7.13 (m, 1H), 6.98-7.02 (m, 2H), 6.85-6.87 (m, 2H), 6.71-6.74 (m, 1H), 6.58-6.62 (m, 1H), 6.39-6.42 (m, 1H), 5.76 (s, 1H), 5.72-5.76 (m, 1H), 4.74-4.81 (m, 1H), 4.01-4.04 (m, 2H), 3.41-3.45 (m, 2H), 3.21-3.25 (m, 1H), 2.65-2.78 (m, 3H), 2.18-2.22 (m, 2H), 1.97-2.10 (m, 5H), 1.45-1.89 (m, 13H), 1.01-1.37 (m, 11H), 0.85 (s, 3H), 0.69 (s, 3H)

### Example 40: Synthesis of N-(3-((8-((5-(2-(3-(2,6-dioxopiperidine))-isoindole-1,3-dione))amino)octyl)oxy)phenyl)-3-oxo-4-aza-5α-androst-1-ene-17β-carboxamide (C40)

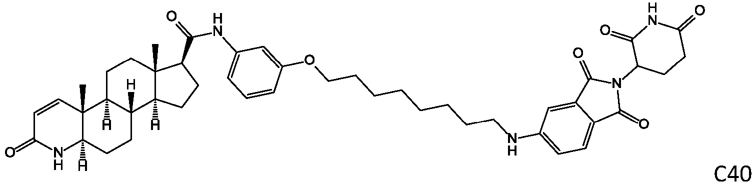

The synthesis was similar to that in Example 29.
The mass spectral data for compound C40 are, MS (ESI): (M+H)⁺, 792.4

The nuclear magnetic resonance data for compound C40 are as follows:
H NMR (400 MHz, CDCl₃) δ: 8.71 (s, 1H), 7.50-7.52 (m, 1H), 7.28-7.31 (m, 1H), 7.05-7.13 (m, 3H), 6.81 (s, 1H), 6.77-6.82 (m, 2H), 6.71-6.73 (m, 1H), 6.54-6.57 (m, 1H), 6.38-6.41 (m, 1H), 5.93 (s, 1H), 5.72-5.74 (m, 1H), 4.95-4.99 (m, 1H), 4.00-4.03 (m, 2H), 3.24-3.38 (m, 3H), 2.65-2.82 (m, 3H), 1.88-2.15 (m, 9H), 1.55-1.71 (m, 12H), 1.15-1.45 (m, 12H), 1.03-1.09 (m, 2H), 0.81 (s, 3H), 0.69 (s, 3H)

### Example 41: Synthesis of N-(3-((3-((4-(2-(3-(2,6-dioxopiperidine))-1-oxo-2,3-dihydro-1H-isoindol))amino)propyl)oxy)phenyl)-3-oxo-4-aza-5α-androst-1-ene-17β-carboxamide (C41)

### Step 1: Synthesis of N-(3-((3-((4-(2-(3-(2,6-dioxopiperidine))-3-hydroxy-1-oxo-2,3-dihydro-1H-isoindol))amino)propyl)oxy)phenyl)-3-oxo-4-aza-5α-androst-1-ene-17β-carboxamide (VIII-41)

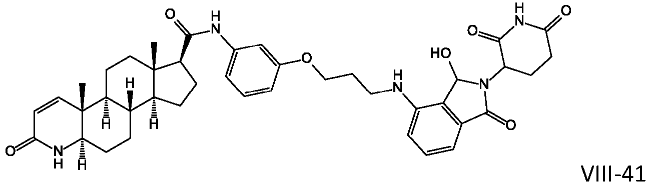

At room temperature, 1.00 g of compound C29, 0.45 g of zinc powder, and 10 mL of glacial acetic acid were sequentially added into a reaction flask, and the reaction system was heated to 70 °C and incubated for 2 h. The reaction solution was filtered before cooling, and the filtrate was concentrated to give a pale yellow solid (compound VIII-41).

### Step 2: Synthesis of N-(3-((3-((4-(2-(3-(2,6-dioxopiperidine))-1-oxo-2,3-dihydro-1H-isoindol))amino)propyl)oxy)phenyl)-3-oxo-4-aza-5α-androst-1-ene-17β-carboxamide (C41)

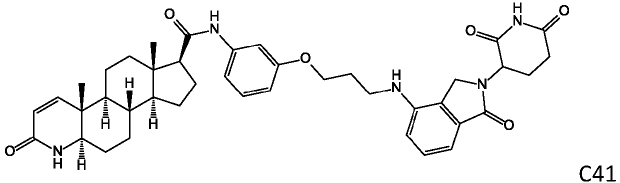

Compound VIII-41 obtained in step 1, 5 mL of triethylsilane, and 10 mL of trifluoroacetic acid were added into a reaction flask, and the reaction system was heated to 60 °C and incubated for 3 h. The reaction solution was purified by column chromatography (eluent: dichloromethane:methanol = 10:1) to give compound C41 (20 mg).
The mass spectral data for compound C41 are, MS (ESI): (M+H)⁺, 708.4

The nuclear magnetic resonance data for compound C41 are as follows:
H NMR (400 MHz, CDCl₃) δ: 8.91-8.94 (m, 1H), 7.28-7.34 (m, 2H), 7.05-7.07 (m, 1H), 6.98-7.05 (m, 2H), 6.81-6.85 (m, 2H), 6.72-6.75 (m, 1H), 6.59-6.61 (m, 1H), 6.38-6.40 (m, 1H), 5.88 (s, 1H), 5.72-5.75 (m, 1H), 4.83-4.86 (m, 1H), 4.23-4.38 (m, 2H), 3.95-3.97 (m, 2H), 3.39-3.41 (m, 2H), 3.20-3.22 (m, 1H), 2.55-2.75 (m, 3H), 2.10-2.17 (m, 2H), 1.88-2.05 (m, 5H), 1.67-1.75 (m, 3H), 1.14-1.53 (m, 7H), 0.92-0.95 (m, 2H), 0.90 (s, 3H), 0.78 (s, 3H)

### Example 42: Synthesis of N-(3-((4-methyl-1-(4-(2-(3-(2,6-dioxopiperidine))-isoindole-1,3-dione))-piperidinyl)oxy)phenyl)-3-oxo-4-aza-5α-androst-1-ene-17β-carboxamide (C42)

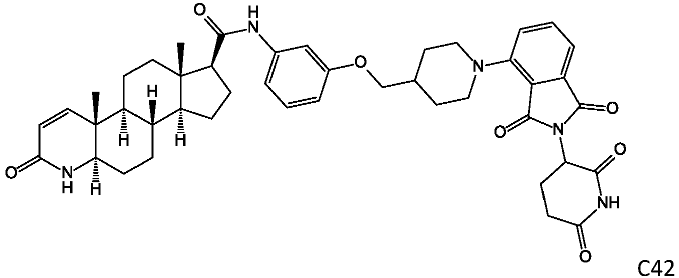

The synthesis was similar to that in Example 29.
The mass spectral data for compound C42 are, MS (ESI): (M+H)⁺, 762.4

The nuclear magnetic resonance data for compound C42 are as follows:
H NMR (400 MHz, CDCl₃) δ: 8.84-8.86 (m, 1H), 7.45-7.48 (m, 1H), 7.25-7.27 (m, 1H), 7.10-7.13 (m, 1H), 6.95-7.03 (m, 2H), 6.82-6.85 (m, 2H), 6.70-6.73 (m, 1H), 6.59-6.61 (m, 1H), 6.38-6.41 (m, 1H), 5.79 (s, 1H), 5.71-5.74 (m, 1H), 4.79-4.83 (m, 1H), 4.01-4.03 (m, 2H), 3.25-3.47 (m, 5H), 2.60-2.79 (m, 3H), 2.15-2.25 (m, 2H), 1.65-2.10 (m, 9H), 1.48-1.53 (m, 2H), 1.16-1.37 (m, 7H), 0.95-0.99 (m, 2H), 0.91 (s, 3H), 0.75 (s, 3H)

### Example 43: Synthesis of N-(3-((4-methyl-1-(5-(2-(3-(2,6-dioxopiperidine))-isoindole-1,3-dione))-piperidinyl)oxy)phenyl)-3-oxo-4-aza-5α-androst-1-ene-17β-carboxamide (C43)

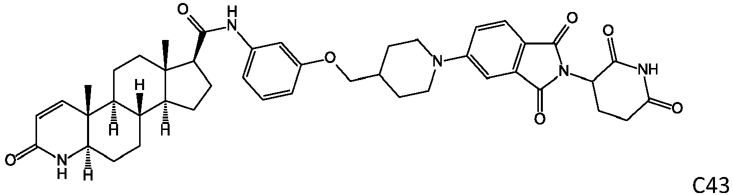

The synthesis was similar to that in Example 29.
The mass spectral data for compound C43 are, MS (ESI): (M+H)⁺, 762.4

The nuclear magnetic resonance data for compound C43 are as follows:
H NMR (400 MHz, CDCl₃) δ: 8.81-8.85 (m, 1H), 7.33-7.35 (m, 1H), 7.21-7.23 (m, 1H), 7.09-7.12 (m, 1H), 7.00-7.02 (m, 2H), 6.93 (s, 1H), 6.80-6.84 (m, 2H), 6.71-6.73 (m, 1H), 6.55-6.57 (m, 1H), 6.38-6.42 (m, 1H), 5.71-5.73 (m, 1H), 5.65 (s, 1H), 4.77-4.80 (m, 1H), 4.05-4.07 (m, 2H), 3.19-3.43 (m, 5H), 2.60-2.79 (m, 3H), 2.07-2.22 (m, 2H), 1.48-2.01 (m, 11H), 1.10-1.37 (m, 7H), 0.96-1.00 (m, 2H), 0.90 (s, 3H), 0.75 (s, 3H)

### Example 44: Synthesis of 1-(4-(1-(3-(3-oxo-4-aza-5α-androst-1-ene-17β-carboxamido)phenyl)piperidine))methyl-4-(4-(2-(3-(2,6-dioxopiperidine))-isoindole-1,3-dione))piperazine (C44)

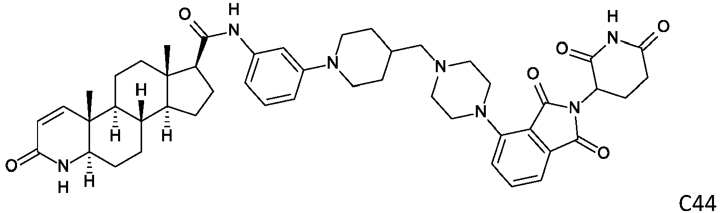

### Step 1: Synthesis of N-(3-hydroxyphenyl)-3-oxo-4-aza-5α-androst-1-ene-17β-carboxamide (VI-44)

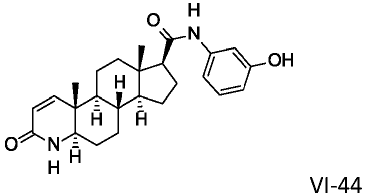

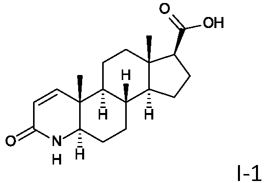

At room temperature, 2.00 g of compound I-1, 0.72 g of 3-hydroxyaniline, 6.55 g of pyBoP, and 50 mL of DMF were sequentially added into a reaction flask, and the mixture was stirred for 5 min before 2.44 g of DIPEA was added dropwise. The reaction system was incubated overnight. The next day, 50 mL of water was added into the reaction flask. The reaction solution was extracted with ethyl acetate (50 mL × 3). The organic phase was sequentially washed once with 50 mL of 1 mol/L aqueous hydrochloric acid, 50 mL of saturated aqueous sodium carbonate, and 50 mL of saturated aqueous sodium chloride, dried over anhydrous sodium sulfate, and concentrated to give a black oily substance (2.56 g). The oily substance was purified by column chromatography (eluent: dichloromethane:methanol = 20:1) to give compound VI-44 (1.51 g).

### Step 2: Synthesis of phenyl 3-(3-oxo-4-aza-5α-androst-1-ene-17β-carboxamido)-nonafluorobutane-1-sulfonate (VII-44)

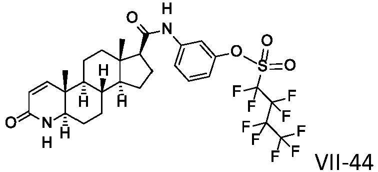

In a nitrogen atmosphere at room temperature, 1.00 g of compound VI-44, 1.00 g of nonafluoro-1-butanesulfonyl fluoride, 2.15 g of cesium carbonate, and 50 mL of DMF were sequentially added into a reaction flask, and the reaction system was incubated at 70 °C for 5 h. The reaction solution was extracted with ethyl acetate (50 mL × 3). The organic phase was sequentially washed once with 50 mL of water and 50 mL of saturated aqueous sodium chloride, dried over anhydrous sodium sulfate, and concentrated to give a black oily substance. The oily substance was purified by column chromatography (eluent: dichloromethane:methanol = 20:1) to give compound VII-44 (1.24 g).

### Step 3: Synthesis of 1-(4-(1-(3-(3-oxo-4-aza-5α-androst-1-ene-17β-carboxamido)phenyl)piperidine))methyl-4-Boc-piperazine (IV-44)

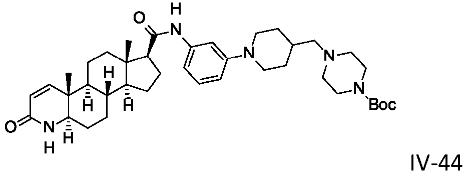

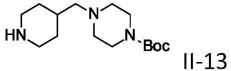

In a nitrogen atmosphere at room temperature, 1.00 g of compound VII-44, 1.00 g of compound II-13, 1.00 g of cesium carbonate, 0.4 g of XPhoS, 0.1 g of palladium acetate, and 20 mL of DMF were sequentially added into a reaction flask. The reaction flask was treated in a microwave reactor for 8 min of reaction at 60 Hz. The reaction solution was extracted with ethyl acetate (50 mL × 3). The organic phase was sequentially washed once with 50 mL of water and 50 mL of saturated aqueous sodium chloride, dried over anhydrous sodium sulfate, and concentrated to give a black oily substance. The oily substance was purified by column chromatography (eluent: = 20:1) to give compound IV-44 (40 mg).

### Step 4: Synthesis of 1-(4-(1-(3-(3-oxo-4-aza-5α-androst-1-ene-17β-carboxamido)phenyl)piperidine))methyl-piperazine (V-44)

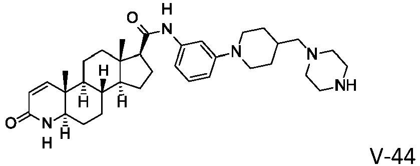

At room temperature, 40 mg of compound IV-44 and 5 mL of dichloromethane were added into a reaction flask, before 5 mL of trifluoroacetic acid was dropwise added. The reaction system was incubated for 10 min. The reaction solution was concentrated to give an oily substance (crude compound V-44), which was directly used in the next reaction without purification.

### Step 5: Synthesis of 1-(4-(1-(3-(3-oxo-4-aza-5α-androst-1-ene-17β-carboxamido)phenyl)piperidine))methyl-4-(4-(2-(3-(2,6-dioxopiperidine))-isoindole-1,3-dione))piperazine (C44)

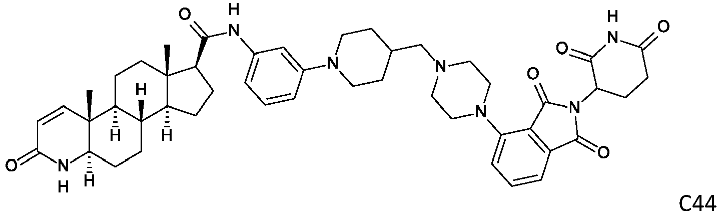

At room temperature, the oily substance obtained in step 4 (crude compound V-44), 20 mg of 4-fluoro-2-(3-(2,6-dioxopiperidine))-isoindole-1,3-dione, 20 mg of DIPEA, and 5 mL of DMF were sequentially added into a reaction flask. The reaction flask was treated in a microwave reactor for 10 min of reaction at 50 Hz. 20 mL of water was added into the reaction flask to quench the reaction, and the mixture was extracted with dichloromethane (20 mL × 3). The organic phase was washed once with 20 mL of water, dried over anhydrous sodium sulfate, and concentrated to give a crude product. The crude product was purified by column chromatography (eluent: dichloromethane:methanol = 10:1) to give compound C44 (2 mg).
The mass spectral data for compound C44 are, MS (ESI): (M+H)⁺, 830.5

The nuclear magnetic resonance data for compound C44 are as follows:
H NMR (400 MHz, CDCl₃) δ: 8.85 (s, 1H), 7.39-7.41 (m, 1H), 7.10-7.12 (m, 2H), 6.94-7.00 (m, 2H), 6.76-6.79 (m, 1H), 6.68-6.70 (m, 1H), 6.21-6.24 (m, 1H), 5.87 (s, 1H), 5.70-5.73 (d, 1H), 4.89-4.92 (m, 1H), 3.41-3.59 (m, 4H), 3.10-3.37 (m, 5H), 2.62-2.80 (m, 5H), 2.07-2.38 (m, 6H), 1.50-1.93 (m, 10H), 1.15-1.49 (m, 8H), 0.98-1.06 (m, 2H), 0.93 (s, 3H), 0.70 (s, 3H)

### Example 45: Synthesis of 1-(4-(1-(3-(3-oxo-4-aza-5α-androst-1-ene-17β-carboxamido)phenyl)piperidine)methyl)-4-(5-(2-(3-(2,6-dioxopiperidine))-isoindole-1,3-dione))piperazine (C45)

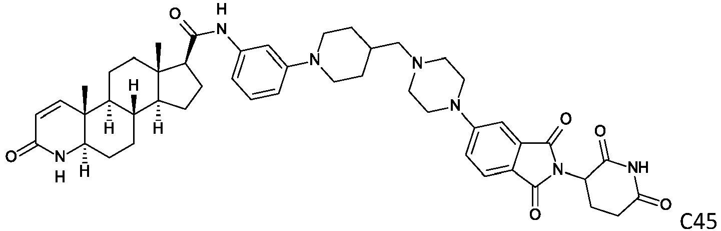

The synthesis was similar to that in Example 44, except that 4-fluoro-2-(3-(2,6-dioxopiperidine))-isoindole-1,3-dione in step 5 of Example 44 was replaced with 5-fluoro-2-(3-(2,6-dioxopiperidine))-isoindole-1,3-dione.
The mass spectral data for compound C45 are, MS (ESI): (M+H)⁺, 708.3

The nuclear magnetic resonance data for compound C45 are as follows:
H NMR (400 MHz, CDCl₃) δ: 8.75-8.79 (m, 1H), 7.35-7.37 (m, 1H), 7.08-7.09 (m, 1H), 7.09-7.12 (m, 1H), 7.00-7.02 (m, 2H), 6.91 (s, 1H), 6.79-6.83 (m, 2H), 6.70-6.72 (m, 1H), 6.38-6.42 (m, 1H), 5.75 (s, 1H), 5.71-5.73 (m, 1H), 4.83-4.85 (m, 1H), 3.45-3.64 (m, 2H), 3.15-3.49 (m, 7H), 2.59-2.75 (m, 5H), 2.07-2.35 (m, 4H), 2.07-2.15 (m, 2H), 1.55-2.01 (m, 10H), 1.07-1.45 (m, 8H), 0.95-1.01 (m, 2H), 0.95 (s, 3H), 0.71 (s, 3H)

### Example 46: Synthesis of N-(3-((3-((4-(2-(3-(2,6-dioxopiperidine))-isoindole-1,3-dione))oxy)propyl)oxy)phenyl)-3-oxo-4-aza-5α-androst-1-ene-17β-carboxamide (C46)

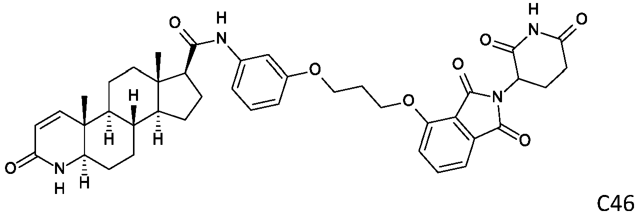

### Step 1: Synthesis of N-(3-hydroxyphenyl)-3-oxo-4-aza-5α-androst-1-ene-17β-carboxamide (X-46)

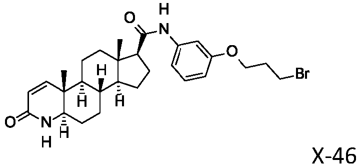

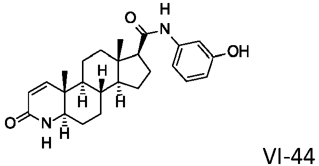

In a nitrogen atmosphere at room temperature, 2.00 g of compound VI-44, 2.00 g of 1,3-dibromopropane, 3.20 g of cesium carbonate, and 50 mL of DMF were sequentially added into a reaction flask, and the reaction system was incubated at 70 °C for 5 h. The reaction solution was extracted with ethyl acetate (100 mL × 3). The organic phase was sequentially washed once with 50 mL of water and 50 mL of saturated aqueous sodium chloride, dried over anhydrous sodium sulfate, and concentrated to give a black oily substance. The oily substance was purified by silica gel column chromatography (eluent: dichloromethane:methanol = 20:1) to give compound X-46 (1g).

### Step 2: Synthesis of N-(3-((3-((4-(2-(3-(2,6-dioxopiperidine))-isoindole-1,3-dione))oxy)propyl)oxy)phenyl)-3-oxo-4-aza-5α-androst-1-ene-17β-carboxamide (C46)

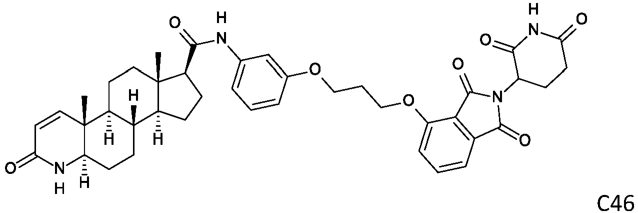

In a nitrogen atmosphere at room temperature, 1 g of compound X-46, 0.57 g of 4-hydroxy-2-(3-(2,6-dioxopiperidine))-isoindole-1,3-dione, 0.92 g of cesium carbonate, 0.3 g of potassium iodide, and 50 mL of DMF were added to a reaction flask, and the reaction system was incubated at 70 °C for 5 h. The reaction solution was extracted with ethyl acetate (100 mL × 3). The organic phase was sequentially washed once with 50 mL of water and 50 mL of saturated aqueous sodium chloride, dried over anhydrous sodium sulfate, and concentrated to give a black oily substance. The oily substance was purified by silica gel column chromatography (eluent: dichloromethane:methanol = 20:1) to give compound C46 (0.2 g).
The mass spectral data for compound C46 are, MS (ESI): (M+H)⁺, 723.3

The nuclear magnetic resonance data for compound C46 are as follows:
H NMR (400 MHz, CDCl₃) δ: 7.48-7.60 (m, 2H), 7.35-7.42 (m, 1H), 6.91-7.17 (m, 3H), 6.66-6.69 (m, 1H), 6.52-6.58 (m, 1H), 6.25-6.28 (m, 1H), 5.59-5.61 (m, 1H), 4.83-4.86 (m, 1H), 4.05-4.26 (m, 2H), 3.85-3.88 (m, 2H), 3.93-3.96 (m, 1H), 2.65-2.87 (m, 3H), 2.17-2.04 (m, 3H), 1.89-1.99 (m, 3H), 1.55-1.63 (m, 5H), 1.18-1.40 (m, 5H), 0.95-1.05 (m, 3H), 0.91 (s, 3H), 0.69 (s, 3H)

### Example 47: Synthesis of N-(5-(2-((2-((5-(2-(3-(2,6-dioxopiperidine))-isoindole-1,3-dione))amino)ethyl)oxy)pyridine))-3-oxo-4-aza-5α-androst-1-ene-17β-carboxamide (C47)

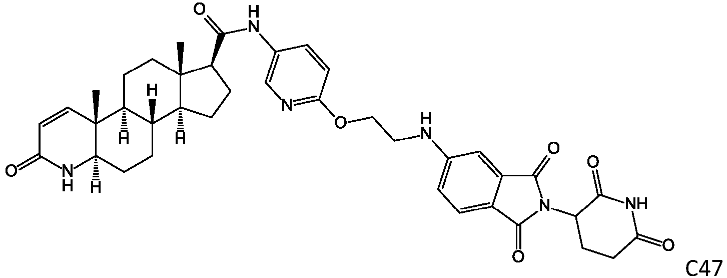

The synthesis was similar to that in Example 17.
The mass spectral data for compound C47 are, MS (ESI): (M+H)⁺, 709.3

The nuclear magnetic resonance data for compound C47 are as follows:
H NMR (400 MHz, CDCl₃) δ: 8.95-8.96 (m, 1H), 7.99 (s, 1H), 7.45-7.51 (m, 2H), 7.17-7.19 (m, 1H), 7.02-7.05 (m, 2H), 6.95 (s, 1H), 6.69-6.71 (m, 2H), 5.75-5.77 (m, 1H), 5.55 (s, 1H), 4.94-4.97 (m, 1H), 3.99-4.02 (m, 2H), 3.25-3.31 (m, 2H), 3.19-3.20 (m, 1H), 2.69-2.77 (m, 3H), 2.01-2.11 (m, 2H), 1.57-1.93 (m, 7H), 1.15-1.47 (m, 6H), 0.95-1.03 (m, 2H), 0.99 (s, 3H), 0.75 (s, 3H)

### Example 48: Synthesis of N-(3-(4-(((4-(2-(3-(2,6-dioxopiperidine))-isoindole-1,3-dione))amino)methyl)piperidine)phenyl)-3-oxo-4-aza-5α-androst-1-ene-17β-carboxamide (C48)

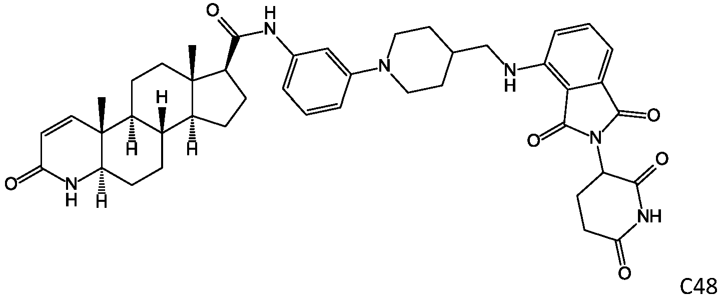

The synthesis was similar to that in Example 56.
The mass spectral data for compound C48 are, MS (ESI): (M+H)⁺, 761.4

The nuclear magnetic resonance data for compound C48 are as follows:
H NMR (400 MHz, CDCl₃) δ: 8.71-8.73 (m, 1H), 7.21-7.25 (m, 1H), 7.10-7.13 (m, 2H), 6.95-7.00 (m, 2H), 6.77-6.80 (m, 1H), 6.68-6.70 (m, 1H), 6.57-6.59 (m, 1H), 6.21-6.24 (m, 2H), 5.88 (s, 1H), 5.71-5.74 (m, 1H), 4.83-4.86 (m, 1H), 3.07-3.38 (m, 7H), 2.60-2.78 (m, 3H), 2.15-2.20 (m, 2H), 1.96-2.06 (m, 2H), 1.60-1.88 (m, 10H), 1.48-1.55 (m, 2H), 1.08-1.40 (m, 8H), 0.88 (s, 3H), 0.70 (s, 3H)

### Example 49: Synthesis of N-(3-((3-((4-(2-(3-(1-pivaloyloxymethyl-2,6-dioxopiperidine))-isoindole-1,3-dione))amino)propyl)oxy)phenyl)-3-oxo-4-aza-5α-androst-1-ene-17β-carboxamide (C49)

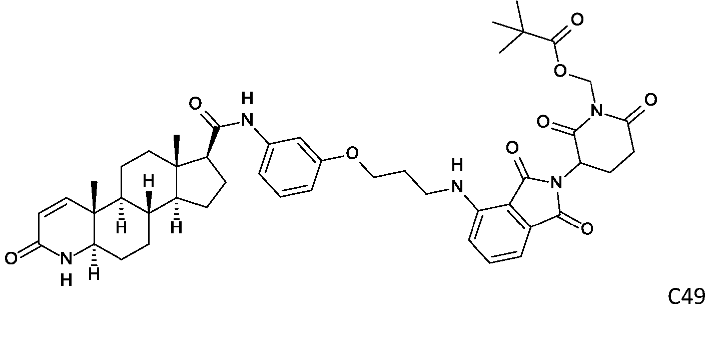

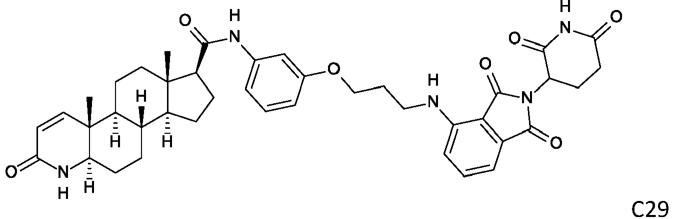

In a nitrogen atmosphere at room temperature, 0.50 g of compound C29, 0.12 g of chloromethyl 2,2-dimethylpropionate, 0.11 g of potassium iodide, 0.19 g of potassium carbonate, and 15 mL of DMF were sequentially added into a reaction flask, and the reaction system was incubated at 70 °C for 5 h. After the reaction was completed, the reaction solution was extracted with ethyl acetate (50 mL × 3). The aqueous phase was washed once with 50 mL of water and 50 mL of saturated aqueous sodium chloride, and the organic phases were combined, dried over anhydrous sodium sulfate, and concentrated to give an oily substance. The oily substance was purified by column chromatography (eluent: dichloromethane:methanol = 15:1) to give compound C49 (82 mg).
The mass spectral data for compound C49 are, MS (ESI): (M+H)⁺, 835.4

The nuclear magnetic resonance data for compound C49 are as follows:
H NMR (400 MHz, CDCl₃) δ: 8.88-8.90 (m, 1H), 7.43-7.45 (m, 1H), 7.33-7.34 (m, 1H), 7.09-7.12 (m, 1H), 6.98-7.01 (m, 2H), 6.84-6.89 (m, 2H), 6.71-6.74 (m, 1H), 6.59-6.61 (m, 1H), 6.38-6.45 (m, 1H), 5.89 (s, 1H), 5.72-5.75 (m, 1H), 4.95-4.99 (m, 1H), 4.85 (s, 2H), 4.01-4.02 (m, 2H), 3.41-3.44 (m, 2H), 3.22-3.26 (m, 1H), 2.61-2.79 (m, 3H), 2.11-2.21 (m, 3H), 1.66-2.05 (m, 7H), 1.17-1.55 (m, 7H), 1.11 (s, 9H), 0.95-1.02 (m, 2H), 0.91 (s, 3H), 0.77 (s, 3H)

### Example 50: Synthesis of N-(3-((3-((4-(2-(3-(2,6-dioxopiperidine))-isoindole-1,3-dione))amino)propyl)amino)phenyl)-3-oxo-4-aza-5α-androst-1-ene-17β-carboxamide (C50)

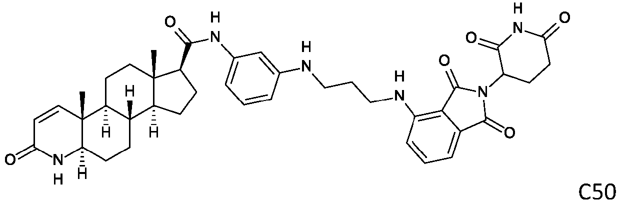

### Step 1: Synthesis of 3-((3-(N-Boc-amino)propyl)amino)aniline (II-50)

### a. Synthesis of 3-((3-(N-Boc-amino)propyl)amino)nitrobenzene (II-50-1)

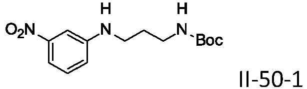

At room temperature, 2.00 g of 3-nitroaniline, 3.01 g of *N*-Boc-3-aminopropionaldehyde, 40 mL of DME, and 0.65 g of glacial acetic acid were sequentially added to a 100-mL three-necked flask, before 6.14 g of STAB was added with stirring. The system was heated to 40 °C and incubated for 3 h. The reaction solution was concentrated to give a yellow solid, and the solid was purified by column chromatography (eluent: petroleum ether:methyl tert-butyl ether = 3:1) to give compound II-50-1 (1.81 g).

### b. Synthesis of 3-((3-(N-Boc-amino)propyl)amino)aniline (II-50)

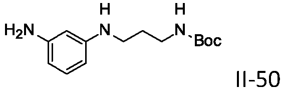

At room temperature, 1.81 g of compound II-50-1, 10 mL of methanol, and 100 mg of 5% palladium on carbon were sequentially added into a 100-mL hydrogenation reactor for 1 h of hydrogenation reaction at 25 °C. When the reaction was completed, the reaction solution was concentrated to give a gray oily substance (crude compound II-50, 1.54 g).

### Step 2: Synthesis of N-(3-((3-(N-Boc-amino)propyl)amino)phenyl)-3-oxo-4-aza-5α-androst-1-ene-17β-carboxamide (IV-50)

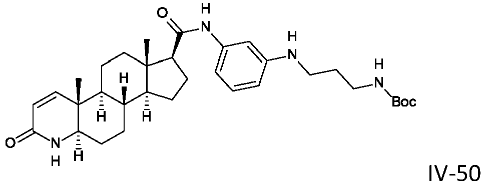

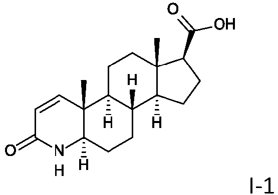

In a nitrogen atmosphere at room temperature, 2.19 g of compound I-1, 1.54 g of crude compound II-50, 5.41 g of pyBoP, and 50 mL of DMF were sequentially added into a 100-mL three-necked flask, and the mixture was stirred for 5 min. 2.68 g of DIPEA was added dropwise to the reaction system, and the reaction system was incubated overnight. 70 mL of water was added to the reaction solution, and the mixture was extracted with ethyl acetate (70 mL × 3). The organic phases were combined, sequentially washed once with 70 mL of 1 mol/L aqueous hydrochloric acid, 70 mL of saturated aqueous sodium carbonate, and 70 mL of saturated aqueous sodium chloride, dried over anhydrous sodium sulfate, and concentrated to give a black oily substance (crude compound IV-50, 4.73 g).

### Step 3: Synthesis of N-(3-((3-aminopropyl)amino)phenyl)-3-oxo-4-aza-5α-androst-1-ene-17β-carboxamide (V-50)

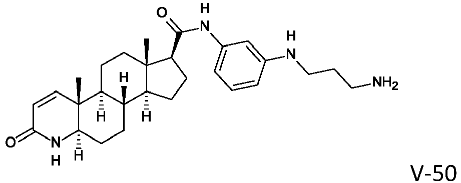

At room temperature, 4.73 g of crude compound IV-50 and 20 mL of dichloromethane were added into a 50-mL three-necked flask, before 20 mL of trifluoroacetic acid was dropwise added. The reaction solution was concentrated to give an oily substance (crude compound V-50), which was directly used in the next reaction without purification.

### Step 4: Synthesis of N-(3-((3-((4-(2-(3-(2,6-dioxopiperidine))-isoindole-1,3-dione))amino)propyl)amino)phenyl)-3-oxo-4-aza-5α-androst-1-ene-17β-carboxamide (C50)

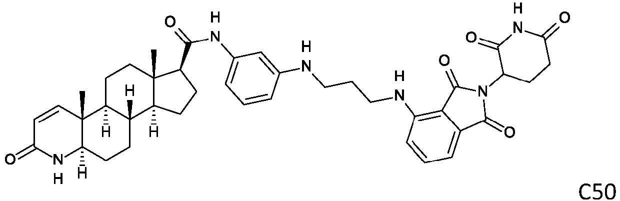

At room temperature, the oily substance obtained in step 3 (crude compound V-50), 2.15 g of 4-fluoro-2-(3-(2,6-dioxopiperidine))-isoindole-1,3-dione, 3.36 g of DIPEA, and 20 mL of DMF were sequentially added into a reaction flask. The reaction flask was treated in a microwave reactor for 10 min of reaction at 50 Hz. 30 mL of water was added into the reaction flask, and the mixture was extracted with dichloromethane (30 mL × 3). The organic phases were combined, washed once with 30 mL of water, dried over anhydrous sodium sulfate, and concentrated to give a crude product. The crude product was purified by column chromatography (eluent: dichloromethane:methanol = 10:1) to give compound C50 (1.87 g).
The mass spectral data for compound C50 are, MS (ESI): (M+H)⁺, 721.4

The nuclear magnetic resonance data for compound C50 are as follows:
H NMR (400 MHz, CDCl₃) δ: 8.85-8.87 (m, 1H), 7.37-7.40 (m, 1H), 7.09-7.11 (m, 2H), 6.94-7.00 (m, 2H), 6.76-6.79 (d, 1H, J=12Hz), 6.68-6.70 (d, 1H, J=12Hz), 6.56-6.58 (m, 1H), 6.21-6.24 (m, 2H), 5.81 (s, 1H), 5.71-5.74 (m, 1H), 4.81-4.85 (m, 1H), 3.05-3.15 (m, 5H), 2.60-2.78 (m, 3H), 2.15-2.20 (m, 2H), 1.96-2.06 (m, 2H), 1.83-1.88 (m, 2H), 1.60-1.73 (m, 5H), 1.48-1.55 (m, 2H), 1.08-1.40 (m, 6H), 0.83 (s, 3H), 0.68 (s, 3H)

### Example 51: Synthesis of N-(3-((3-((4-(2-(3-(2,6-dioxopiperidine))-isoindole-1,3-dione))amino)propyl)oxy)-4-chloro-phenyl)-3-oxo-4-aza-5α-androst-1-ene-17β-carboxamide (C51)

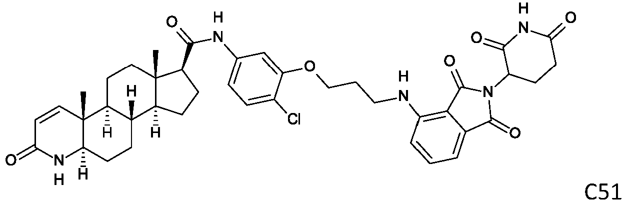

The synthesis was similar to that in Example 29.
The mass spectral data for compound C51 are, MS (ESI): (M+H)⁺, 756.3

The nuclear magnetic resonance data for compound C51 are as follows:
H NMR (400 MHz, CDCl₃) δ: 8.69 (s, 1H), 7.41-7.43 (m, 1H), 7.32-7.34 (m, 1H), 6.98-7.05 (m, 2H), 6.82-6.86 (m, 2H), 6.70-6.73 (m, 1H), 6.59-6.61 (m, 1H), 6.38-6.41 (m, 1H), 5.83 (s, 1H), 5.73-5.75 (m, 1H), 4.84-4.87 (m, 1H), 4.00-4.02 (m, 2H), 3.42-3.45 (m, 2H), 3.23-3.26 (m, 1H), 2.63-2.76 (m, 3H), 2.12-2.22 (m, 2H), 1.80-2.10 (m, 5H), 1.61-1.75 (m, 3H), 1.49-1.53 (m, 2H), 1.13-1.37 (m, 5H), 0.92-0.99 (m, 2H), 0.91 (s, 3H), 0.78 (s, 3H)

### Example 52: Synthesis of N-(3-((3-((4-(2-(3-(2,6-dioxopiperidine))-isoindole-1,3-dione))amino)propyl)oxy)-4-methyl-phenyl)-3-oxo-4-aza-5α-androst-1-ene-17β-carboxamide (C52)

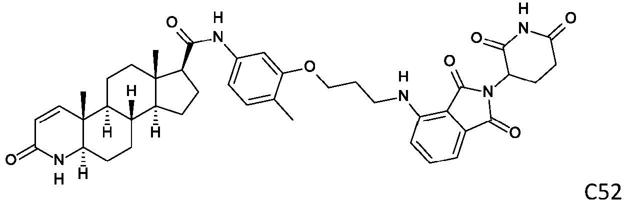

The synthesis was similar to that in Example 29.
The mass spectral data for compound C52 are, MS (ESI): (M+H)⁺, 736.4

The nuclear magnetic resonance data for compound C52 are as follows:
H NMR (400 MHz, CDCl₃) δ: 8.82-8.94 (m, 1H), 7.40-7.43 (m, 1H), 7.32-7.35 (m, 1H), 7.02-7.10 (m, 2H), 6.82-6.88 (m, 2H), 6.70-6.74 (m, 1H), 6.59-6.61 (m, 1H), 6.38-6.42 (m, 1H), 5.89 (s, 1H), 5.75-5.77 (m, 1H), 4.79-4.83 (m, 1H), 4.03-4.05 (m, 2H), 3.41-3.44 (m, 2H), 3.21-3.28 (m, 1H), 2.59-2.75 (m, 3H), 2.38 (s, 3H), 2.16-2.22 (m, 2H), 1.80-2.10 (m, 5H), 1.61-1.75 (m, 3H), 1.16-1.55 (m, 7H), 0.92-0.95 (m, 2H), 0.89 (s, 3H), 0.74 (s, 3H)

### Example 53: Synthesis of N-(2-(4-((3-((4-(2-(3-(2,6-dioxopiperidine))-isoindole-1,3-dione))amino)propyl)oxy)-pyridine))-3-oxo-4-aza-5α-androst-1-ene-17β-carboxamide (C53)

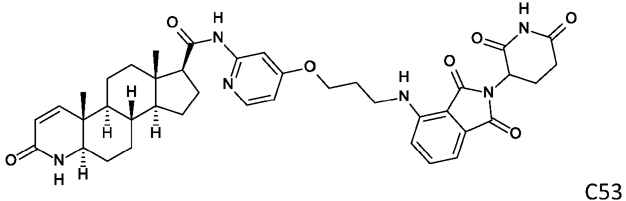

The synthesis was similar to that in Example 29.
The mass spectral data for compound C53 are, MS (ESI): (M+H)⁺, 723.3

The nuclear magnetic resonance data for compound C53 are as follows:
H NMR (400 MHz, CDCl₃) δ: 8.82-8.94 (m, 1H), 8.05-8.07 (m, 1H), 7.75-7.78 (m, 1H), 7.49-7.51 (m, 1H), 7.18-7.12 (m, 1H), 6.98-7.02 (m, 1H), 6.81-6.83 (m, 1H), 6.77-6.78 (m, 1H), 6.59-6.61 (m, 1H), 6.41-6.43 (m, 1H), 5.98 (s, 1H), 5.85-5.88 (m, 1H), 4.94-4.98 (m, 1H), 4.12-4.14 (m, 2H), 3.47-3.49 (m, 2H), 3.25-3.27 (m, 1H), 2.61-2.77 (m, 3H), 2.15-2.25 (m, 2H), 1.80-2.10 (m, 5H), 1.48-1.77 (m, 5H), 1.16-1.37 (m, 5H), 0.95-1.01 (m, 2H), 0.93 (s, 3H), 0.79 (s, 3H)

### Example 54: Synthesis of N-(3-((4-((4-(2-(3-(2,6-dioxopiperidine))-isoindole-1,3-dione))amino)cyclohexyl)amino)phenyl)-3-oxo-4-aza-5α-androst-1-ene-17β-carboxamide (C54)

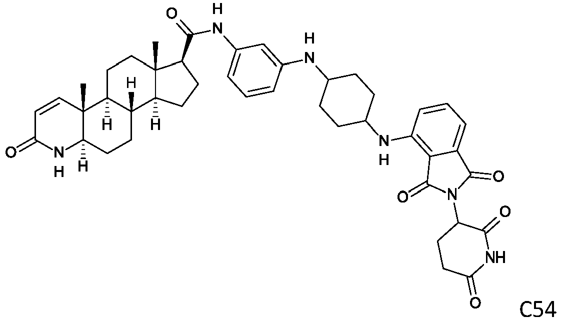

The synthesis was similar to that in Example 56.
The mass spectral data for compound C54 are, MS (ESI): (M+H)⁺, 761.4

The nuclear magnetic resonance data for compound C54 are as follows:
H NMR (400 MHz, CDCl₃) δ: 8.54-8.64 (m, 1H), 7.46-7.49 (m, 1H), 7.28-7.35 (m, 1H), 6.97-7.18 (m, 3H), 6.83-6.89 (m, 1H), 6.41-6.55 (m, 1H), 6.21-6.35 (m, 2H), 6.15-6.18 (m, 1H), 5.83 (m, 1H), 5.61 (s, 1H), 4.88-4.91 (m, 1H), 3.65-3.75 (m, 1H), 3.31-3.47 (m, 2H), 2.62-2.87 (m, 3H), 2.01-2.31 (m, 5H), 1.75-1.95 (m, 8H), 1.15-1.66 (m, 9H), 1.02-1.09 (m, 2H), 0.96 (s, 3H), 0.77 (s, 3H)

### Example 55: Synthesis of N-(3-((3-((4-(2-(3-(2,6-dioxopiperidine))-isoindole-1,3-dione))amino)propyl)oxy)-5-chloro-phenyl)-3-oxo-4-aza-5α-androst-1-ene-17β-carboxamide (C55)

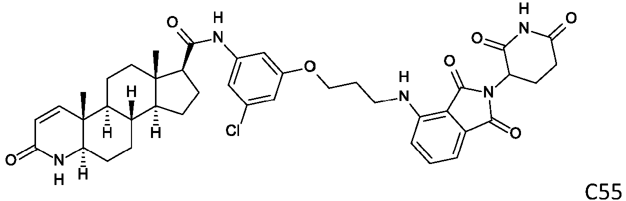

The synthesis was similar to that in Example 29.
The mass spectral data for compound C55 are, MS (ESI): (M+H)⁺, 756.3

The nuclear magnetic resonance data for compound C55 are as follows:
H NMR (400 MHz, CDCl₃) δ: 8.67 (s, 1H), 7.41-7.43 (m, 1H), 7.20-7.23 (m, 1H), 7.02-7.09 (m, 2H), 6.81-6.86 (m, 2H), 6.71-6.74 (m, 1H), 6.55-6.58 (m, 1H), 6.39-6.43 (m, 1H), 5.89 (s, 1H), 5.78-5.81 (m, 1H), 4.87-4.50 (m, 1H), 4.05-4.07 (m, 2H), 3.43-3.46 (m, 2H), 3.23-3.26 (m, 1H), 2.65-2.79 (m, 3H), 1.95-2.15 (m, 7H), 1.71-1.81 (m, 3H), 1.13-1.59 (m, 7H), 0.95-1.05 (m, 2H), 0.95 (s, 3H), 0.79 (s, 3H)

### Example 56: Synthesis of N-(3-((2-((4-(2-(3-(2,6-dioxopiperidine))-isoindole-1,3-dione))amino)ethyl)amino)phenyl)-3-oxo-4-aza-5α-androst-1-ene-17β-carboxamide (C56)

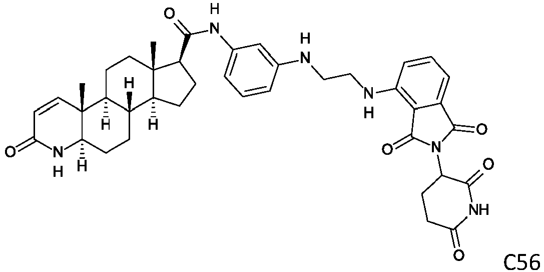

### Step 1: Synthesis of 3-((2-(N-Boc-amino)ethyl)amino)aniline (II-56)

### a. Synthesis of 3-((2-(N-Boc-amino)ethyl)amino)nitrobenzene (II-56-1)

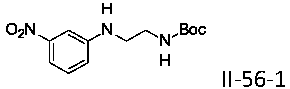

At room temperature, 2.00 g of 3-nitroaniline, 2.77 g of *N*-Boc-2-aminoacetaldehyde, 40 mL of DME, and 0.65 g of glacial acetic acid were sequentially added to a 100-mL three-necked flask, before 6.14 g of STAB was added with stirring. The system was heated to 40 °C and incubated for 3 h. The reaction solution was concentrated to give a yellow solid, and the solid was purified by column chromatography (eluent: petroleum ether:methyl *tert*-butyl ether = 3:1) to give compound II-56-1 (1.70 g).

### b. Synthesis of 3-((2-(N-Boc-amino)ethyl)amino)aniline (II-56)

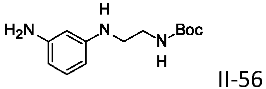

At room temperature, 1.70 g of compound II-56-1, 10 mL of methanol, and 100 mg of 5% palladium on carbon were sequentially added into a 100-mL hydrogenation reactor for 1 h of hydrogenation reaction at 25 °C. When the reaction was completed, the reaction solution was concentrated to give a gray oily substance (crude compound II-56, 1.38 g).

### Step 2: Synthesis of N-(3-((2-(N-Boc-amino)ethyl)amino)phenyl)-3-oxo-4-aza-5α-androst-1-ene-17β-carboxamide (IV-56)

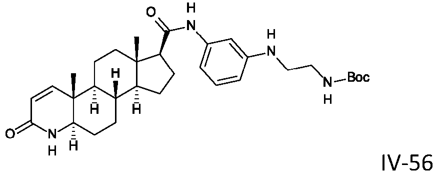

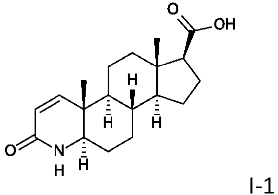

In a nitrogen atmosphere at room temperature, 2.09 g of compound I-1, 1.38 g of crude compound II-56, 5.14 g of pyBoP, and 50 mL of DMF were sequentially added into a 100-mL three-necked flask, and the mixture was stirred for 5 min. 2.38 g of DIPEA was added dropwise to the reaction system, and the reaction system was incubated overnight. 70 mL of water was added to the reaction solution, and the mixture was extracted with ethyl acetate (70 mL × 3). The organic phases were combined, sequentially washed once with 70 mL of 1 mol/L aqueous hydrochloric acid, 70 mL of saturated aqueous sodium carbonate, and 70 mL of saturated aqueous sodium chloride, dried over anhydrous sodium sulfate, and concentrated to give a black oily substance (crude compound IV-56, 4.35 g).

### Step 3: Synthesis of N-(3-((2-aminoethyl)amino)phenyl)-3-oxo-4-aza-5α-androst-1-ene-17β-carboxamide (V-56)

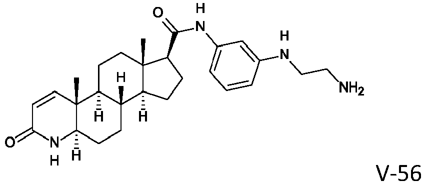

At room temperature, 4.35 g of crude compound IV-56 and 20 mL of dichloromethane were added into a 50-mL three-necked flask, before 20 mL of trifluoroacetic acid was dropwise added. The reaction solution was concentrated to give an oily substance (crude compound V-56), which was directly used in the next reaction without purification.

### Step 4: Synthesis of N-(3-((2-((4-(2-(3-(2,6-dioxopiperidine))-isoindole-1,3-dione))amino)ethyl)amino)phenyl)-3-oxo-4-aza-5α-androst-1-ene-17β-carboxamide (C56)

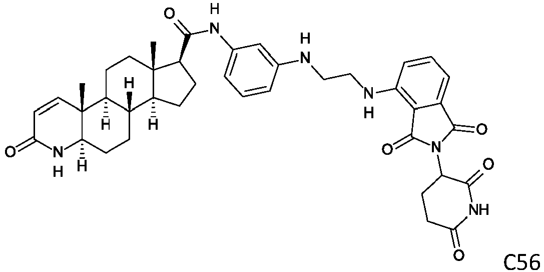

At room temperature, the oily substance obtained in step 3 (crude compound V-56), 1.98 g of 4-fluoro-2-(3-(2,6-dioxopiperidine))-isoindole-1,3-dione, 3.08 g of DIPEA, and 20 mL of DMF were sequentially added into a reaction flask. The reaction flask was treated in a microwave reactor for 10 min of reaction at 50 Hz. 30 mL of water was added into the reaction flask, and the mixture was extracted with dichloromethane (30 mL × 3). The organic phases were combined, sequentially washed once with 30 mL of water, dried over anhydrous sodium sulfate, and concentrated to give a crude product. The crude product was purified by column chromatography (eluent: dichloromethane:methanol = 10:1) to give compound C56 (1.52 g).
The mass spectral data for compound C56 are, MS (ESI): (M+H)⁺, 707.3

The nuclear magnetic resonance data for compound C56 are as follows:
HNMR (400MHz, CDCl₃)δ: 8.80-8.82 (m, 1H), 7.38-7.41 (m, 1H), 7.10-7.13 (m, 2H), 6.98-7.01 (m, 2H), 6.76-6.79 (d, 1H, J=12Hz), 6.55-6.58 (m, 1H), 6.56-6.59 (m, 1H), 6.21-6.24 (m, 2H), 5.88 (s, 1H), 5.70-5.73 (m, 1H), 4.82-4.87 (m, 1H), 3.05-3.15 (m, 5H), 2.60-2.78 (m, 3H), 2.10-2.18 (m, 2H), 1.88-2.06 (m, 2H), 1.60-1.83 (m, 5H), 1.45-1.55 (m, 2H), 1.10-1.40 (m, 6H), 0.84 (s, 3H), 0.69 (s, 3H)

### Example 57: Synthesis of N-(3-((3-((4-(2-(3-(2,6-dioxopiperidine))-isoindole-1,3-dione))amino)cyclopentyl)oxy)phenyl)-3-oxo-4-aza-5α-androst-1-ene-17β-carboxamide (C57)

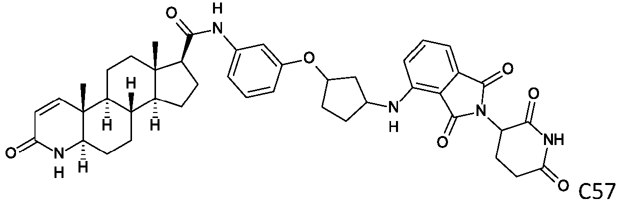

The synthesis was similar to that in Example 29.
The mass spectral data for compound C57 are, MS (ESI): (M+H)⁺, 748.4

The nuclear magnetic resonance data for compound C57 are as follows:
H NMR (400 MHz, CDCl₃) δ: 8.87-8.89 (m, 1H), 7.39-7.41 (m, 1H), 7.31-7.34 (m, 1H), 7.09-7.13 (m, 1H), 6.97-7.01 (m, 2H), 6.80-6.87 (m, 2H), 6.71-6.75 (m, 1H), 6.59-6.61 (m, 1H), 6.39-6.45 (m, 1H), 5.95 (s, 1H), 5.71-5.75 (m, 1H), 4.79-4.82 (m, 1H), 4.15-4.20 (m, 1H), 3.48-3.53 (m, 1H), 3.23-3.27 (m, 1H), 2.65-2.85 (m, 3H), 2.28-2.36 (m, 2H), 2.14-2.24 (m, 4H), 1.80-2.10 (m, 5H), 1.61-1.75 (m, 3H), 1.45-1.53 (m, 2H), 1.10-1.37 (m, 5H), 0.92-0.99 (m, 2H), 0.89 (s, 3H), 0.77 (s, 3H)

### Example 58: Synthesis of N-(3-(N-(N'-(4-(2-(3-(2,6-dioxopiperidine))-isoindole-1,3-dione))cyclohexanediamine))phenyl)-3-oxo-4-aza-5α-androst-1-ene-17β-carboxamide (C58)

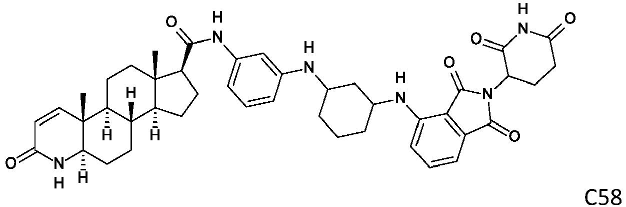

The synthesis was similar to that in Example 59.
The mass spectral data for compound C58 are, MS (ESI): (M+H)⁺, 761.4

The nuclear magnetic resonance data for compound C58 are as follows:
H NMR (400 MHz, CDCl₃) δ: 8.37-8.48 (m, 1H), 7.42-7.48 (m, 1H), 6.96-7.21 (m, 5H), 6.73-6.81 (m, 2H), 6.43-6.57 (m, 1H), 6.16-6.35 (m, 2H), 5.80-5.83 (m, 1H), 5.55 (s, 1H), 4.88-4.91 (m, 1H), 3.73-3.80 (m, 1H), 3.42-3.55 (m, 1H), 3.28-3.35 (m, 1H), 2.67-2.91 (m, 3H), 2.21-2.33 (m, 2H), 2.00-2.12 (m, 2H), 1.01-1.93 (m, 21H), 0.96 (s, 3H), 0.75 (s, 3H)

### Example 59: Synthesis of N-(3-(N-(N'-(4-(2-(3-(2,6-dioxopiperidine))-isoindole-1,3-dione))-1R,3R-cyclohexanediamine))phenyl)-3-oxo-4-aza-5α-androst-1-ene-17β-carboxamide (C59)

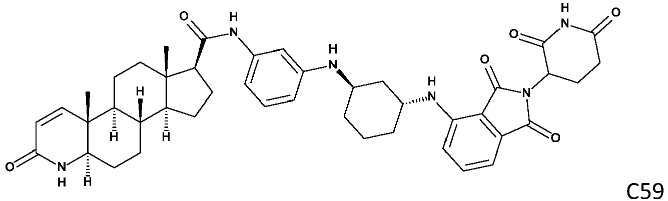

### Step 1: Synthesis of 3-(N-(N'-Boc-1R,3R-cyclohexanediamine))aniline (compound II-59)

### a. Synthesis of 3-(N-(N'-Boc-1R,3R-cyclohexanediamine))nitrobenzene (compound II-59-1)

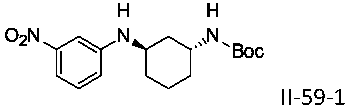

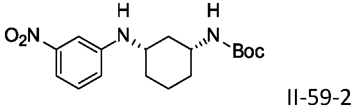

At room temperature, 5.00 g of (1*R*)-*N*-Boc-3-oxo-cyclohexylamine, 3.24 g of 3-nitroaniline, 30 mL of DME, and 1.05 g of glacial acetic acid were sequentially added to a 250-mL three-necked flask, before 9.96 g of STAB was added with stirring. The system was heated to 40 °C and incubated for 3 h. The reaction solution was concentrated to give a yellow solid. The yellow solid was purified by column chromatography (eluent: petroleum ether:methyl *tert-*butyl ether = 5:1) to give compound II-59-1 (3.11 g) and compound II-59-2 (0.82 g).

### b. Synthesis of 3-(N-(N'-Boc-1R,3R-cyclohexanediamine))aniline (compound II-59)

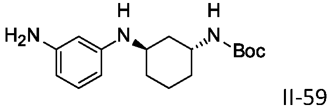

At room temperature, 1.00 g of compound II-59-1, 10 mL of methanol, and 100 mg of 5% palladium on carbon were sequentially added into a 100-mL hydrogenation reactor for 1 h of hydrogenation reaction at 25 °C. When the reaction was completed, the reaction solution was concentrated to give a dark gray oily substance (crude compound II-59, 0.90 g).

### Step 2: Synthesis of N-(3-(N-(N'-Boc-1R,3R-cyclohexanediamine))phenyl)-3-oxo-4-aza-5α-androst-1-ene-17β-carboxamide (compound IV-59)

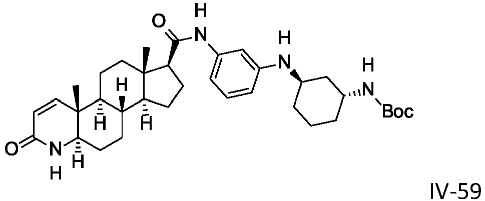

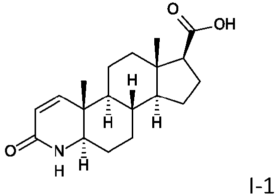

In a nitrogen atmosphere at room temperature, 1.14 g of compound I-1, 0.90 g of crude compound II-59, 3.41 g of pyBoP, and 30 mL of DMF were sequentially added into a 100-mL three-necked flask, and the mixture was stirred for 5 min. 1.27 g of DIPEA was added dropwise to the reaction system, and the reaction system was incubated overnight. 50 mL of water was added to the reaction solution, and the mixture was extracted with ethyl acetate (50 mL × 3). The organic phases were combined, sequentially washed once with 50 mL of 1 mol/L aqueous hydrochloric acid, 50 mL of saturated aqueous sodium carbonate, and 50 mL of saturated aqueous sodium chloride, dried over anhydrous sodium sulfate, and concentrated to give a black oily substance (crude compound IV-59, 2.56 g).

### Step 3: Synthesis of N-(3-(N-(1R,3R-cyclohexanediamine))phenyl)-3-oxo-4-aza-5α-androst-1-ene-17β-carboxamide (compound V-59)

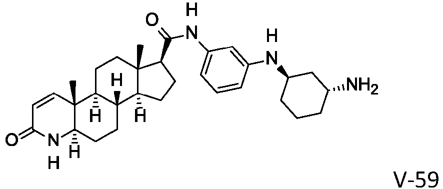

At room temperature, 2.56 g of crude compound IV-59 and 10 mL of dichloromethane were added into a 100-mL three-necked flask before 20 mL of trifluoroacetic acid was dropwise added, and the reaction system was incubated for 10 min. The reaction solution was concentrated to give an oily substance, before 100 mL of water was added. The mixture was adjusted to pH 11 with 2 mol/L aqueous sodium hydroxide and extracted with 100 mL of isobutanol. The organic phase was concentrated, and the residue was separated by column chromatography (eluent: dichloromethane:methanol = 8:1) to give an off-white solid (compound V-59, 1.21 g).

### Step 4: Synthesis of N-(3-(N-(N'-(4-(2-(3-(2,6-dioxopiperidine))-isoindole-1,3-dione))-1R,3R-cyclohexanediamine))phenyl)-3-oxo-4-aza-5α-androst-1-ene-17β-carboxamide (C59)

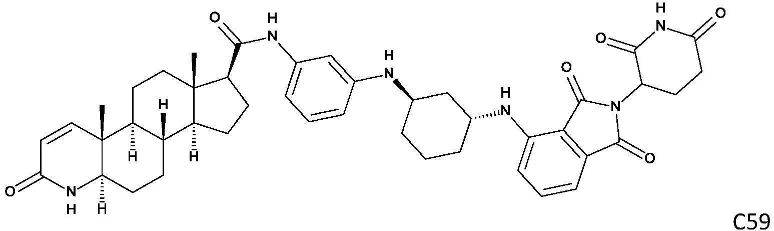

At room temperature, 1.21 g of compound V-59, 0.98 g of 4-fluoro-2-(3-(2,6-dioxopiperidine))-isoindole-1,3-dione, 1.53 g of DIPEA, and 10 mL of DMF were added into a reaction flask. The reaction flask was treated in a microwave reactor for 10 min of reaction at 50 Hz. 20 mL of water was added into the reaction flask, and the mixture was extracted with dichloromethane (20 mL × 3). The organic phases were combined, sequentially washed once with 20 mL of water, dried over anhydrous sodium sulfate, and concentrated to give a crude product. The crude product was purified by column chromatography (eluent: dichloromethane:methanol = 10:1) to give compound C59 (0.70 g).
The mass spectral data for compound C59 are, MS (ESI): (M+H)⁺, 761.4

The nuclear magnetic resonance data for compound C59 are as follows:
H NMR (400 MHz, CDCl₃) δ: 8.48-8.50 (m, 1H), 7.42-7.48 (m, 1H), 7.15-7.21 (m, 1H), 7.08-7.12 (m, 3H), 6.73-6.81 (m, 2H), 6.51-6.57 (m, 1H), 6.28-6.35 (m, 2H), 5.80 (d, 1H, J=12Hz), 5.60 (s, 1H), 4.88-4.91 (m, 1H), 3.73-3.80 (m, 2H), 3.28-3.33 (m, 1H), 2.67-2.90 (m, 3H), 2.21-2.33 (m, 2H), 2.00-2.12 (m, 2H), 1.90-1.71 (m, 8H), 1.58-1.15 (m, 11H), 1.01-1.11 (m, 2H), 0.96 (s, 3H), 0.75 (s, 3H)

### Example 60: Synthesis of N-(3-(N-(N'-(4-(2-(3-(2,6-dioxopiperidine))-isoindole-1,3-dione))-1S,3S-cyclohexanediamine))phenyl)-3-oxo-4-aza-5α-androst-1-ene-17β-carboxamide (C60)

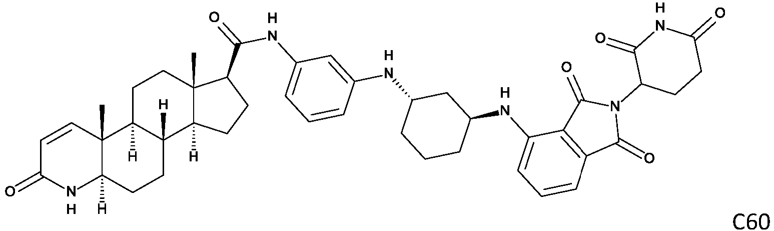

The synthesis was similar to that in Example 59.
The mass spectral data for compound C60 are, MS (ESI): (M+H)⁺, 761.4

The nuclear magnetic resonance data for compound C60 are as follows:
H NMR (400 MHz, CDCl₃) δ: 8.36-8.41 (m, 1H), 7.40-7.42 (m, 1H), 7.18-7.26 (m, 1H), 7.05-7.09 (m, 3H), 6.94-6.97 (m, 1H), 6.76-6.83 (m, 2H), 6.53-6.57 (m, 1H), 6.28-6.34 (m, 2H), 5.80-5.83 (m, 1H), 5.49 (s, 1H), 4.89-4.92 (m, 1H), 3.74-3.82 (m, 2H), 3.31-3.35 (m, 1H), 2.72-2.90 (m, 3H), 2.23-2.27 (m, 2H), 2.01-2.13 (m, 2H), 1.71-1.90 (m, 8H), 1.20-1.58 (m, 11H), 1.01-1.08 (m, 2H), 0.96 (s, 3H), 0.75 (m, 3H)

### Example 61: Synthesis of N-(3-(N-(N'-(4-(2-(3-(2,6-dioxopiperidine))-isoindole-1,3-dione))-1S,3R-cyclohexanediamine))phenyl)-3-oxo-4-aza-5α-androst-1-ene-17β-carboxamide (C61)

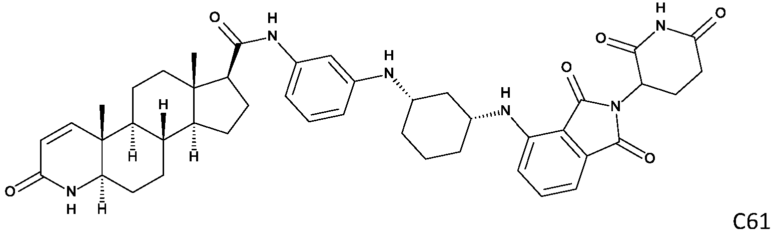

### Step 1: Synthesis of 3-(N-(N'-Boc-1S,3R-cyclohexanediamine))aniline (compound II-61)

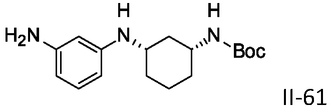

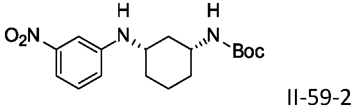

At room temperature, 0.72 g of compound II-59-2, 10 mL of methanol, and 72 mg of 5% palladium on carbon were sequentially added into a 100-mL hydrogenation reactor for 1 h of hydrogenation reaction at 25 °C. When the reaction was completed, the reaction solution was concentrated to give a dark gray oily substance (crude compound II-61, 0.68 g).

### Step 2: Synthesis of N-(3-(N-(N'-Boc-1S,3R-cyclohexanediamine))phenyl)-3-oxo-4-aza-5α-androst-1-ene-17β-carboxamide (compound IV-61)

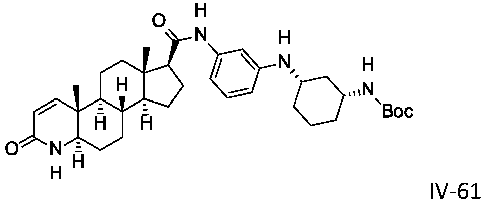

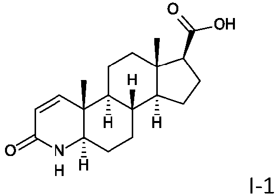

In a nitrogen atmosphere at room temperature, 0.85 g of compound I-1, 0.68 g of crude compound II-61, 2.51 g of pyBoP, and 30 mL of DMF were sequentially added into a 100-mL three-necked flask, and the mixture was stirred for 5 min. 0.94 g of DIPEA was added dropwise to the reaction system, and the reaction system was incubated overnight. 50 mL of water was added to the reaction solution, and the mixture was extracted with ethyl acetate (50 mL × 3). The organic phases were combined, sequentially washed once with 50 mL of 1 mol/L aqueous hydrochloric acid, 50 mL of saturated aqueous sodium carbonate, and 50 mL of saturated aqueous sodium chloride, dried over anhydrous sodium sulfate, and concentrated to give a black oily substance (crude compound IV-61, 2.34 g).

### Step 3: Synthesis of N-(3-(N-(1S,3R-cyclohexanediamine))phenyl)-3-oxo-4-aza-5α-androst-1-ene-17β-carboxamide (compound V-61)

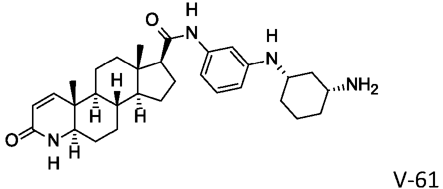

At room temperature, 2.34 g of crude compound IV-61 and 10 mL of dichloromethane were added into a 100-mL three-necked flask before 20 mL of trifluoroacetic acid was dropwise added, and the reaction system was incubated for 10 min. The reaction solution was concentrated to give an oily substance, before 100 mL of water was added. The mixture was adjusted to pH 11 with 2 mol/L aqueous sodium hydroxide and extracted with 100 mL of isobutanol. The organic phase was concentrated, and the residue was separated by column chromatography (eluent: dichloromethane:methanol = 8:1) to give an off-white solid (compound V-61, 1.06 g).

### Step 4: Synthesis of N-(3-(N-(N'-(4-(2-(3-(2,6-dioxopiperidine))-isoindole-1,3-dione))-1S,3R-cyclohexanediamine))phenyl)-3-oxo-4-aza-5α-androst-1-ene-17β-carboxamide (C61)

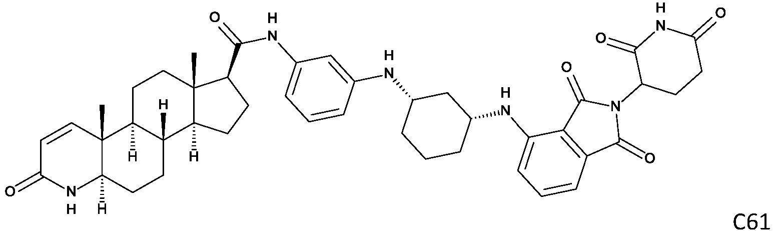

At room temperature, 1.06 g of compound V-61, 0.87 g of 4-fluoro-2-(3-(2,6-dioxopiperidine))-isoindole-1,3-dione, 1.36 g of DIPEA, and 10 mL of DMF were added into a reaction flask. The reaction flask was treated in a microwave reactor for 10 min of reaction at 50 Hz. 20 mL of water was added into the reaction flask, and the mixture was extracted with dichloromethane (20 mL × 3). The organic phases were combined, sequentially washed once with 20 mL of water, dried over anhydrous sodium sulfate, and concentrated to give a crude product. The crude product was purified by column chromatography (eluent: dichloromethane:methanol = 10:1) to give compound C61 (0.56 g).
The mass spectral data for compound C61 are, MS (ESI): (M+H)⁺, 761.4

The nuclear magnetic resonance data for compound C61 are as follows:
H NMR (400 MHz, CDCl₃) δ: 8.62-8.64 (m, 1H), 7.44-7.48 (m, 1H), 7.35-7.38 (m, 1H), 7.25-7.28 (m, 1H), 7.18-7.24 (m, 2H), 6.83-6.87 (m, 2H), 6.45-6.47 (m, 1H), 6.26-6.35 (m, 2H), 5.80 (d, 1H, J=12Hz), 5.70 (s, 1H), 4.85-4.90 (m, 1H), 3.65-3.68 (m, 1H), 3.80-3.83 (m, 1H), 3.30-3.35 (m, 1H), 2.67-2.90 (m, 3H), 2.05-2.33 (m, 4H), 1.95-1.70 (m, 13H), 1.58-1.15 (m, 6H), 1.03-1.11 (m, 2H), 0.95 (s, 3H), 0.77 (s, 3H)

### Example 62: Synthesis of N-(3-(N-(N'-(4-(2-(3-(2,6-dioxopiperidine))-1-oxo-2,3-dihydro-1H-isoindol))-1R,3R-cyclohexanediamine))phenyl)-3-oxo-4-aza-5α-androst-1-ene-17β-carboxamide (C62)

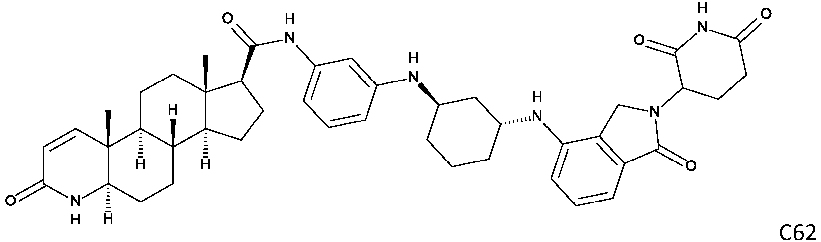

### Step 1: Synthesis of N-(3-(N-(N'-(4-(2-(3-(2,6-dioxopiperidine))-1-oxo-3-hydroxy-2,3-dihydro-1H-isoindol))-1R,3R-cyclohexanediamine))phenyl)-3-oxo-4-aza-5α-androst-1-ene-17β-carboxamide (VIII-62)

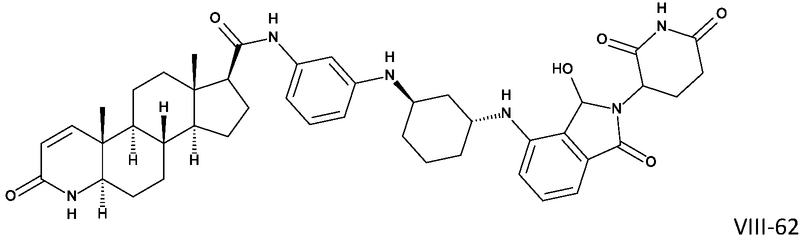

At room temperature, 1.00 g of compound C59, 0.44 g of zinc powder, and 10 mL of glacial acetic acid were sequentially added into a reaction flask, and the reaction system was heated to 70 °C and incubated for 2 h. The reaction solution was filtered before cooling, and the filtrate was concentrated to give a pale yellow solid (compound VIII-62).

### Step 2: Synthesis of N-(3-(N-(N'-(4-(2-(3-(2,6-dioxopiperidine))-1-oxo-2,3-dihydro-1H-isoindol))-1R,3R-cyclohexanediamine))phenyl)-3-oxo-4-aza-5α-androst-1-ene-17β-carboxamide (C62)

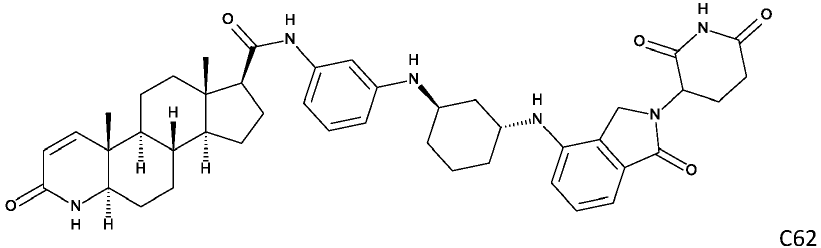

The pale yellow solid (compound VIII-62) obtained in step 1, 5 mL of triethylsilane, and 10 mL of trifluoroacetic acid were added into a reaction flask, and the reaction system was heated to 60 °C and incubated for 3 h. The reaction solution was purified by column chromatography (eluent: dichloromethane:methanol = 10:1) to give compound C62 (15 mg).
The mass spectral data for compound C62 are, MS (ESI): (M+H)⁺, 747.4

The nuclear magnetic resonance data for compound C62 are as follows:
H NMR (400 MHz, CDCl₃) δ: 9.00 (s, 1H), 7.28-7.33 (m, 1H), 7.11-7.21 (m, 1H), 7.00-7.12 (m, 3H), 6.81-6.84 (m, 1H), 6.73-6.87 (m, 1H), 6.44-6.50 (m, 1H), 6.20-6.31 (m, 2H), 5.85 (d, 1H, J=12Hz), 5.67 (s, 1H), 5.02-5.07 (m, 1H), 4.23-4.38 (m, 2H), 3.75-3.85 (m, 2H), 3.28-3.33 (m, 1H), 2.65-2.69 (m, 1H), 2.45-2.48 (m, 1H), 2.38-2.41 (m, 1H), 2.10-2.28 (m, 2H), 2.01-2.11 (m, 2H), 1.90-1.43 (m, 14H), 1.35-1.15 (m, 5H), 1.08-1.11 (m, 2H), 0.92 (s, 3H), 0.75 (s, 3H)

### Example 63: Preparation of N-(3-(N-(N'-(4-(2-(3S-(2,6-dioxopiperidine))-isoindole-1,3-dione))-1R,3R-cyclohexanediamine))phenyl)-3-oxo-4-aza-5α-androst-1-ene-17β-carboxamide (C63)

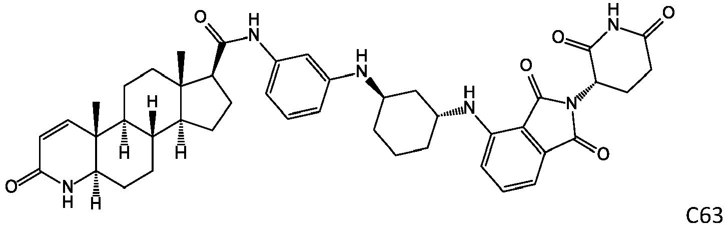

Compound C59 was separated by preparative chromatography, and the second fraction was collected to give compound C63. The conditions for the preparative chromatography are as follows:
Column: Daicel CHIRALPAK IA (5.0 cm I.D. × 25 cm L, 10 µm)
Mobile phase: methanol/dichloromethane = 60/40 (V/V)
Flow rate: 60 mL/min
Wavelength: 254 nm
Column temperature: 38 °C
The mass spectral data for compound C63 are, MS (ESI): (M+H)⁺, 761.4

The nuclear magnetic resonance data for compound C63 are as follows:
H NMR (400 MHz, CDCl₃) δ: 8.56 (s, 1H), 7.39-7.43 (m, 1H), 7.29 (s, 1H), 7.04-7.08 (m, 3H), 6.77-6.83 (m, 2H), 6.57-6.59 (m, 1H), 6.37-6.39 (m, 1H), 6.26-6.28 (m, 1H), 5.80-5.82 (m, 1H), 5.74 (s, 1H), 4.89-4.93 (m, 1H), 3.73-3.83 (m, 2H), 3.30-3.34 (m, 1H), 2.72-2.90 (m, 3H), 2.23-2.25 (m, 2H), 2.10-2.14 (m, 2H), 1.72-1.93 (m, 8H), 1.25-1.60 (m, 11H), 1.04-1.07 (m, 2H), 0.95 (s, 3H), 0.74 (s, 3H)

### Example 64: Preparation of N-(3-(N-(N'-(4-(2-(3R-(2,6-dioxopiperidine))-isoindole-1,3-dione))-1R,3R-cyclohexanediamine))phenyl)-3-oxo-4-aza-5α-androst-1-ene-17β-carboxamide (C64)

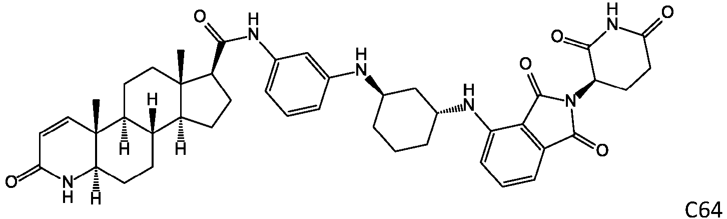

Compound C59 was separated by preparative chromatography, and the first fraction was collected to give compound C64. The conditions were similar to those in Example 63.
The mass spectral data for compound C64 are, MS (ESI): (M+H)⁺, 761.4

The nuclear magnetic resonance data for compound C64 are as follows:
H NMR (400 MHz, CDCl₃) δ: 8.43 (s, 1H), 7.40-7.44 (m, 1H), 7.21 (s, 1H), 7.05-7.10 (m, 2H), 7.00 (s, 1H), 6.78-6.83 (m, 2H), 6.57-6.59 (m, 1H), 6.36-6.38 (m, 1H), 6.26-6.28 (m, 1H), 5.80-5.83 (m, 1H), 5.67 (s, 1H), 4.88-4.94 (m, 1H), 3.71-3.82 (m, 2H), 3.30-3.34 (m, 1H), 2.71-2.90 (m, 3H), 2.25-2.26 (m, 2H), 2.10-2.12 (m, 2H), 1.71-1.91 (m, 8H), 1.27-1.60 (m, 11H), 1.02-1.06 (m, 2H), 0.96 (s, 3H), 0.74 (s, 3H)

### Example 65: Synthesis of N-(3-(N-(N'-(4-(2-(35-(2,6-dioxopiperidine))-1-oxo-2,3-dihydro-1H-isoindol))-1R,3R-cyclohexanediamine))phenyl)-3-oxo-4-aza-5α-androst-1-ene-17β-carboxamide (C65)

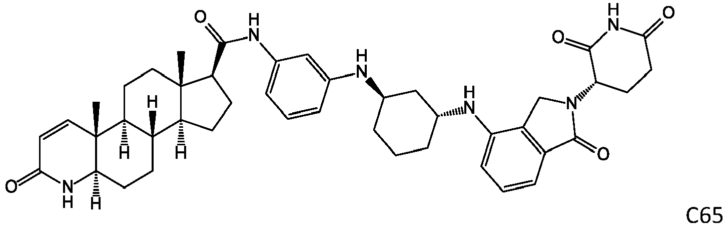

### Step 1: Synthesis of N-(3-(N-(N'-(4-(2-(3S-(2,6-dioxopiperidine))-1-oxo-3-hydroxy-2,3-dihydro-1H-isoindol))-1R,3R-cyclohexanediamine))phenyl)-3-oxo-4-aza-5α-androst-1-ene-17β-carboxamide (VIII-65)

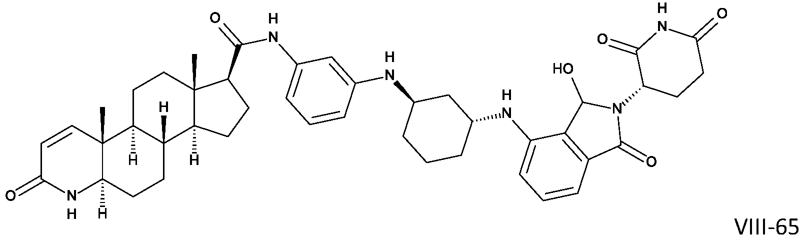

At room temperature, 1.00 g of compound C63, 0.44 g of zinc powder, and 10 mL of glacial acetic acid were sequentially added into a reaction flask, and the reaction system was heated to 70 °C and incubated for 2 h. The reaction solution was filtered before cooling, and the filtrate was concentrated to give crude compound VIII-65.

### Step 2: Synthesis of N-(3-(N-(N'-(4-(2-(35-(2,6-dioxopiperidine))-1-oxo-2,3-dihydro-1H-isoindol))-1R,3R-cyclohexanediamine))phenyl)-3-oxo-4-aza-5α-androst-1-ene-17β-carboxamide (C65)

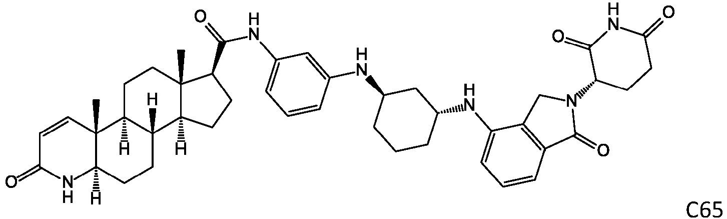

5 mL of triethylsilane and 10 mL of trifluoroacetic acid were added to crude compound VIII-65 obtained in step 1, and the reaction system was heated to 60 °C and incubated for 3 h. The reaction solution was purified by column chromatography (eluent: dichloromethane:methanol = 10:1) to give compound C65 (14 mg).
The mass spectral data for compound C65 are, MS (ESI): (M+H)⁺, 747.4

The nuclear magnetic resonance data for compound C65 are as follows:
H NMR (400 MHz, CDCl₃) δ: 9.01 (s, 1H), 7.29-7.33 (m, 1H), 7.11-7.21 (m, 1H), 7.00-7.12 (m, 3H), 6.82-6.85 (m, 1H), 6.82-6.85 (m, 1H), 6.45-6.50 (m, 1H), 6.22-6.25 (m, 2H), 5.85-5.86 (m, 1H), 5.78 (s, 1H), 5.02-5.07 (m, 1H), 4.24-4.37 (m, 2H), 3.76-3.80 (m, 2H), 3.28-3.33 (m, 1H), 2.65-2.69 (m, 1H), 2.45-2.48 (m, 1H), 2.38-2.41 (m, 1H), 2.10-2.28 (m, 2H), 2.01-2.12 (m, 2H), 1.91-1.43 (m, 14H), 1.35-1.15 (m, 5H), 1.08-1.11 (m, 2H), 0.93 (s, 3H), 0.76 (s, 3H)

### Example 66: Synthesis of N-(3-(N-(N'-(4-(2-(3R-(2,6-dioxopiperidine))-1-oxo-2,3-dihydro-1H-isoindol))-1R,3R-cyclohexanediamine))phenyl)-3-oxo-4-aza-5α-androst-1-ene-17β-carboxamide (C66)

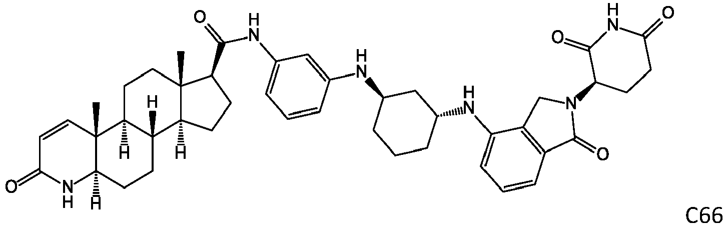

### Step 1: Synthesis of N-(3-(N-(N'-(4-(2-(3R-(2,6-dioxopiperidine))-1-oxo-3-hydroxy-2,3-dihydro-1H-isoindol))-1R,3R-cyclohexanediamine))phenyl)-3-oxo-4-aza-5α-androst-1-ene-17β-carboxamide (VIII-66)

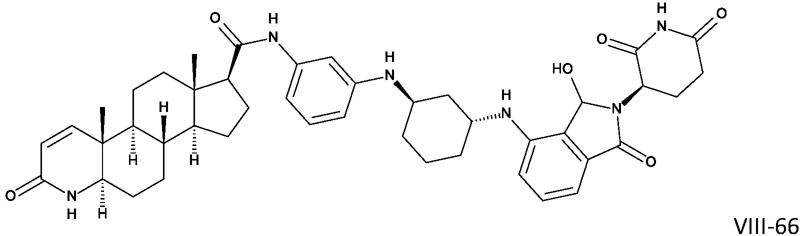

At room temperature, 1.00 g of compound C64, 0.44 g of zinc powder, and 10 mL of glacial acetic acid were sequentially added into a reaction flask, and the reaction system was heated to 70 °C and incubated for 2 h. The reaction solution was filtered before cooling, and the filtrate was concentrated to give crude compound VIII-66.

### Step 2: Synthesis of N-(3-(N-(N'-(4-(2-(3R-(2,6-dioxopiperidine))-1-oxo-2,3-dihydro-1H-isoindol))-1R,3R-cyclohexanediamine))phenyl)-3-oxo-4-aza-5α-androst-1-ene-17β-carboxamide (C66)

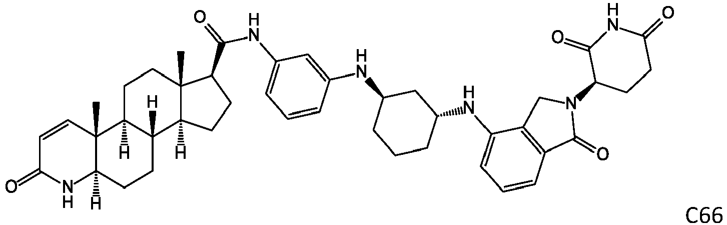

5 mL of triethylsilane and 10 mL of trifluoroacetic acid were added to crude compound VIII-66 obtained in step 1, and the reaction system was heated to 60 °C and incubated for 3 h. The reaction solution was purified by column chromatography (eluent: dichloromethane:methanol = 10:1) to give compound C66 (14.5 mg).
The mass spectral data for compound C66 are, MS (ESI): (M+H)⁺, 747.4

The nuclear magnetic resonance data for compound C66 are as follows:
H NMR (400 MHz, CDCl₃) δ: 9.02 (s, 1H), 7.28-7.33 (m, 1H), 7.01-7.20 (m, 4H), 6.81-6.84 (m, 1H), 6.81-6.84 (m, 1H), 6.44-6.50 (m, 1H), 6.29-6.31 (m, 2H), 5.89-5.90 (m, 1H), 5.67 (s, 1H), 5.02-5.07 (m, 1H), 4.23-4.38 (m, 2H), 3.79-3.84 (m, 2H), 3.29-3.34 (m, 1H), 2.66-2.70 (m, 1H), 2.46-2.49 (m, 1H), 2.39-2.42 (m, 1H), 2.12-2.26 (m, 2H), 2.01-2.11 (m, 2H), 1.89-1.45 (m, 14H), 1.35-1.11 (m, 5H), 1.04-1.09 (m, 2H), 0.92 (s, 3H), 0.75 (s, 3H)

### Example 67: Synthesis of N-(3-(N-(N'-(4-(2-(35-(1-pivaloyloxymethyl-2,6-dioxopiperidine))-isoindole-1,3-dione))-1R,3R-cyclohexanediamine))phenyl)-3-oxo-4-aza-5α-androst-1-ene-17β-carboxamide (C67)

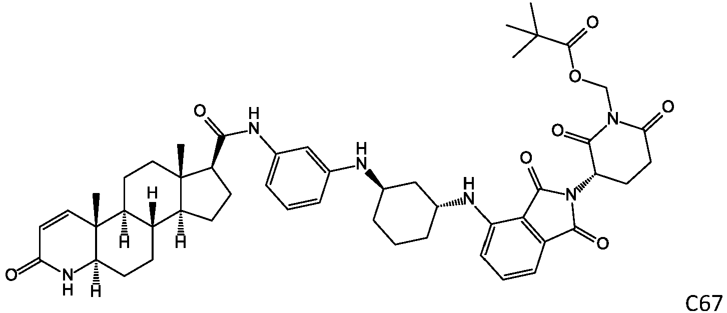

The synthesis was similar to that in Example 49.
The mass spectral data for compound C67 are, MS (ESI): (M+H)⁺, 875.5

The nuclear magnetic resonance data for compound C67 are as follows:
H NMR (400 MHz, CDCl₃) δ: 8.61-8.63 (m, 1H), 7.45-7.48 (m, 1H), 7.19-7.23 (m, 1H), 7.08-7.14 (m, 3H), 6.75-6.82 (m, 2H), 6.52-6.58 (m, 1H), 6.27-6.34 (m, 2H), 5.79-5.82 (m, 1H), 5.70 (s, 1H), 4.87-4.91 (m, 1H), 4.81 (s, 2H), 3.73-3.81 (m, 2H), 3.28-3.33 (m, 1H), 2.65-2.89 (m, 3H), 2.20-2.32 (m, 2H), 2.01-2.12 (m, 2H), 1.90-1.73 (m, 8H), 1.58-1.15 (m, 11H), 1.01-1.11 (m, 5H), 0.95 (s, 3H), 0.74 (s, 3H)

### Example 68: Synthesis of N-(3-(N-(N'-(4-(2-(3-(2,6-dioxopiperidine))-isoindole-1,3-dione))-1,3-dideutero-1R,3R-cyclohexanediamine))phenyl)-3-oxo-4-aza-5α-androst-1-ene-17β-carboxamide (C68)

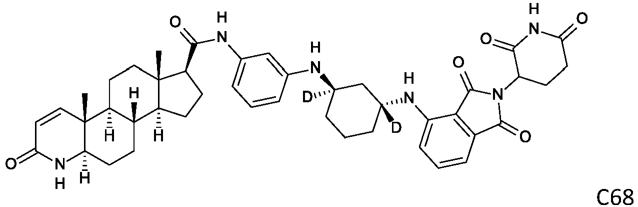

### Step 1: Synthesis of 3-(N-(N'-Boc-1,3-dideutero-1R,3R-cyclohexanediamine))aniline (II-68)

### a. Synthesis of 3-(N-(1,3-dideutero-1R,3R-cyclohexanediamine))nitrobenzene (II-68-1)

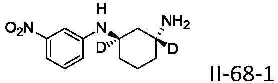

100 mg of *m*-fluoronitrobenzene and 820 mg of 1,3-dideutero-1*R*,3*R*-cyclohexanediamine were transferred into a three-necked flask. The mixture was heated to 100 °C in a nitrogen atmosphere and stirred for reaction for 12 h. The reaction solution was cooled to room temperature and concentrated to give a crude product. The crude product was purified by column chromatography (eluent: dichloromethane:methanol = 6:1) to give a pale yellow solid (compound II-68-1, 21 mg).
MS (ESI): (M+H)⁺, 238.1

### b. Synthesis of 3-(N-(N'-Boc-1,3-dideutero-1R,3R-cyclohexanediamine))nitrobenzene (compound II-68-2)

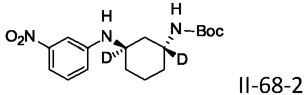

21 mg of compound II-68-1, 9.8 mg of triethylamine, 21 mg of Boc anhydride, and 2 mL of dichloromethane were sequentially added to a reaction flask, and the reaction system was stirred at room temperature for 30 min. The reaction solution was washed once with 0.5 mL of 1 mol/L aqueous hydrochloric acid and 0.5 mL of water, and the aqueous phase was extracted with dichloromethane (0.5 mL × 2). The organic phases were combined, dried over anhydrous sodium sulfate, and concentrated to give crude compound II-68-2 (29 mg).
MS (ESI): (M+H)⁺, 338.2

### c. Synthesis of 3-(N-(N'-Boc-1,3-dideutero-1R,3R-cyclohexanediamine))aniline (II-68)

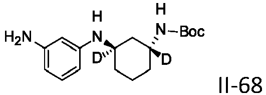

29 mg of crude compound II-68-2, 2 mL of methanol, and 2.9 mg of 5% palladium on carbon were added into a 100-mL hydrogenation reactor, and the system was incubated with continuous hydrogen introduction at 25 °C and 50 psi until the reaction was completed. The reaction solution was concentrated to give crude compound II-68 (24 mg).
MS (ESI): (M+H)⁺, 308.2

### Step 2: Synthesis of N-(3-(N-(N'-Boc-1,3-dideutero-1R,3R-cyclohexanediamine))phenyl)-3-oxo-4-aza-5α-androst-1-ene-17β-carboxamide (IV-68)

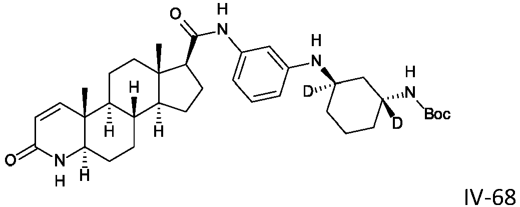

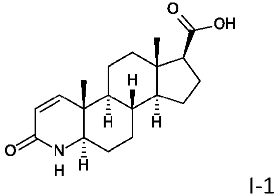

In a nitrogen atmosphere, 11 mg of compound I-1, 15 mg of crude compound II-68, 32.8 mg of pyBoP, 12.2 mg of DIPEA, and 0.5 mL of DMF were sequentially added into a reaction flask, and the mixture was stirred overnight until the reaction was completed. 2.5 mL of water was added into the reaction flask. The reaction solution was extracted with ethyl acetate (2.5 mL × 3). The organic phases were combined, sequentially washed once with 1 mL of 1 mol/L aqueous hydrochloric acid and 1 mL of saturated aqueous sodium carbonate, dried over anhydrous sodium sulfate, and concentrated to give a crude product. The crude product was purified by column chromatography (eluent: dichloromethane:methanol = 20:1) to give compound IV-68 (19 mg).

### Step 3: Synthesis of N-(3-(N-(1,3-dideutero-1R,3R-cyclohexanediamine))phenyl)-3-oxo-4-aza-5α-androst-1-ene-17β-carboxamide (V-68)

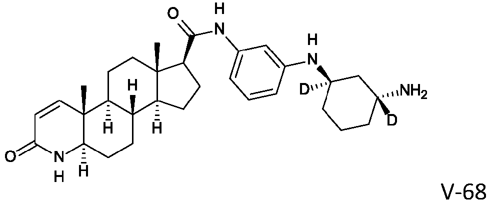

At room temperature, 19 mg of compound IV-68 and 1 mL of dichloromethane were added into a reaction flask, before 0.1 mL of trifluoroacetic acid was dropwise added. The reaction system was incubated for 10 min. The reaction solution was concentrated to give crude compound V-68 (16 mg).

### Step 4: Synthesis of N-(3-(N-(N'-(4-(2-(3-(2,6-dioxopiperidine))-isoindole-1,3-dione))-1,3-dideutero-1R,3R-cyclohexanediamine))phenyl)-3-oxo-4-aza-5α-androst-1-ene-17β-carboxamide (C68)

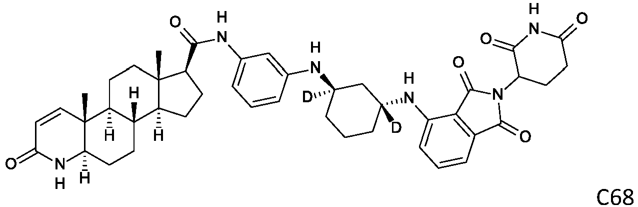

16 mg of crude compound V-68, 13 mg of 4-fluoro-2-(3-(2,6-dioxopiperidine))-isoindole-1,3-dione, 30 mg of DIPEA, and 1 mL of DMF were sequentially added into a reaction flask. The reaction flask was treated in a microwave reactor for 10 min of reaction at 50 Hz. The reaction solution was extracted with water and dichloromethane together (2.5 mL × 3). The organic phases were combined, dried over anhydrous sodium sulfate, and concentrated to give a crude product. The crude product was purified by column chromatography (eluent: dichloromethane:methanol = 15:1) to give compound C68 (10 mg).
The mass spectral data for compound C68 are, MS (ESI): (M+H)⁺, 763.4

The nuclear magnetic resonance data for compound C68 are as follows:
H NMR (400 MHz, CDCl₃) δ: 8.48-8.50 (m, 1H), 7.42-7.48 (m, 1H), 7.15-7.21 (m, 1H), 7.08-7.12 (m, 3H), 6.73-6.81 (m, 2H), 6.43-6.57 (m, 1H), 6.16-6.35 (m, 2H), 5.80 (d, 1H, J=12Hz), 5.60 (s, 1H), 4.88-4.91 (m, 1H), 3.40-3.57 (m, 1H), 2.67-2.90 (m, 3H), 2.21-2.33 (m, 2H), 2.00-2.12 (m, 2H), 1.90-1.71 (m, 8H), 1.58-1.15 (m, 11H), 1.01-1.11 (m, 2H), 0.96 (s, 3H), 0.75 (s, 3H)

### Example 69: Synthesis of N-(3-(N-(N'-(4-(2-(3-(2,6-dioxopiperidine))-isoindole-1,3-dione))-2-methyl-1R,3R-cyclohexanediamine))phenyl)-3-oxo-4-aza-5α-androst-1-ene-17β-carboxamide (C69)

### Step 1: Synthesis of 3-(N-(N'-Boc-2-methyl-1R,3R-cyclohexanediamine))aniline (II-69)

### a. Synthesis of 3-(N-(2-methyl-1R,3R-cyclohexanediamine))nitrobenzene (compound II-69-1)

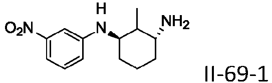

100 mg of *m*-fluoronitrobenzene and 900 mg of 2-methyl-1*R*,3*R*-cyclohexanediamine were transferred into a three-necked flask. The mixture was heated to 100 °C in a nitrogen atmosphere and stirred for reaction for 12 h. The reaction solution was cooled to room temperature and concentrated to give a crude product. The crude product was purified by column chromatography (eluent: dichloromethane:methanol = 6:1) to give a pale yellow solid (compound II-69-1, 26 mg).
MS (ESI): (M+H)⁺, 250.1

### b. Synthesis of 3-(N-(N'-Boc-2-methyl-1R,3R-cyclohexanediamine))nitrobenzene (II-69-2)

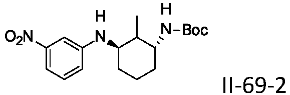

26 mg of compound II-69-1, 14.2 mg of triethylamine, 30 mg of Boc anhydride, and 2 mL of dichloromethane were sequentially added to a reaction flask, and the reaction system was stirred at room temperature for 30 min. The reaction solution was washed once with 0.5 mL of 1 mol/L aqueous hydrochloric acid and 0.5 mL of water, and the aqueous phase was extracted with dichloromethane (2 mL × 2). The organic phases were combined, dried over anhydrous sodium sulfate, and concentrated to give crude compound II-69-2 (36 mg).
MS (ESI): (M+H)⁺, 350.2

### c. Synthesis of 3-(N-(N'-Boc-2-methyl-1R,3R-cyclohexanediamine))aniline (II-69)

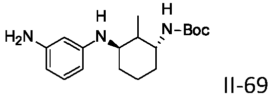

36 mg of crude compound II-69-2, 2 mL of methanol, and 3.6 mg of 5% palladium on carbon were added into a 100-mL hydrogenation reactor, and the system was incubated with continuous hydrogen introduction at 25 °C and 50 psi until the reaction was completed. The reaction solution was concentrated to give crude compound II-69 (30 mg).

### Step 2: Synthesis of N-(3-(N-(N'-Boc-2-methyl-1R,3R-cyclohexanediamine))phenyl)-3-oxo-4-aza-5α-androst-1-ene-17β-carboxamide (compound IV-69)

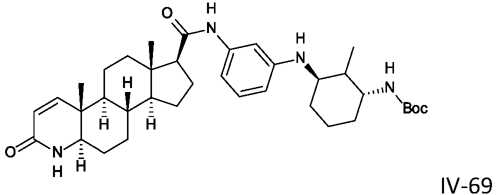

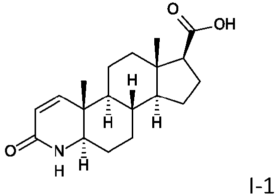

In a nitrogen atmosphere, 11 mg of compound I-1, 16 mg of crude compound II-69, 32.8 mg of pyBoP, 12.2 mg of DIPEA, and 0.5 mL of DMF were sequentially added into a reaction flask, and the mixture was stirred overnight until the reaction was completed. 2.5 mL of water was added into the reaction flask. The reaction solution was extracted with ethyl acetate (2.5 mL × 3). The organic phases were combined, sequentially washed once with 1 mL of 1 mol/L aqueous hydrochloric acid and 1 mL of saturated aqueous sodium carbonate, dried over anhydrous sodium sulfate, and concentrated to give a crude product. The crude product was purified by column chromatography (eluent: dichloromethane:methanol = 20:1) to give compound IV-69 (19.5 mg).
MS (ESI): (M+H)⁺, 619.4

### Step 3: Synthesis of N-(3-(N-(2-methyl-1R,3R-cyclohexanediamine))phenyl)-3-oxo-4-aza-5α-androst-1-ene-17β-carboxamide (V-69)

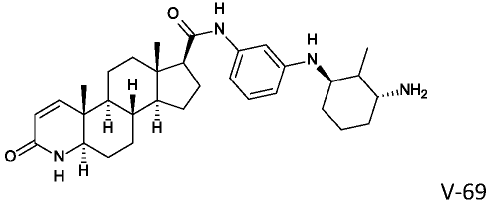

At room temperature, 19.5 mg of compound IV-69 and 1 mL of dichloromethane were added into a reaction flask, before 0.1 mL of trifluoroacetic acid was dropwise added. The reaction system was incubated for 10 min. The reaction solution was concentrated to give crude compound V-69 (16 mg).

### Step 4: Synthesis of N-(3-(N-(N'-(4-(2-(3-(2,6-dioxopiperidine))-isoindole-1,3-dione))-2-methyl-1R,3R-cyclohexanediamine))phenyl)-3-oxo-4-aza-5α-androst-1-ene-17β-carboxamide (compound C69)

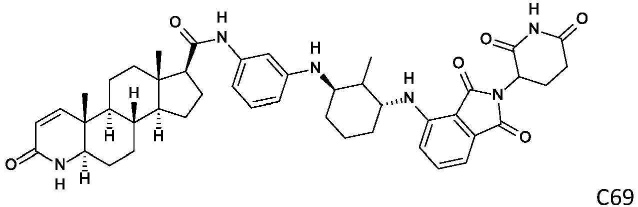

16 mg of crude compound V-69, 13 mg of 4-fluoro-2-(3-(2,6-dioxopiperidine))-isoindole-1,3-dione, 30 mg of DIPEA, and 1 mL of DMF were sequentially added into a reaction flask. The reaction flask was treated in a microwave reactor for 10 min of reaction at 50 Hz. 2.5 mL of water was added, and the mixture was extracted with water and dichloromethane together (2.5 mL × 3). The organic phases were combined, dried over anhydrous sodium sulfate, and concentrated to give a crude product. The crude product was purified by column chromatography (eluent: dichloromethane:methanol = 15:1) to give compound C69 (9.5 mg). The mass spectral data for compound C69 are, MS (ESI): (M+H)⁺, 775.4

The nuclear magnetic resonance data for compound C69 are as follows:
H NMR (400 MHz, CDCl₃) δ: 8.53-8.55 (m, 1H), 7.40-7.47 (m, 1H), 7.17-7.20 (m, 1H), 7.12-7.18 (m, 3H), 6.72-6.81 (m, 2H), 6.51-6.57 (m, 1H), 6.29-6.35 (m, 2H), 5.80 (d, 1H, J=12Hz), 5.71 (s, 1H), 4.81-4.85 (m, 1H), 3.85-3.75 (m, 2H), 3.40-3.59 (m, 1H), 2.77-2.90 (m, 3H), 2.11-2.33 (m, 4H), 1.71-2.00 (m, 7H), 1.23-1.58 (m, 14H), 1.03-1.19 (m, 2H), 1.05 (s, 3H), 0.88 (s, 3H)

### Example 70: Synthesis of mixture of N-(3-(N-(N'-(4-(2-(3-(2,6-dioxopiperidine))-isoindole-1,3-dione))-6S-methyl-1S,3R-cyclohexanediamine))phenyl)-3-oxo-4-aza-5α-androst-1-ene-17β-carboxamide and N-(3-(N-(N'-(4-(2-(3-(2,6-dioxopiperidine))-isoindole-1,3-dione))-4R-methyl-1R,3S-cyclohexanediamine))phenyl)-3-oxo-4-aza-5α-androst-1-ene-17β-carboxamide (C70)

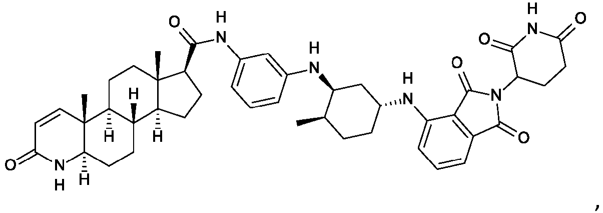

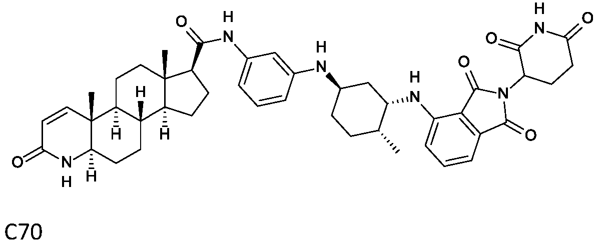

### Step 1: Synthesis of mixture of 3-(N-(N'-Boc-6R-methyl-1S,3R-cyclohexanediamine))aniline and 3-(N-(N'-Boc-4R-methyl-1R,3S-cyclohexanediamine))aniline (II-70)

### a. Synthesis of mixture of 3-(N-(6R-methyl-1S,3R-cyclohexanediamine))nitrobenzene and 3-(N-(4R-methyl-1R,3S-cyclohexanediamine))nitrobenzene (II-70-1)

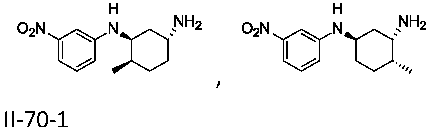

100 mg of *m*-fluoronitrobenzene and 900 mg of 6*R*-methyl-1*S*,3*R*-cyclohexanediamine were transferred into a three-necked flask. The mixture was heated to 100 °C in a nitrogen atmosphere and stirred for reaction for 12 h. The reaction solution was cooled to room temperature and concentrated to give a crude product. The crude product was purified by column chromatography (eluent: dichloromethane:methanol = 6:1) to give a pale yellow solid (compound II-70-1, 24 mg).
MS (ESI): (M+H)⁺, 250.1

### b. Synthesis of mixture of 3-(N-(N'-Boc-6R-methyl-1S,3R-cyclohexanediamine))nitrobenzene and 3-(N-(N'-Boc-4R-methyl-1R,3S-cyclohexanediamine))nitrobenzene (II-70-2)

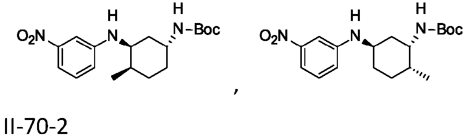

24 mg of compound II-70-1, 13.2 mg of triethylamine, 28 mg of Boc anhydride, and 2 mL of dichloromethane were added to a reaction flask, and the reaction system was stirred at room temperature for 30 min. The reaction solution was washed once with 0.5 mL of 1 mol/L aqueous hydrochloric acid and 0.5 mL of water, and the aqueous phase was extracted with dichloromethane (2.5 mL × 2). The organic phases were combined, dried over anhydrous sodium sulfate, and concentrated to give crude compound II-70-2 (36 mg).
MS (ESI): (M+H)⁺, 350.2

### c. Synthesis of mixture of 3-(N-(N'-Boc-6R-methyl-1S,3R-cyclohexanediamine))aniline and 3-(N-(N'-Boc-4R-methyl-1R,3S-cyclohexanediamine))aniline (II-70)

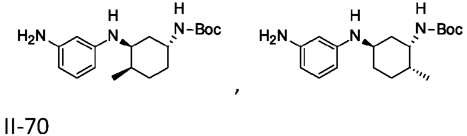

36 mg of crude compound II-70-2, 2 mL of methanol, and 3.6 mg of 5% palladium on carbon were added into a 100-mL hydrogenation reactor, and the system was incubated with continuous hydrogen introduction at 25 °C and 50 psi until the reaction was completed. The reaction solution was concentrated to give crude compound II-70 (33 mg).

### Step 2: Synthesis of mixture of N-(3-(N-(N'-Boc-6R-methyl-1S,3R-cyclohexanediamine))phenyl)-3-oxo-4-aza-5α-androst-1-ene-17β-carboxamide and N-(3-(N-(N'-Boc-4R-methyl-1R,3S-cyclohexanediamine))phenyl)-3-oxo-4-aza-5α-androst-1-ene-17β-carboxamide (IV-70)

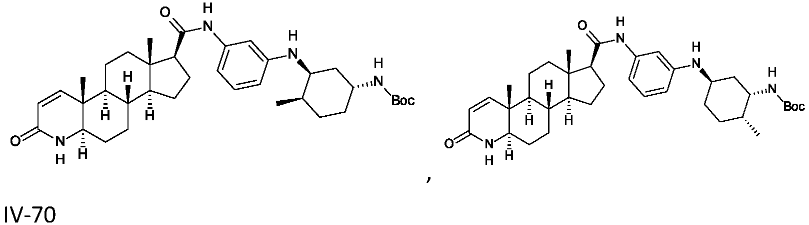

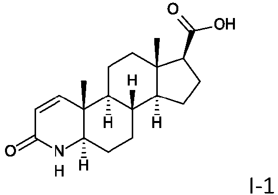

In a nitrogen atmosphere, 11 mg of compound I-1, 10 mg of crude compound II-70, 32.8 mg of pyBoP, 12.2 mg of DIPEA, and 0.5 mL of DMF were sequentially added into a reaction flask, and the mixture was stirred overnight until the reaction was completed. 2.5 mL of water was added into the reaction flask. The reaction solution was extracted with ethyl acetate (2.5 mL × 3). The organic phases were combined, sequentially washed once with 1 mL of 1 mol/L aqueous hydrochloric acid and 1 mL of saturated aqueous sodium carbonate, dried over anhydrous sodium sulfate, and concentrated to give a crude product. The crude product was purified by column chromatography (eluent: dichloromethane:methanol = 20:1) to give compound IV-70 (18.6 mg).
MS (ESI): (M+H)⁺, 619.4

### Step 3: Synthesis of mixture of N-(3-(N-(6R-methyl-1S,3R-cyclohexanediamine))phenyl)-3-oxo-4-aza-5α-androst-1-ene-17β-carboxamide and N-(3-(N-(4R-methyl-1R,3S-cyclohexanediamine))phenyl)-3-oxo-4-aza-5α-androst-1-ene-17β-carboxamide (V-70)

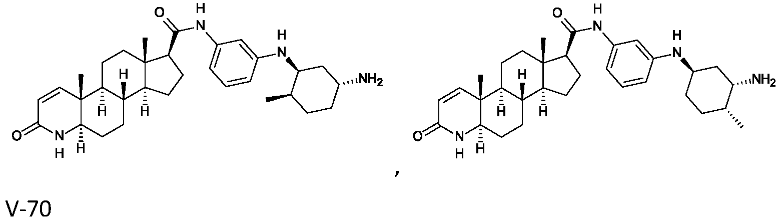

At room temperature, 18.6 mg of compound IV-70 and 1 mL of dichloromethane were added into a reaction flask, before 0.1 mL of trifluoroacetic acid was dropwise added. The reaction system was incubated for 10 min. The reaction solution was concentrated to give crude compound V-70 (14.5 mg).
MS (ESI): (M+H)⁺, 519.4

### Step 4: Synthesis of mixture of N-(3-(N-(N'-(4-(2-(3-(2,6-dioxopiperidine))-isoindole-1,3-dione))-6R-methyl-1S,3R-cyclohexanediamine))phenyl)-3-oxo-4-aza-5α-androst-1-ene-17β-carboxamide and N-(3-(N-(N'-(4-(2-(3-(2,6-dioxopiperidine))-isoindole-1,3-dione))-4R-methyl-1R,3S-cyclohexanediamine))phenyl)-3-oxo-4-aza-5α-androst-1-ene-17β-carboxamide (C70)

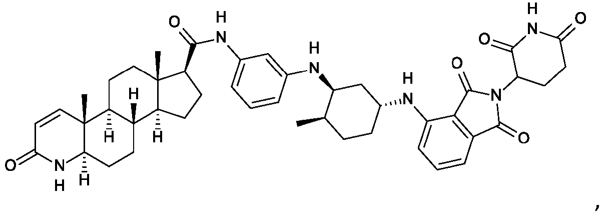

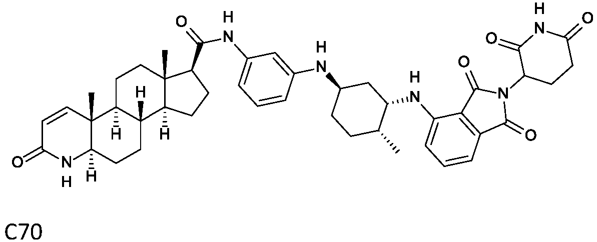

14.5 mg of crude compound V-70, 11.3 mg of 4-fluoro-2-(3-(2,6-dioxopiperidine))-isoindole-1,3-dione, 26.3 mg of DIPEA, and 1 mL of DMF were sequentially added into a reaction flask. The reaction flask was treated in a microwave reactor for 10 min of reaction at 50 Hz. 2.5 mL of water was added, and the mixture was extracted with water and dichloromethane together (2.5 mL × 3). The organic phases were combined, dried over anhydrous sodium sulfate, and concentrated to give a crude product. The crude product was purified by silica gel column chromatography (eluent: dichloromethane:methanol = 15:1) to give compound C70 (9.6 mg).
The mass spectral data for compound C70 are, MS (ESI): (M+H)⁺, 775.4

### Example 71: Synthesis of mixture of N-(3-(N-(N'-(4-(2-(3-(2,6-dioxopiperidine))-isoindole-1,3-dione))-(1R,4S)-bicyclo[2,2,1]heptane-2R,6R-diamine))phenyl)-3-oxo-4-aza-5α-androst-1-ene-17β-carboxamide and N-(3-(N-(N'-(4-(2-(3-(2,6-dioxopiperidine))-isoindole-1,3-dione))-(1S,4R)-bicyclo[2,2,1]heptane-2R,6R-diamine))phenyl)-3-oxo-4-aza-5α-androst-1-ene-17β-carboxamide (C71)

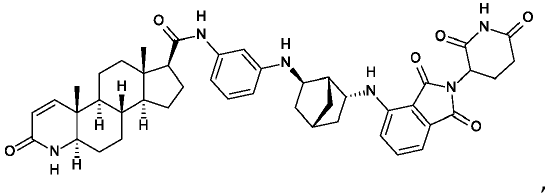

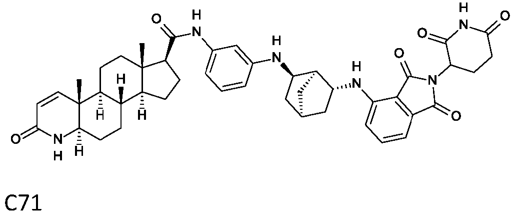

### Step 1: Synthesis of mixture of 3-(N-(N'-Boc-(1R,4S)-bicyclo[2,2,1]heptane-2R,6R-diamine))aniline and 3-(N-(N'-Boc-(1S,4R)-bicyclo[2,2,1]heptane-2R,6R-diamine))aniline (II-71)

### a. Synthesis of mixture of 3-(N-((1R,4S)-bicyclo[2,2,1]heptane-2R,6R-diamine))nitrobenzene and 3-(N-((1S,4R)-bicyclo[2,2,1]heptane-2R,6R-diamine))nitrobenzene (II-71-1)

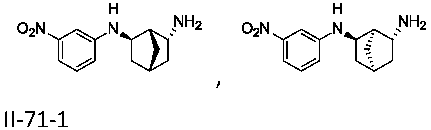

100 mg of *m*-fluoronitrobenzene and 893 mg of (1*R*,4*S*)-bicyclo[2,2,1]heptane-2*R*,6*R*-diamine were transferred into a three-necked flask. The mixture was heated to 100 °C in a nitrogen atmosphere and stirred for reaction for 12 h. The reaction solution was cooled to room temperature and concentrated to give a crude product. The crude product was purified by column chromatography (eluent: dichloromethane:methanol = 6:1) to give compound II-71-1 (21 mg).
MS (ESI): (M+H)⁺, 248.1

### b. Synthesis of mixture of 3-(N-(N'-Boc-(1R,4S)-bicyclo[2,2,1]heptane-2R,6R-diamine))nitrobenzene and 3-(N-(N'-Boc-(1S,4R)-bicyclo[2,2,1]heptane-2R,6R-diamine))nitrobenzene (II-71-2)

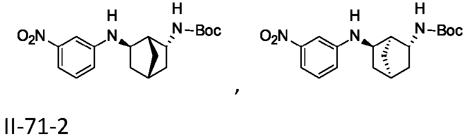

21 mg of compound II-71-1, 9.4 mg of triethylamine, 20.4 mg of Boc anhydride, and 2 mL of dichloromethane were added to a reaction flask, and the reaction system was stirred at room temperature for 30 min. The reaction solution was washed once with 0.5 mL of 1 mol/L aqueous hydrochloric acid and 0.5 mL of water respectively, and the aqueous phase was extracted with dichloromethane (2.5 mL × 2). The organic phases were combined, dried over anhydrous sodium sulfate, and concentrated to give crude compound II-71-2 (29.5 mg).
MS (ESI): (M+H)⁺, 348.2

### c. Synthesis of mixture of 3-(N-(N'-Boc-(1R,4S)-bicyclo[2,2,1]heptane-2R,6R-diamine))aniline and 3-(N-(N'-Boc-(1S,4R)-bicyclo[2,2,1]heptane-2R,6R-diamine))aniline (II-71)

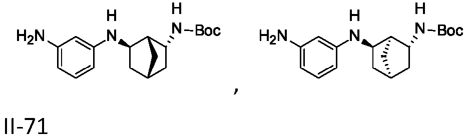

29.5 mg of crude compound II-71-2, 2 mL of methanol, and 3 mg of 5% palladium on carbon were added into a 100-mL hydrogenation reactor, and the system was incubated with continuous hydrogen introduction at 25 °C and 50 psi until the reaction was completed. The reaction solution was concentrated to give crude compound II-71 (27 mg).
MS (ESI): (M+H)⁺, 318.2

### Step 2: Synthesis of mixture of N-(3-(N-(N'-Boc-(1R,4S)-bicyclo[2,2,1]heptane-2R,6R-diamine))phenyl)-3-oxo-4-aza-5α-androst-1-ene-17β-carboxamide and N-(3-(N-(N'-Boc-(1S,4R)-bicyclo[2,2,1]heptane-2R,6R-diamine))phenyl)-3-oxo-4-aza-5α-androst-1-ene-17β-carboxamide (IV-71)

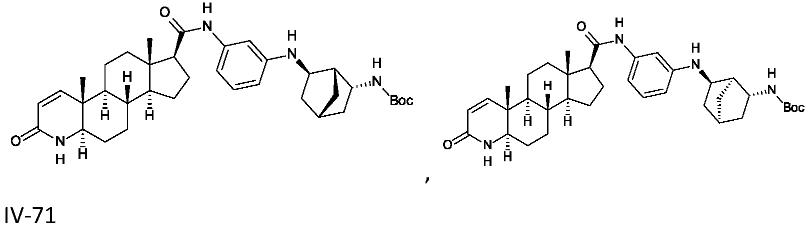

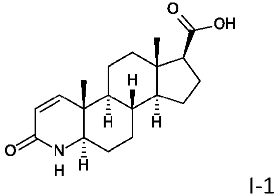

In a nitrogen atmosphere, 11 mg of compound I-1, 10 mg of crude compound II-71, 32.8 mg of pyBoP, 12.2 mg of DIPEA, and 0.5 mL of DMF were sequentially added into a reaction flask, and the mixture was stirred overnight until the reaction was completed. 2.5 mL of water was added into the reaction flask. The reaction solution was extracted with ethyl acetate (2.5 mL × 3). The organic phases were combined, sequentially washed once with 1 mL of 1 mol/L aqueous hydrochloric acid and 1 mL of saturated aqueous sodium carbonate, dried over anhydrous sodium sulfate, and concentrated to give a crude product. The crude product was purified by column chromatography (eluent: dichloromethane:methanol = 20:1) to give compound IV-71 (17 mg).
MS (ESI): (M+H)⁺, 617.4

### Step 3: Synthesis of mixture of N-(3-(N-((1R,4S)-bicyclo[2,2,1]heptane-2R,6R-diamine))phenyl)-3-oxo-4-aza-5α-androst-1-ene-17β-carboxamide and N-(3-(N-((1S,4R)-bicyclo[2,2,1]heptane-2R,6R-diamine))phenyl)-3-oxo-4-aza-5α-androst-1-ene-17β-carboxamide (V-71)

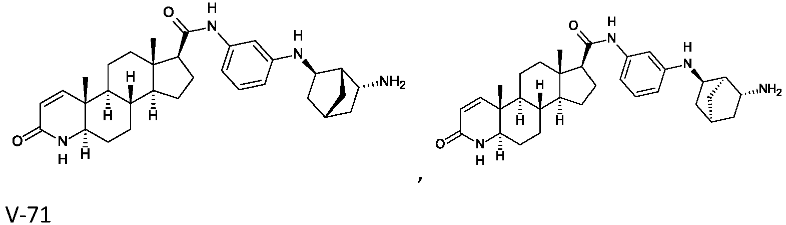

At room temperature, 17 mg of compound IV-71 and 1 mL of dichloromethane were added into a reaction flask, before 0.1 mL of trifluoroacetic acid was dropwise added. The reaction system was incubated for 10 min. The reaction solution was concentrated to give crude compound V-71 (14 mg).
MS (ESI): (M+H)⁺, 517.3

### Step 4: Synthesis of mixture of N-(3-(N-(N'-(4-(2-(3-(2,6-dioxopiperidine))-isoindole-1,3-dione))-(1R,4S)-bicyclo[2,2,1]heptane-2R,6R-diamine))phenyl)-3-oxo-4-aza-5α-androst-1-ene-17β-carboxamide and N-(3-(N-(N'-(4-(2-(3-(2,6-dioxopiperidine))-isoindole-1,3-dione))-(1S,4R)-bicyclo[2,2,1]heptane-2R,6R-diamine))phenyl)-3-oxo-4-aza-5α-androst-1-ene-17β-carboxamide (C71)

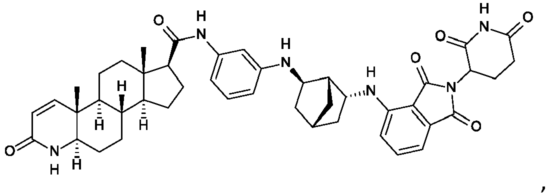

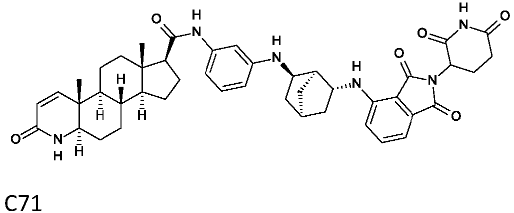

14 mg of crude compound V-71, 13 mg of 4-fluoro-2-(3-(2,6-dioxopiperidine))-isoindole-1,3-dione, 30 mg of DIPEA, and 1 mL of DMF were sequentially added into a reaction flask. The reaction flask was treated in a microwave reactor for 10 min of reaction at 50 Hz. 2.5 mL of water was added, and the mixture was extracted with water and dichloromethane together (2.5 mL × 3). The organic phases were combined, dried over anhydrous sodium sulfate, and concentrated to give a crude product. The crude product was purified by column chromatography (eluent: dichloromethane:methanol = 15:1) to give compound C71 (9.2 mg). The mass spectral data for compound C71 are, MS (ESI): (M+H)⁺, 773.4

### Example 72: Synthesis of mixture of N-(3-(N-(N'-(4-(2-(3-(2,6-dioxopiperidine))-isoindole-1,3-dione))-(1R,4S)-bicyclo[2,2,2]octane-2R,6R-diamine))phenyl)-3-oxo-4-aza-5α-androst-1-ene-17β-carboxamide and N-(3-(N-(N'-(4-(2-(3-(2,6-dioxopiperidine))-isoindole-1,3-dione))-(1S,4R)-bicyclo[2,2,2]octane-2R,6R-diamine))phenyl)-3-oxo-4-aza-5α-androst-1-ene-17β-carboxamide (C72)

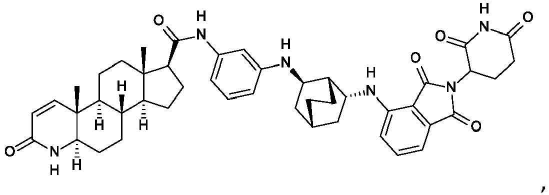

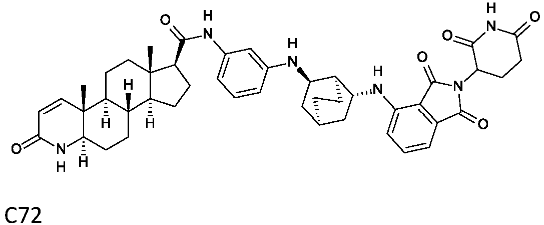

### Step 1: Synthesis of mixture of 3-(N-(N'-Boc-(1R,4S)-bicyclo[2,2,2]octane-2R,6R-diamine))aniline and 3-(N-(N'-Boc-(1S,4R)-bicyclo[2,2,2]octane-2R,6R-diamine))aniline (II-72)

### a. Synthesis of mixture of 3-(N-((1R,4S)-bicyclo[2,2,2]octane-2R,6R-diamine))nitrobenzene and 3-(N-((1S,4R)-bicyclo[2,2,2]octane-2R,6R-diamine))nitrobenzene (II-72-1)

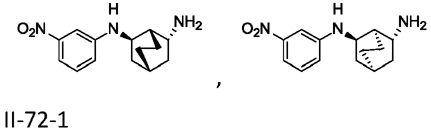

100 mg of *m*-fluoronitrobenzene and 985 mg of (1*R*,4*S*)-bicyclo[2,2,2]octane-2*R*,6*R*-diamine were transferred into a three-necked flask. The mixture was heated to 100 °C in a nitrogen atmosphere and stirred for reaction for 12 h. The reaction solution was cooled to room temperature and concentrated to give a crude product. The crude product was purified by column chromatography (eluent: dichloromethane:methanol = 6:1) to give compound II-72-1 (21 mg).
MS (ESI): (M+H)⁺, 262.1

### b. Synthesis of mixture of 3-(N-(N'-Boc-(1R,4S)-bicyclo[2,2,2]octane-2R,6R-diamine))nitrobenzene and 3-(N-(N'-Boc-(1S,4R)-bicyclo[2,2,2]octane-2R,6R-diamine))nitrobenzene (II-72-2)

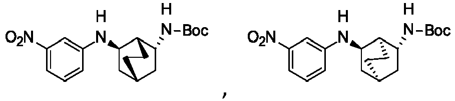

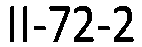

21 mg of compound II-72-1, 8.9 mg of triethylamine, 19.3 mg of Boc anhydride, and 2 mL of dichloromethane were added to a reaction flask, and the reaction system was stirred at room temperature for 30 min. The reaction solution was washed once with 0.5 mL of 1 mol/L aqueous hydrochloric acid and 0.5 mL of water respectively, and the aqueous phase was extracted with dichloromethane (2.5 mL × 2). The organic phases were combined, dried over anhydrous sodium sulfate, and concentrated to give crude compound II-72-2 (29 mg).
MS (ESI): (M+H)⁺, 362.2

### c. Synthesis of mixture of 3-(N-(N'-Boc-(1R,4S)-bicyclo[2,2,2]octane-2R,6R-diamine))aniline and 3-(N-(N'-Boc-(1S,4R)-bicyclo[2,2,2]octane-2R,6R-diamine))aniline (II-72)

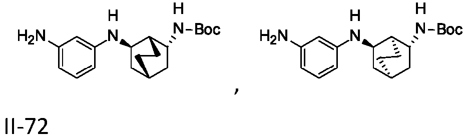

29 mg of crude compound 11-72-2, 2 mL of methanol, and 3 mg of 5% palladium on carbon were added into a 100-mL hydrogenation reactor, and the system was incubated with continuous hydrogen introduction at 25 °C and 50 psi until the reaction was completed. The reaction solution was concentrated to give crude compound II-72 (26 mg).
MS (ESI): (M+H)⁺, 332.2

### Step 2: Synthesis of mixture of N-(3-(N-(N'-Boc-(1R,4S)-bicyclo[2,2,2]octane-2R,6R-diamine))phenyl)-3-oxo-4-aza-5α-androst-1-ene-17β-carboxamide and N-(3-(N-(N'-Boc-(1S,4R)-bicyclo[2,2,2]octane-2R,6R-diamine))phenyl)-3-oxo-4-aza-5α-androst-1-ene-17β-carboxamide (IV-72)

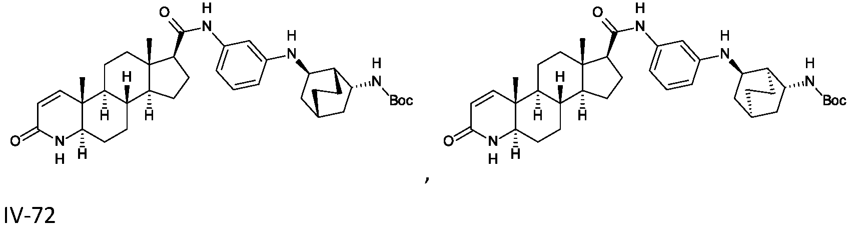

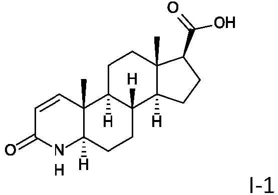

In a nitrogen atmosphere, 11 mg of compound I-1, 10.5 mg of crude compound II-72, 32.8 mg of pyBoP, 12.2 mg of DIPEA, and 0.5 mL of DMF were sequentially added into a reaction flask, and the mixture was stirred overnight until the reaction was completed. 2.5 mL of water was added into the reaction flask. The reaction solution was extracted with ethyl acetate (2.5 mL × 3). The organic phases were combined, sequentially washed once with 1 mL of 1 mol/L aqueous hydrochloric acid and 1 mL of saturated aqueous sodium carbonate, dried over anhydrous sodium sulfate, and concentrated to give a crude product. The crude product was purified by column chromatography (eluent: dichloromethane:methanol = 20:1) to give compound IV-72 (17.5 mg).
MS (ESI): (M+H)⁺, 631.4

### Step 3: Synthesis of mixture of N-(3-(N-((1R,4S)-bicyclo[2,2,2]octane-2R,6R-diamine))phenyl)-3-oxo-4-aza-5α-androst-1-ene-17β-carboxamide and N-(3-(N-((1S,4R)-bicyclo[2,2,2]octane-2R,6R-diamine))phenyl)-3-oxo-4-aza-5α-androst-1-ene-17β-carboxamide (V-72)

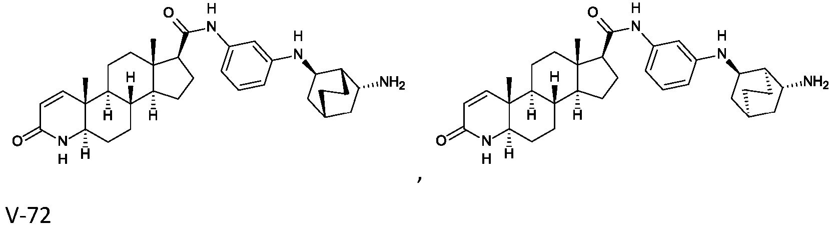

At room temperature, 17.5 mg of compound IV-72 and 1 mL of dichloromethane were added into a reaction flask, before 0.1 mL of trifluoroacetic acid was dropwise added. The reaction system was incubated for 10 min. The reaction solution was concentrated to give crude compound V-72 (14 mg).
MS (ESI): (M+H)⁺, 531.4

### Step 4: Synthesis of mixture of N-(3-(N-(N'-(4-(2-(3-(2,6-dioxopiperidine))-isoindole-1,3-dione))-(1R,4S)-bicyclo[2,2,2]octane-2R,6R-diamine))phenyl)-3-oxo-4-aza-5α-androst-1-ene-17β-carboxamide and N-(3-(N-(N'-(4-(2-(3-(2,6-dioxopiperidine))-isoindole-1,3-dione))-(1S,4R)-bicyclo[2,2,2]octane-2R,6R-diamine))phenyl)-3-oxo-4-aza-5α-androst-1-ene-17β-carboxamide (C72)

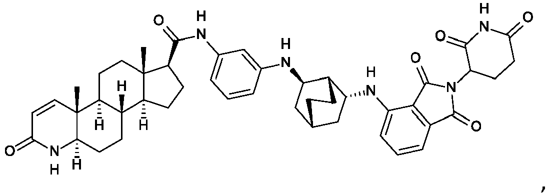

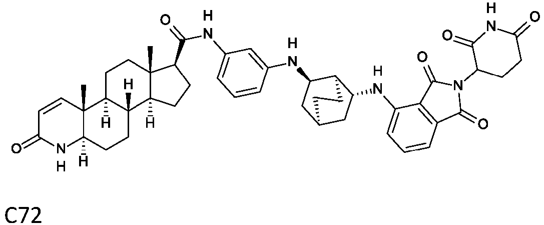

14 mg of crude compound V-72, 13 mg of 4-fluoro-2-(3-(2,6-dioxopiperidine))-isoindole-1,3-dione, 30 mg of DIPEA, and 1 mL of DMF were sequentially added into a reaction flask. The reaction flask was treated in a microwave reactor for 10 min of reaction at 50 Hz. 2.5 mL of water was added, and the mixture was extracted with water and dichloromethane together (2.5 mL × 3). The organic phases were combined, dried over anhydrous sodium sulfate, and concentrated to give a crude product. The crude product was purified by column chromatography (eluent: dichloromethane:methanol = 15:1) to give compound C72 (7.8 mg). The mass spectral data for compound C72 are, MS (ESI): (M+H)⁺, 787.4

### Example 73: Synthesis of mixture of N-(3-(N-(N'-(4-(2-(3-(2,6-dioxopiperidine))-isoindole-1,3-dione))-(1R,5S)-bicyclo[3,2,2]nonane-7R,8R-diamine))phenyl)-3-oxo-4-aza-5α-androst-1-ene-17β-carboxamide and N-(3-(N-(N'-(4-(2-(3-(2,6-dioxopiperidine))-isoindole-1,3-dione))-(1S,5R)-bicyclo[3,2,2]nonane-7R,8R-diamine))phenyl)-3-oxo-4-aza-5α-androst-1-ene-17β-carboxamide (C73)

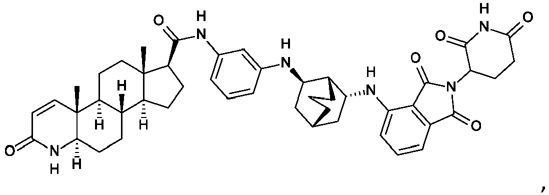

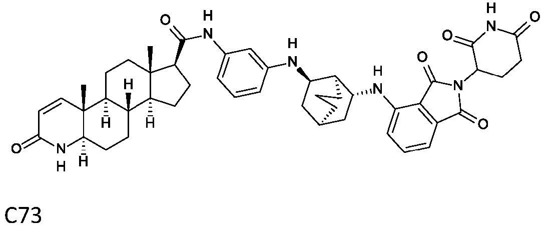

### Step 1: Synthesis of mixture of 3-(N-(N'-Boc-(1R,5S)-bicyclo[3,2,2]nonane-7R,8R-diamine))aniline and 3-(N-(N'-Boc-(1S,5R)-bicyclo[3,2,2]nonane-7R,8R-diamine))aniline (II-73)

### a. Synthesis of mixture of 3-(N-((1R,5S)-bicyclo[3,2,2]nonane-7R,8R-diamine))nitrobenzene and 3-(N-((1S,5R)-bicyclo[3,2,2]nonane-7R,8R-diamine))nitrobenzene (II-73-1)

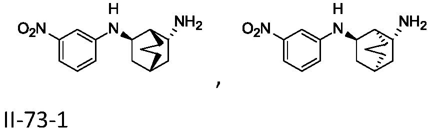

100 mg of *m*-fluoronitrobenzene and 1.09 g of (1*R*,5*S*)-bicyclo[3,2,2]nonane-7*R*,8*R*-diamine were transferred into a three-necked flask. The mixture was heated to 100 °C in a nitrogen atmosphere and stirred for reaction for 12 h. The reaction solution was cooled to room temperature and concentrated to give a crude product. The crude product was purified by column chromatography (eluent: dichloromethane:methanol = 6:1) to give compound II-73-1 (19 mg).
MS (ESI): (M+H)⁺, 276.2

### b. Synthesis of mixture of 3-(N-(N'-Boc-(1R,5S)-bicyclo[3,2,2]nonane-7R,8R-diamine))nitrobenzene and 3-(N-(N'-Boc-(1S,5R)-bicyclo[3,2,2]nonane-7R,8R-diamine))nitrobenzene (II-73-2)

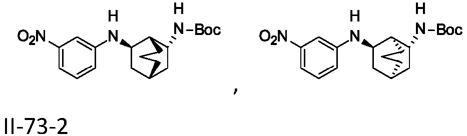

19 mg of compound II-73-1, 7.6 mg of triethylamine, 16.6 mg of Boc anhydride, and 2 mL of dichloromethane were added to a reaction flask, and the reaction system was stirred at room temperature for 30 min. The reaction solution was washed once with 0.5 mL of 1 mol/L aqueous hydrochloric acid and 0.5 mL of water, and the aqueous phase was extracted with dichloromethane (2.5 mL × 2). The organic phases were combined, dried over anhydrous sodium sulfate, and concentrated to give crude compound II-73-2 (25 mg).
MS (ESI): (M+H)⁺, 376.2

### c. Synthesis of mixture of 3-(N-(N'-Boc-(1R,5S)-bicyclo[3,2,2]nonane-7R,8R-diamine))aniline and 3-(N-(N'-Boc-(1S,5R)-bicyclo[3,2,2]nonane-7R,8R-diamine))aniline (II-73)

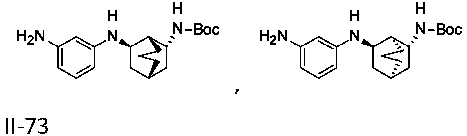

25 mg of crude compound II-73-2, 2 mL of methanol, and 2.5 mg of 5% palladium on carbon were added into a 100-mL hydrogenation reactor, and the system was incubated with continuous hydrogen introduction at 25 °C and 50 psi until the reaction was completed. The reaction solution was concentrated to give crude compound II-73 (23 mg).
MS (ESI): (M+H)⁺, 346.2

### Step 2: Synthesis of mixture of N-(3-(N-(N'-Boc-(1R,5S)-bicyclo[3,2,2]nonane-7R,8R-diamine))phenyl)-3-oxo-4-aza-5α-androst-1-ene-17β-carboxamide and N-(3-(N-(N'-Boc-(1S,5R)-bicyclo[3,2,2]nonane-7R,8R-diamine))phenyl)-3-oxo-4-aza-5α-androst-1-ene-17β-carboxamide (IV-73)

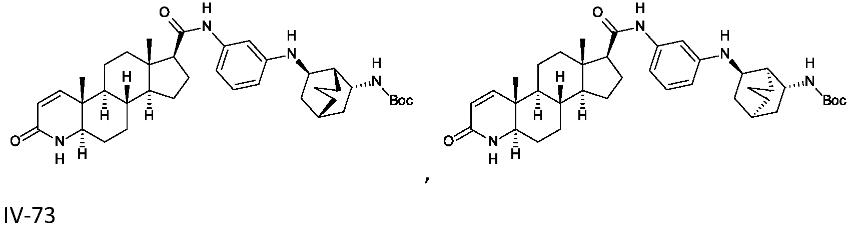

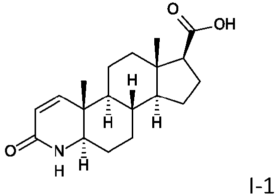

In a nitrogen atmosphere, 11 mg of compound I-1, 10.9 mg of crude compound II-73, 32.8 mg of pyBoP, 12.2 mg of DIPEA, and 0.5 mL of DMF were sequentially added into a reaction flask, and the mixture was stirred overnight until the reaction was completed. 2.5 mL of water was added into the reaction flask. The reaction solution was extracted with ethyl acetate (2.5 mL × 3). The organic phases were combined, sequentially washed once with 1 mL of 1 mol/L aqueous hydrochloric acid and 1 mL of saturated aqueous sodium carbonate respectively, dried over anhydrous sodium sulfate, and concentrated to give a crude product. The crude product was subjected to column chromatography (eluent: dichloromethane:methanol = 20:1) to give compound IV-73 (17.2 mg).
MS (ESI): (M+H)⁺, 645.4

### Step 3: Synthesis of mixture of N-(3-(N-((1R,5S)-bicyclo[3,2,2]nonane-7R,8R-diamine))phenyl)-3-oxo-4-aza-5α-androst-1-ene-17β-carboxamide and N-(3-(N-((1S,5R)-bicyclo[3,2,2]nonane-7R,8R-diamine))phenyl)-3-oxo-4-aza-5α-androst-1-ene-17β-carboxamide (V-73)

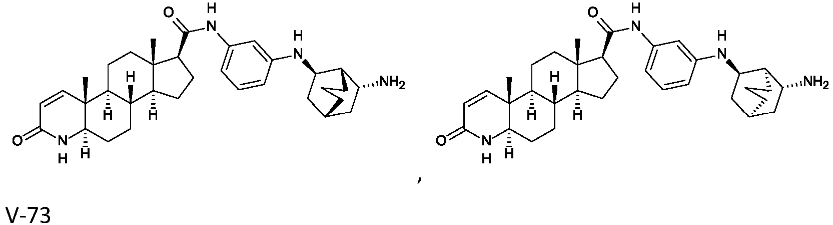

At room temperature, 17.2 mg of compound IV-73 and 1 mL of dichloromethane were added into a reaction flask, before 0.1 mL of trifluoroacetic acid was dropwise added. The reaction system was incubated for 10 min. The reaction solution was concentrated to give crude compound V-73 (14.2 mg).
MS (ESI): (M+H)⁺, 545.4

### Step 4: Synthesis of mixture of N-(3-(N-(N'-(4-(2-(3-(2,6-dioxopiperidine))-isoindole-1,3-dione))-(1R,5S)-bicyclo[3,2,2]nonane-7R,8R-diamine))phenyl)-3-oxo-4-aza-5α-androst-1-ene-17β-carboxamide and N-(3-(N-(N'-(4-(2-(3-(2,6-dioxopiperidine))-isoindole-1,3-dione))-(1S,5R)-bicyclo[3,2,2]nonane-7R,8R-diamine))phenyl)-3-oxo-4-aza-5α-androst-1-ene-17β-carboxamide (C73)

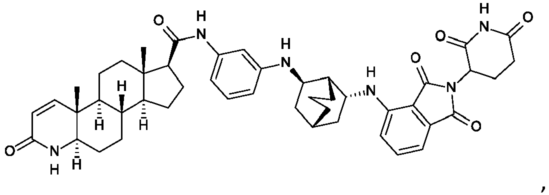

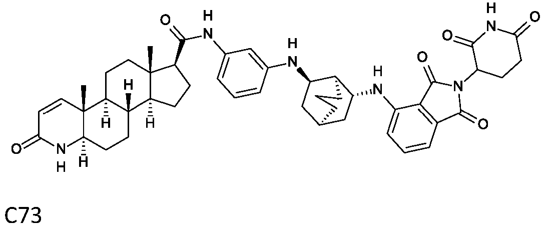

14.2 mg of crude compound V-73, 13 mg of 4-fluoro-2-(3-(2,6-dioxopiperidine))-isoindole-1,3-dione, 30 mg of DIPEA, and 1 mL of DMF were sequentially added into a reaction flask. The reaction flask was treated in a microwave reactor for 10 min of reaction at 50 Hz. 2.5 mL of water was added, and the mixture was extracted with water and dichloromethane together (2.5 mL × 3). The organic phases were combined, dried over anhydrous sodium sulfate, and concentrated to give a crude product. The crude product was purified by column chromatography (eluent: dichloromethane:methanol = 15:1) to give compound C73 (7.6 mg). The mass spectral data for compound C73 are, MS (ESI): (M+H)⁺, 801.4

### Example 74: Synthesis of N-(3-(N-(N'-(4-(2-(3-(2,6-dioxopiperidine))-isoindole-1,3-dione))-5,5-difluoro-1S,3S-cyclohexanediamine))phenyl)-3-oxo-4-aza-5α-androst-1-ene-17β-carboxamide (C74)

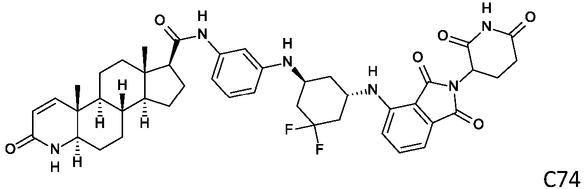

### Step 1: Synthesis of 3-(N-(N'-Boc-5,5-difluoro-1S,3S-cyclohexanediamine))aniline (II-74)

### a. Synthesis of 3-(N-(5,5-difluoro-1S,3S-cyclohexanediamine))nitrobenzene (II-74-1)

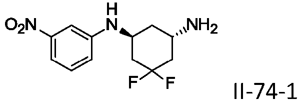

100 mg of *m*-fluoronitrobenzene and 1.06 g of 5,5-difluoro-15,35-cyclohexanediamine were transferred into a three-necked flask. The mixture was heated to 100 °C in a nitrogen atmosphere and stirred for reaction for 12 h. The reaction solution was cooled to room temperature and subjected to column chromatography (eluent: dichloromethane:methanol = 5:1) to give a pale yellow solid (compound II-74-1, 20 mg).
MS (ESI): (M+H)⁺, 272.1

### b. Synthesis of 3-(N-(N'-Boc-5,5-difluoro-1S,3S-cyclohexanediamine))nitrobenzene (II-74-2)

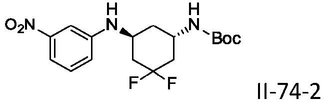

20 mg of compound II-74-1, 8.2 mg of triethylamine, 18 mg of Boc anhydride, and 2 mL of dichloromethane were sequentially added to a reaction flask, and the reaction system was stirred at room temperature for 30 min. The reaction solution was washed once with 0.5 mL of 1 mol/L aqueous hydrochloric acid and 0.5 mL of water respectively, and the aqueous phase was extracted with dichloromethane (2.5 mL × 2). The organic phases were combined, dried over anhydrous sodium sulfate, and concentrated to give crude compound II-74-2 (27 mg).
MS (ESI): (M+H)⁺, 372.2

### c. Synthesis of 3-(N-(N'-Boc-5,5-difluoro-1S,3S-cyclohexanediamine))aniline (II-74)

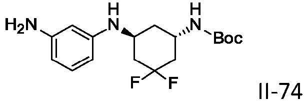

27 mg of crude compound II-74-2, 2 mL of methanol, and 2.7 mg of 5% palladium on carbon were sequentially added into a 100-mL hydrogenation reactor, and the system was incubated with continuous hydrogen introduction at 25 °C and 50 psi until the reaction was completed. The reaction solution was concentrated to give crude compound II-74 (24.8 mg).
MS (ESI): (M+H)⁺, 342.2

### Step 2: Synthesis of N-(3-(N-(N'-Boc-5,5-difluoro-1S,3S-cyclohexanediamine))phenyl)-3-oxo-4-aza-5α-androst-1-ene-17β-carboxamide (IV-74)

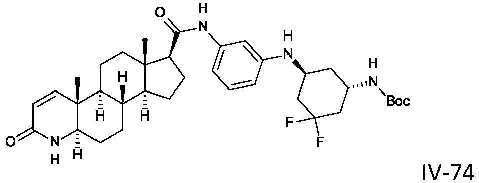

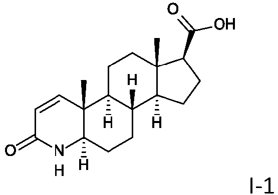

In a nitrogen atmosphere, 11 mg of compound I-1, 10.7 mg of crude compound II-74, 32.8 mg of pyBoP, 12.2 mg of DIPEA, and 0.5 mL of DMF were sequentially added into a reaction flask, and the mixture was stirred overnight until the reaction was completed. 2.5 mL of water was added into the reaction flask. The reaction solution was extracted with ethyl acetate (2.5 mL × 3). The organic phases were combined, sequentially washed once with 1 mL of 1 mol/L aqueous hydrochloric acid and 1 mL of saturated aqueous sodium carbonate, dried over anhydrous sodium sulfate, and concentrated to give a crude product. The crude product was purified by column chromatography (eluent: dichloromethane:methanol = 15:1) to give compound IV-74 (18.3 mg).
MS (ESI): (M+H)⁺, 641.2

### Step 3: Synthesis of N-(3-(N-(5,5-difluoro-1S,3S-cyclohexanediamine))phenyl)-3-oxo-4-aza-5α-androst-1-ene-17β-carboxamide (V-74)

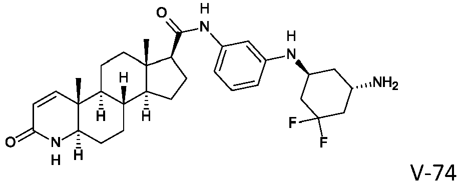

At room temperature, 18.3 mg of compound IV-74 and 1 mL of dichloromethane were added into a reaction flask, before 0.1 mL of trifluoroacetic acid was dropwise added. The reaction system was incubated for 10 min. The reaction solution was concentrated to give crude compound V-74 (14.8 mg).
MS (ESI): (M+H)⁺, 541.3

### Step 4: Synthesis of N-(3-(N-(N'-(4-(2-(3-(2,6-dioxopiperidine))-isoindole-1,3-dione))-5,5-difluoro-1S,3S-cyclohexanediamine))phenyl)-3-oxo-4-aza-5α-androst-1-ene-17β-carboxamide (C74)

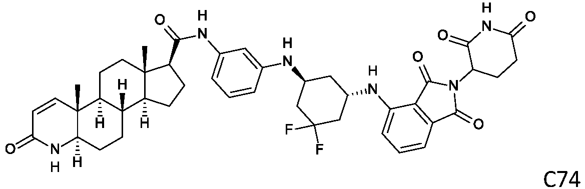

14.8 mg of crude compound V-74, 13 mg of 4-fluoro-2-(3-(2,6-dioxopiperidine))-isoindole-1,3-dione, 30 mg of DIPEA, and 1 mL of DMF were sequentially added into a reaction flask. The reaction flask was treated in a microwave reactor for 10 min of reaction at 50 Hz. 2.5 mL of water was added, and the mixture was extracted with water and dichloromethane together (2.5 mL × 3). The organic phases were combined, dried over anhydrous sodium sulfate, and concentrated to give a crude product. The crude product was purified by column chromatography (eluent: dichloromethane:methanol = 15:1) to give compound C74 (7 mg). The mass spectral data for compound C74 are, MS (ESI): (M+H)⁺, 797.4

The nuclear magnetic resonance data for compound C74 are as follows:
H NMR (400 MHz, CDCl₃) δ: 8.78-8.80 (m, 1H), 7.48-7.50 (m, 1H), 7.12-7.28 (m, 4H), 6.75-6.82 (m, 2H), 6.55-6.59 (m, 1H), 6.25-6.35 (m, 2H), 5.95 (d, 1H, J=12Hz), 5.90 (s, 1H), 5.13-5.18 (m, 1H), 4.02-4.09 (m, 1H), 3.95-3.99 (m, 1H), 3.50-3.59 (m, 1H), 2.80-2.98 (m, 3H), 2.00-2.43 (m, 8H), 1.71-1.90 (m, 5H), 1.15-1.58 (m, 8H), 1.21-1.31 (m, 2H), 1.10 (s, 3H), 0.92 (s, 3H)

### Example 75: Synthesis of N-(3-(N-(N'-(4-(2-(3-(2,6-dioxopiperidine))-isoindole-1,3-dione))-tetrahydropyran-3S,5S-diamine))phenyl)-3-oxo-4-aza-5α-androst-1-ene-17β-carboxamide (C75)

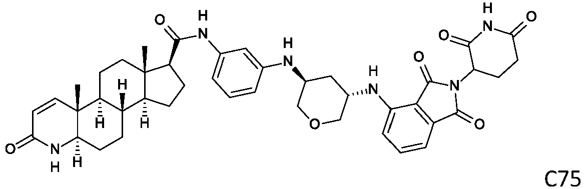

### Step 1: Synthesis of 3-(N-(N'-Boc-tetrahydropyran-3S,5S-diamine))aniline (II-75)

### a. Synthesis of 3-(N-(tetrahydropyran-3S,5S-diamine))nitrobenzene (II-75-1)

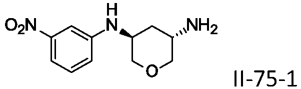

100 mg of *m*-fluoronitrobenzene and 822 mg of tetrahydrofuran-35,55-diamine were transferred into a three-necked flask. The mixture was heated to 100 °C in a nitrogen atmosphere and stirred for reaction for 12 h. The reaction solution was cooled to room temperature and subjected to column chromatography (eluent: dichloromethane:methanol = 5:1) to give a pale yellow solid (compound II-75-1, 12 mg).
MS (ESI): (M+H)⁺, 238.1

### b. Synthesis of 3-(N-(N'-Boc-tetrahydropyran-3S,5S-diamine))nitrobenzene (II-75-2)

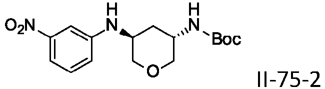

12 mg of compound II-75-1, 5.6 mg of triethylamine, 12 mg of Boc anhydride, and 2 mL of dichloromethane were sequentially added to a reaction flask, and the reaction system was stirred at room temperature for 30 min. The reaction solution was washed once with 0.5 mL of 1 mol/L aqueous hydrochloric acid and 0.5 mL of water, and the aqueous phase was extracted with dichloromethane (2.5 mL × 2). The organic phases were combined, dried over anhydrous sodium sulfate, and concentrated to give crude compound II-75-2 (17 mg).
MS (ESI): (M+H)⁺, 338.2

### c. Synthesis of 3-(N-(N'-Boc-tetrahydropyran-3S,5S-diamine))aniline (II-75)

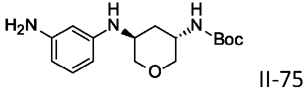

17 mg of crude compound II-75-2, 2 mL of methanol, and 1.7 mg of 5% palladium on carbon were sequentially added into a 100-mL hydrogenation reactor, and the system was incubated with continuous hydrogen introduction at 25 °C and 50 psi until the reaction was completed. The reaction solution was concentrated to give crude compound II-75 (15.4 mg).
MS (ESI): (M+H)⁺, 308.2

### Step 2: Synthesis of N-(3-(N-(N'-Boc-tetrahydropyran-3S,5S-diamine))phenyl)-3-oxo-4-aza-5α-androst-1-ene-17β-carboxamide (IV-75)

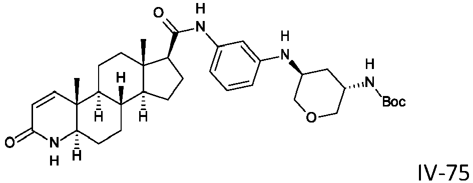

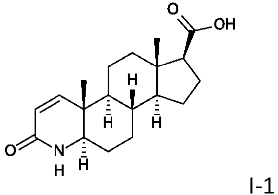

In a nitrogen atmosphere, 11 mg of compound I-1, 9.7 mg of crude compound II-75, 32.8 mg of pyBoP, 12.2 mg of DIPEA, and 0.5 mL of DMF were sequentially added into a reaction flask, and the mixture was stirred overnight until the reaction was completed. 2.5 mL of water was added into the reaction flask. The reaction solution was extracted with ethyl acetate (2.5 mL × 3). The organic phases were combined, sequentially washed once with 1 mL of 1 mol/L aqueous hydrochloric acid and 1 mL of saturated aqueous sodium carbonate, dried over anhydrous sodium sulfate, and concentrated to give a crude product. The crude product was purified by column chromatography (eluent: dichloromethane:methanol = 15:1) to give compound IV-75 (19 mg).
MS (ESI): (M+H)⁺, 607.4

### Step 3: Synthesis of N-(3-(N-(tetrahydropyran-3S,5S-diamine))phenyl)-3-oxo-4-aza-5α-androst-1-ene-17β-carboxamide (V-75)

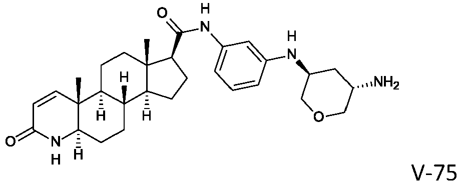

At room temperature, 19 mg of compound IV-75 and 1 mL of dichloromethane were added into a reaction flask, before 0.1 mL of trifluoroacetic acid was dropwise added. The reaction system was incubated for 10 min. The reaction solution was concentrated to give crude compound V-75 (15.8 mg).
MS (ESI): (M+H)⁺, 507.3

### Step 4: Synthesis of N-(3-(N-(N'-(4-(2-(3-(2,6-dioxopiperidine))-isoindole-1,3-dione))-tetrahydropyran-3S,5S-diamine))phenyl)-3-oxo-4-aza-5α-androst-1-ene-17β-carboxamide (C75)

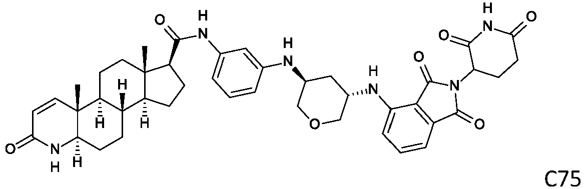

15.8 mg of crude compound V-75, 13 mg of 4-fluoro-2-(3-(2,6-dioxopiperidine))-isoindole-1,3-dione, 30 mg of DIPEA, and 1 mL of DMF were sequentially added into a reaction flask. The reaction flask was treated in a microwave reactor for 10 min of reaction at 50 Hz. 2.5 mL of water was added, and the mixture was extracted with water and dichloromethane together (2.5 mL × 3). The organic phases were combined, dried over anhydrous sodium sulfate, and concentrated to give a crude product. The crude product was purified by column chromatography (eluent: dichloromethane:methanol = 8:1) to give compound C75 (4.8 mg). The mass spectral data for compound C75 are, MS (ESI): (M+H)⁺, 763.4

The nuclear magnetic resonance data for compound C75 are as follows:
H NMR (400 MHz, CDCl₃) δ: 8.71-8.73 (m, 1H), 7.46-7.51 (m, 1H), 7.25-7.29 (m, 1H), 7.12-7.15 (m, 1H), 7.08-7.11 (m, 2H), 6.77-6.83 (m, 2H), 6.48-6.52 (m, 1H), 6.30-6.38 (m, 2H), 5.95 (d, 1H, J=12Hz), 5.68 (s, 1H), 4.95-5.01 (m, 1H), 4.12-4.15 (m, 2H), 4.04-4.08 (m, 2H), 3.89-4.00 (m, 2H), 3.40-3.57 (m, 1H), 2.75-2.95 (m, 3H), 2.21-2.48 (m, 2H), 2.00-2.12 (m, 4H), 1.71-1.90 (m, 4H), 1.25-1.58 (m, 7H), 1.10-1.18 (m, 2H), 1.06 (s, 3H), 0.88 (s, 3H)

### Example 76: Synthesis of N-(3-(N-(N'-(4-(2-(3-(2,6-dioxopiperidine))-isoindole-1,3-dione))-piperidine-3S,5S-diamine))phenyl)-3-oxo-4-aza-5α-androst-1-ene-17β-carboxamide (C76)

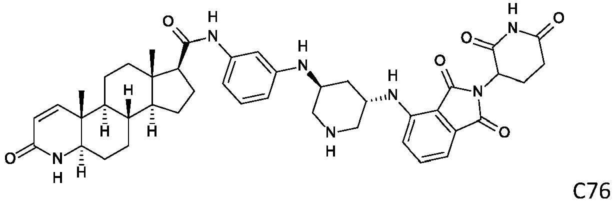

### Step 1: Synthesis of 3-(N-(1,N'-bis-Boc-piperidine-3S,5S-diamine))aniline (II-76)

### a. Synthesis of 3-(N-(piperidine-3S,5S-diamine))nitrobenzene (II-76-1)

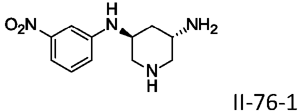

100 mg of *m*-fluoronitrobenzene and 815 mg of piperidine-3*S*,5*S*-diamine were transferred into a three-necked flask. The mixture was heated to 100 °C in a nitrogen atmosphere and stirred for reaction for 12 h. The reaction solution was cooled to room temperature and subjected to column chromatography (eluent: dichloromethane:methanol = 5:1) to give a pale yellow solid (compound II-76-1, 9 mg).
MS (ESI): (M+H)⁺, 237.1

### b. Synthesis of 3-(N-(1,N'-bis-Boc-piperidine-3S,5S-diamine))nitrobenzene (II-76-2)

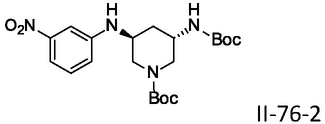

9 mg of compound II-76-1, 4.2 mg of triethylamine, 18.2 mg of Boc anhydride, and 2 mL of dichloromethane were sequentially added to a reaction flask, and the reaction system was stirred at room temperature for 30 min. The reaction solution was washed once with 0.5 mL of 1 mol/L aqueous hydrochloric acid and 0.5 mL of water, and the aqueous phase was extracted with dichloromethane (2.5 mL × 2). The organic phases were combined, dried over anhydrous sodium sulfate, and concentrated to give crude compound II-76-2 (16.6 mg).
MS (ESI): (M+H)⁺, 437.2

### c. Synthesis of 3-(N-(1,N'-bis-Boc-piperidine-3S,5S-diamine))aniline (II-76)

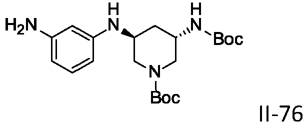

16.6 mg of crude compound II-76-2, 2 mL of methanol, and 1.6 mg of 5% palladium on carbon were sequentially added into a 100-mL hydrogenation reactor, and the system was incubated with continuous hydrogen introduction at 25 °C and 50 psi until the reaction was completed. The reaction solution was concentrated to give crude compound II-76 (15.4 mg). MS (ESI): (M+H)⁺, 407.3

### Step 2: Synthesis of N-(3-(N-(1,N'-bis-Boc-piperidine-3S,5S-diamine))phenyl)-3-oxo-4-aza-5α-androst-1-ene-17β-carboxamide (IV-76)

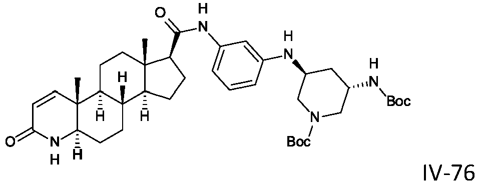

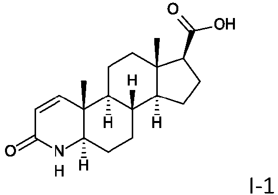

In a nitrogen atmosphere, 11 mg of compound I-1, 12.8 mg of crude compound II-76, 32.8 mg of pyBoP, 12.2 mg of DIPEA, and 0.5 mL of DMF were sequentially added into a reaction flask, and the mixture was stirred overnight until the reaction was completed. 2.5 mL of water was added into the reaction flask. The reaction solution was extracted with ethyl acetate (2.5 mL × 3). The organic phases were combined, sequentially washed once with 1 mL of 1 mol/L aqueous hydrochloric acid and 1 mL of saturated aqueous sodium carbonate, dried over anhydrous sodium sulfate, and concentrated to give a crude product. The crude product was purified by column chromatography (eluent: dichloromethane:methanol = 15:1) to give compound IV-76 (21 mg).
MS (ESI): (M+H)⁺, 706.4

### Step 3: Synthesis of N-(3-(N-(piperidine-3S,5S-diamine))phenyl)-3-oxo-4-aza-5α-androst-1-ene-17β-carboxamide (V-76)

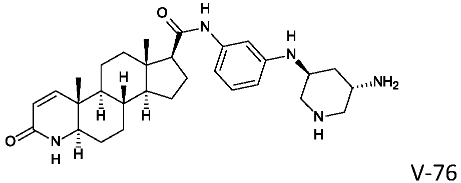

At room temperature, 21 mg of compound IV-76 and 1 mL of dichloromethane were added into a reaction flask, before 0.1 mL of trifluoroacetic acid was dropwise added. The reaction system was incubated for 10 min. The reaction solution was concentrated to give crude compound V-76 (14.8 mg).
MS (ESI): (M+H)⁺, 506.3

### Step 4: Synthesis of N-(3-(N-(N'-(4-(2-(3-(2,6-dioxopiperidine))-isoindole-1,3-dione))-piperidine-3S,5S-diamine))phenyl)-3-oxo-4-aza-5α-androst-1-ene-17β-carboxamide (C76)

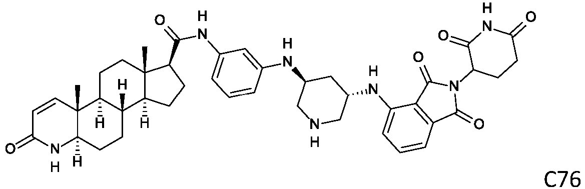

21 mg of crude compound V-76, 13 mg of 4-fluoro-2-(3-(2,6-dioxopiperidine))-isoindole-1,3-dione, 30 mg of DIPEA, and 1 mL of DMF were sequentially added into a reaction flask. The reaction flask was treated in a microwave reactor for 10 min of reaction at 50 Hz. 2.5 mL of water was added, and the mixture was extracted with water and dichloromethane together (2.5 mL × 3). The organic phases were combined, dried over anhydrous sodium sulfate, and concentrated to give a crude product. The crude product was purified by column chromatography (eluent: dichloromethane:methanol = 8:1) to give compound C76 (3.3 mg). The mass spectral data for compound C76 are, MS (ESI): (M+H)⁺, 762.4

The nuclear magnetic resonance data for compound C76 are as follows:
H NMR (400 MHz, CDCl₃) δ: 8.57-8.59 (m, 1H), 7.45-7.49 (m, 1H), 7.20-7.25 (m, 1H), 7.12-7.18 (m, 3H), 6.73-6.81 (m, 2H), 6.57-6.60 (m, 1H), 6.35-6.40 (m, 2H), 5.89 (d, 1H, J=12Hz), 5.71 (s, 1H), 4.91-4.96 (m, 1H), 3.90-4.02 (m, 2H), 3.82-3.86 (m, 2H), 3.71-3.74 (m, 2H), 3.45-3.62 (m, 1H), 2.67-2.90 (m, 3H), 2.21-2.33 (m, 2H), 2.05-2.18 (m, 4H), 1.71-1.90 (m, 4H), 1.15-1.58 (m, 7H), 1.01-1.11 (m, 2H), 0.99 (s, 3H), 0.85 (s, 3H)

### Example 77: Synthesis of N-(3-(N-(N'-(4-(2-(3-(2,6-dioxopiperidine))-isoindole-1,3-dione))-1-methylpiperidine-3S,5S-diamine))phenyl)-3-oxo-4-aza-5α-androst-1-ene-17β-carboxamide (C77)

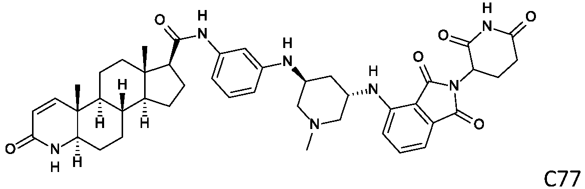

### Step 1: Synthesis of 3-(N-(N'-Boc-1-methylpiperidine-3S,5S-diamine))aniline (II-77)

### a. Synthesis of 3-(N-(1-methylpiperidine-3S,5S-diamine))nitrobenzene (II-77-1)

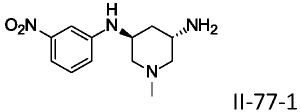

100 mg of *m*-fluoronitrobenzene and 915 mg of *N*-methylpiperidine-35,55-diamine were transferred into a three-necked flask. The mixture was heated to 100 °C in a nitrogen atmosphere and stirred for reaction for 12 h. The reaction solution was cooled to room temperature and subjected to column chromatography (eluent: dichloromethane:methanol = 6:1) to give a pale yellow solid (compound II-77-1, 13 mg).
MS (ESI): (M+H)⁺, 251.1

### b. Synthesis of 3-(N-(N'-Boc-1-methylpiperidine-3S,5S-diamine))nitrobenzene (II-77-2)

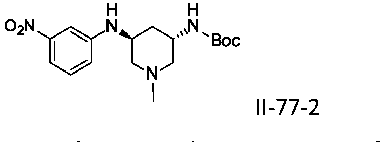

13 mg of compound II-77-1, 5.8 mg of triethylamine, 11.3 mg of Boc anhydride, and 2 mL of dichloromethane were sequentially added to a reaction flask, and the reaction system was stirred at room temperature for 30 min. The reaction solution was washed once with 0.5 mL of 1 mol/L aqueous hydrochloric acid and 0.5 mL of water respectively, and the aqueous phase was extracted with dichloromethane (2.5 mL × 2). The organic phases were combined, dried over anhydrous sodium sulfate, and concentrated to give crude compound II-77-2 (18 mg).
MS (ESI): (M+H)⁺, 351.2

### c. Synthesis of 3-(N-(N'-Boc-1-methylpiperidine-3S,5S-diamine))aniline (II-77)

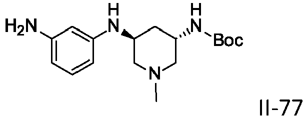

18 mg of crude compound II-77-2, 2 mL of methanol, and 1.8 mg of 5% palladium on carbon were sequentially added into a 100-mL hydrogenation reactor, and the system was incubated with continuous hydrogen introduction at 25 °C and 50 psi until the reaction was completed. The reaction solution was concentrated to give crude compound II-77 (16.4 mg).
MS (ESI): (M+H)⁺, 321.2

### Step 2: Synthesis of N-(3-(N-(N'-Boc-1-methylpiperidine-3S,5S-diamine))phenyl)-3-oxo-4-aza-5α-androst-1-ene-17β-carboxamide (IV-77)

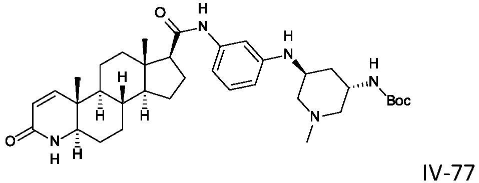

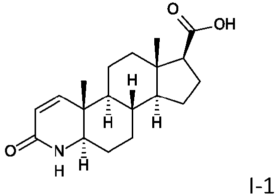

In a nitrogen atmosphere, 11 mg of compound I-1, 10 mg of crude compound II-77, 32.8 mg of pyBoP, 12.2 mg of DIPEA, and 0.5 mL of DMF were sequentially added into a reaction flask, and the mixture was stirred overnight until the reaction was completed. 2.5 mL of water was added into the reaction flask. The reaction solution was extracted with ethyl acetate (2.5 mL × 3). The organic phases were combined, sequentially washed once with 1 mL of 1 mol/L aqueous hydrochloric acid and 1 mL of saturated aqueous sodium carbonate, dried over anhydrous sodium sulfate, and concentrated to give a crude product. The crude product was purified by column chromatography (eluent: dichloromethane:methanol = 15:1) to give compound IV-77 (18.7 mg).
MS (ESI): (M+H)⁺, 620.4

### Step 3: Synthesis of N-(3-(N-(1-methylpiperidine-3S,5S-diamine))phenyl)-3-oxo-4-aza-5α-androst-1-ene-17β-carboxamide (V-77)

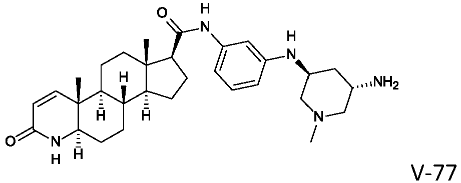

At room temperature, 14.8 mg of compound IV-77 and 1 mL of dichloromethane were added into a reaction flask, before 0.1 mL of trifluoroacetic acid was dropwise added. The reaction system was incubated for 10 min. The reaction solution was concentrated to give crude compound V-77 (12.4 mg).
MS (ESI): (M+H)⁺, 520.4

### Step 4: Synthesis of N-(3-(N-(N'-(4-(2-(3-(2,6-dioxopiperidine))-isoindole-1,3-dione))-1-methylpiperidine-3S,5S-diamine))phenyl)-3-oxo-4-aza-5α-androst-1-ene-17β-carboxamide (C77)

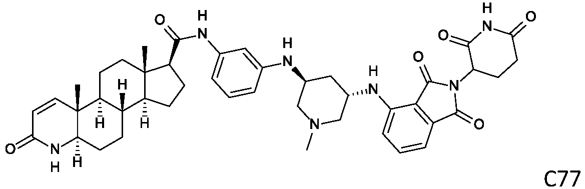

16.3 mg of crude compound V-77, 13 mg of 4-fluoro-2-(3-(2,6-dioxopiperidine))-isoindole-1,3-dione, 30 mg of DIPEA, and 1 mL of DMF were sequentially added into a reaction flask. The reaction flask was treated in a microwave reactor for 10 min of reaction at 50 Hz. 2.5 mL of water was added, and the mixture was extracted with water and dichloromethane together (2.5 mL × 3). The organic phases were combined, dried over anhydrous sodium sulfate, and concentrated to give a crude product. The crude product was purified by column chromatography (eluent: dichloromethane:methanol = 10:1) to give compound C77 (5.1 mg). The mass spectral data for compound C77 are, MS (ESI): (M+H)⁺, 776.4

The nuclear magnetic resonance data for compound C77 are as follows:
H NMR (400 MHz, CDCl₃) δ: 8.59-8.61 (m, 1H), 7.47-7.50 (m, 1H), 7.15-7.21 (m, 1H), 7.08-7.12 (m, 3H), 6.73-6.81 (m, 2H), 6.51-6.57 (m, 1H), 6.35-6.39 (m, 2H), 5.96 (d, 1H, J=12Hz), 5.88 (s, 1H), 5.02-5.10 (m, 1H), 3.92-3.98 (m, 2H), 3.73-3.84 (m, 4H), 3.58 (s, 3H), 3.40-3.57 (m, 1H), 2.67-2.90 (m, 3H), 2.28-2.43 (m, 2H), 2.05-2.15 (m, 4H), 1.67-1.95 (m, 4H), 1.15-1.60 (m, 7H), 1.05-1.18 (m, 2H), 0.96 (s, 3H), 0.75 (s, 3H)

### Example 78: Synthesis of N-(3-(N-(N'-(7-fluoro-4-(2-(3-(2,6-dioxopiperidine))-isoindole-1,3-dione))-1R,3R-cyclohexanediamine))phenyl)-3-oxo-4-aza-5α-androst-1-ene-17β-carboxamide (C78)

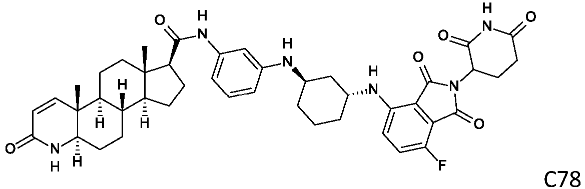

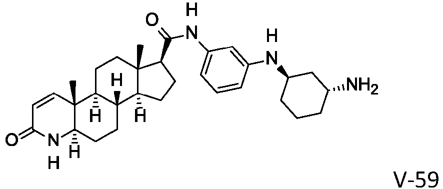

At room temperature, 1.21 g of compound V-59, 1.04 g of 4,7-difluoro-2-(3-(2,6-dioxopiperidine))-isoindole-1,3-dione, 1.53 g of DIPEA, and 10 mL of DMF were added into a reaction flask. The reaction flask was treated in a microwave reactor for 10 min of reaction at 50 Hz. 20 mL of water was added into the reaction flask, and the mixture was extracted with dichloromethane (20 mL × 3). The organic phases were combined, washed once with 20 mL of water, dried over anhydrous sodium sulfate, and concentrated to give a crude product. The crude product was purified by column chromatography (eluent: dichloromethane:methanol = 10:1) to give compound C78 (0.22 g).
The mass spectral data for compound C78 are, MS (ESI): (M+H)⁺, 779.4

The nuclear magnetic resonance data for compound C78 are as follows:
H NMR (400 MHz, CDCl₃) δ: 8.62-8.64 (m, 1H), 7.15-7.18 (m, 1H), 7.20-7.24 (m, 3H), 6.83-6.87 (m, 2H), 6.40-6.47 (m, 1H), 6.36-6.39 (m, 2H), 5.80 (d, 1H, J=12Hz), 5.70 (s, 1H), 4.85-4.91 (m, 1H), 4.23-4.38 (m, 2H), 3.75-3.85 (m, 2H), 3.28-3.33 (m, 1H), 2.65-2.81 (m, 3H), 2.15-2.30 (m, 2H), 2.01-2.11 (m, 2H), 1.90-1.73 (m, 10H), 1.55-1.15 (m, 9H), 1.08-1.11 (m, 2H), 0.92 (s, 3H), 0.75 (s, 3H)

### Example 79: Synthesis of N-(3-(N-(N'-(6-fluoro-4-(2-(3-(2,6-dioxopiperidine))-isoindole-1,3-dione))-1R,3R-cyclohexanediamine))phenyl)-3-oxo-4-aza-5α-androst-1-ene-17β-carboxamide (C79)

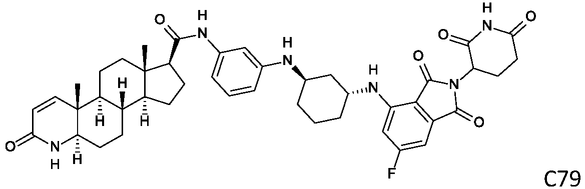

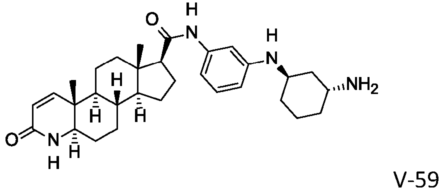

At room temperature, 1.21 g of compound V-59, 1.04 g of 4,6-difluoro-2-(3-(2,6-dioxopiperidine))-isoindole-1,3-dione, 1.53 g of DIPEA, and 10 mL of DMF were added into a reaction flask. The reaction flask was treated in a microwave reactor for 10 min of reaction at 50 Hz. 20 mL of water was added into the reaction flask, and the mixture was extracted with dichloromethane (20 mL × 3). The organic phases were combined, washed once with 20 mL of water, dried over anhydrous sodium sulfate, and concentrated to give a crude product. The crude product was purified by column chromatography (eluent: dichloromethane:methanol = 10:1) to give compound C79 (0.13 g).
The mass spectral data for compound C79 are, MS (ESI): (M+H)⁺, 779.4

The nuclear magnetic resonance data for compound C79 are as follows:
H NMR (400 MHz, CDCl₃) δ: 8.75-8.77 (m, 1H), 7.40-7.47 (m, 1H), 7.17-7.20 (m, 1H), 7.13-7.18 (m, 2H), 6.72-6.81 (m, 2H), 6.55-6.59 (m, 1H), 6.31-6.35 (m, 2H), 5.80 (d, 1H, J=12Hz), 5.71 (s, 1H), 4.88-4.91 (m, 1H), 3.40-3.57 (m, 1H), 2.67-2.90 (m, 3H), 2.21-2.33 (m, 2H), 2.08-2.18 (m, 2H), 1.90-1.71 (m, 8H), 1.58-1.15 (m, 11H), 1.01-1.11 (m, 2H), 0.96 (s, 3H), 0.75 (s, 3H)

### Example 80: Synthesis of N-(3-(N-(N-methyl-N'-(4-(2-(3-(2,6-dioxopiperidine))-isoindole-1,3-dione))-1R,3R-cyclohexanediamine))phenyl)-3-oxo-4-aza-5α-androst-1-ene-17β-carboxamide (C80)

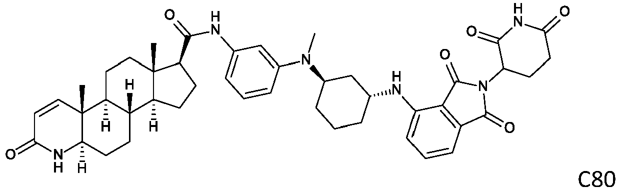

### Step 1:

### a. Synthesis of 3-(N-(N-methyl-N'-Boc-1R,3R-cyclohexanediamine))nitrobenzene (II-80-1)

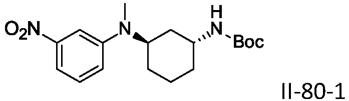

0.2 g of 3-(*N*-(*N*'-Boc-1*R*,3*R*-cyclohexanediamine))nitrobenzene, 127 mg of iodomethane, and 166 mg of potassium carbonate were sequentially added to 1.5 mL of *N*,*N-*dimethylformamide, and the reaction system was stirred overnight at room temperature. 10 mL of water was added, and the mixture was extracted with ethyl acetate (10 mL × 3). The organic phases were combined, sequentially washed once with 3 mL of saturated brine and 3 mL of water, dried, and concentrated by rotary evaporation to remove the solvent to give crude compound II-80-1 (0.18 g), which was directly used in the next reaction.

### b. Synthesis of 3-(N-(N-methyl-N'-Boc-1R,3R-cyclohexanediamine))aniline (II-80)

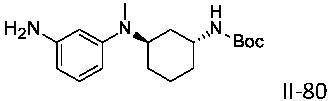

0.18 g of crude compound II-80-1 obtained in the previous step was added into a 100-mL hydrogenation reactor before 10 mL of methanol and 18 mg of 5% palladium on carbon were added. The system was incubated with continuous hydrogen introduction at 25 °C and 50 psi until the reaction was completed. The reaction solution was filtered and concentrated to give crude compound II-80 (0.16 g), which was directly used in the next reaction.

### Step 2: Synthesis of N-(3-(N-(N-methyl-N'-Boc-1R,3R-cyclohexanediamine))phenyl)-3-oxo-4-aza-5α-androst-1-ene-17β-carboxamide (IV-80)

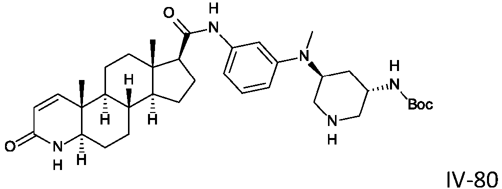

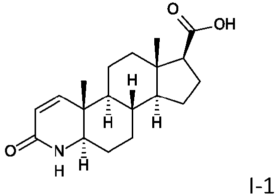

In a nitrogen atmosphere, 36.4 mg of compound I-1, 32 mg of crude compound II-80, 101 mg of pyBoP, 38.7 mg of *N*,*N*-diisopropylethylamine, and 1.5 mL of DMF were sequentially added into a reaction flask. The mixture was stirred overnight before 5 mL of water was added. The mixture was extracted with ethyl acetate (10 mL × 3). The organic phases were combined, sequentially washed once with 3 mL of 1 mol/L diluted aqueous hydrochloric acid and 3 mL of saturated aqueous sodium carbonate, dried over anhydrous sodium sulfate, concentrated, and purified by column chromatography (eluent: dichloromethane:methanol = 20:1) to give a product (compound IV-80, 60 mg).
MS (ESI): (M+H)⁺, 620.4

### Step 3: Synthesis of N-(3-(N-(N-methyl-1R,3R-cyclohexanediamine))phenyl)-3-oxo-4-aza-5α-androst-1-ene-17β-carboxamide (V-80)

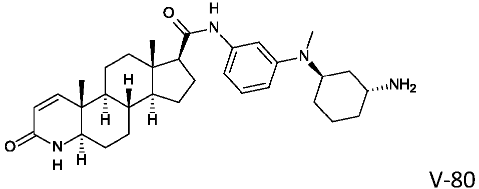

At room temperature, 60 mg of compound IV-80 and 2 mL of dichloromethane were added into a glass flask, before 0.2 mL of trifluoroacetic acid was slowly and dropwise added. The reaction system was incubated for 10 min. The reaction solution was concentrated to give the target product (crude compound V-80), which was directly used in the next reaction without separation.
MS (ESI): (M+H)⁺, 519.4

### Step 4: Synthesis of N-(3-(N-(N-methyl-N'-(4-(2-(3-(2,6-dioxopiperidine))-isoindole-1,3-dione))-1R,3R-cyclohexanediamine))phenyl)-3-oxo-4-aza-5α-androst-1-ene-17β-carboxamide (C80)

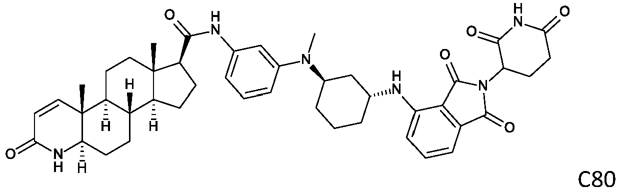

Crude compound V-80 obtained in step 3, 26 mg of 4-fluoro-2-(2,6-dioxo-3-piperidinyl)-isoindole-1,3-dione, 60 mg of DIPEA, and 1 mL of DMF were treated in a microwave reactor at 50 Hz for reaction for 10 min. The reaction solution was extracted with water and dichloromethane thrice. The organic phase was dried over anhydrous sodium sulfate, concentrated, and eluted by column chromatography (eluent: dichloromethane:methanol = 15:1) to give compound C80 (19 mg).
The mass spectral data for compound C80 are, MS (ESI): (M+H)⁺, 775.4

The nuclear magnetic resonance data for compound C80 are as follows:
H NMR (400 MHz, CDCl₃) δ: 8.55-8.57 (m, 1H), 7.44-7.48 (m, 1H), 7.16-7.22 (m, 1H), 7.05-7.12 (m, 3H), 6.73-6.81 (m, 2H), 6.51-6.57 (m, 1H), 6.25-6.35 (m, 2H), 5.80 (d, 1H, J=12Hz), 5.77 (s, 1H), 4.85-4.92 (m, 1H), 3.70-3.83 (m, 2H), 3.49 (s, 3H), 3.28-3.33 (m, 1H), 2.66-2.90 (m, 3H), 2.21-2.35 (m, 2H), 2.08-2.20 (m, 2H), 1.91-1.62 (m, 12H), 1.49-1.31 (m, 7H), 1.06-1.12 (m, 2H), 0.99 (s, 3H), 0.73 (s, 3H)

### Example 81: Synthesis of N-(3-(N-(N'-(4-(2-(3-(2,6-dioxopiperidine))-isoindole-1,3-dione))-1R,3R-cyclohexanediamine))phenyl)-3-oxo-4-aza-5α-androstane-17β-carboxamide (C81)

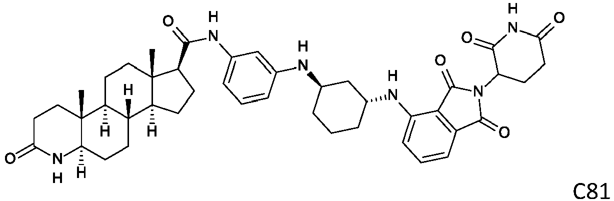

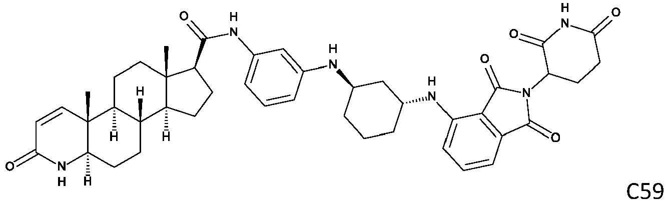

35 mg of compound C59 was added to a 100-mL hydrogenation reactor before 3 mL of methanol and 5 mg of 5% palladium on carbon were added. The system was incubated for 2 h at 50 °C with continuous hydrogen introduction at 50 psi. When the monitoring indicated the completion of the reaction, the reaction solution was filtered, concentrated, and purified by column chromatography (eluent: dichloromethane:methanol = 10:1) to give a product (compound C81, 33 mg).
The mass spectral data for compound C81 are, MS (ESI): (M+H)⁺, 763.4

The nuclear magnetic resonance data for compound C81 are as follows:
H NMR (400 MHz, CDCl₃) δ: 8.90-8.92 (m, 1H), 7.38-7.41 (m, 1H), 7.05-7.09 (m, 1H), 7.08-7.12 (m, 3H), 6.73-6.81 (m, 1H), 6.53-6.59 (m, 1H), 6.16-6.35 (m, 1H), 6.06-6.10 (m, 1H), 5.17 (s, 1H), 4.88-4.91 (m, 1H), 3.73-3.80 (m, 2H), 3.28-3.33 (m, 1H), 2.67-2.90 (m, 3H), 2.41 (m, 2H), 2.21-2.33 (m, 2H), 1.95-2.10 (m, 3H), 1.91-1.69 (m, 10H), 1.52-1.17 (m, 10H), 0.98-1.11 (m, 2H), 0.91 (s, 3H), 0.70 (s, 3H)

### Example 82: Detection of anti-proliferative toxicity against prostate cancer cell lines Vcap, 22Rv1, and PC-3

### (1) Cell culture:

Vcap cells (purchased from the Cell Bank of the Chinese Academy of Sciences, Cat. No. SCSP-5034) were subcultured in a culture medium (DMEM medium + 1% penicillin-streptomycin dual-antibiotic solution + 10% fetal bovine serum) in an incubator at 5% carbon dioxide and 37 °C.

22Rv1 cells (purchased from the Cell Bank of the Chinese Academy of Sciences, Cat. No. TCHu100) were subcultured in a culture medium (RPMI-1640 medium + 1% penicillin-streptomycin dual-antibiotic solution + 10% fetal bovine serum) in an incubator at 5% carbon dioxide and 37 °C.

PC-3 cells (purchased from the Cell Bank of the Chinese Academy of Sciences, Cat. No. SCSP-532) were subcultured in a culture medium (F12K medium + 1% penicillin-streptomycin dual-antibiotic solution + 10% fetal bovine serum) in an incubator at 5% carbon dioxide and 37 °C.

### (2) Cell seeding:

VCap cells were seeded at 20000 cells/well (90 µL) on a 96-well plate and cultured for 72 h. The original medium was removed, and a new medium of 90 µL was added.

22Rv1 cells were seeded at 5000 cells/well (90 µL) on a 96-well plate and cultured for 48 h. PC-3 cells were seeded at 4000 cells/well (90 µL) on a 96-well plate and cultured for 24 h.

### (3) Preparation of compound stock solution:

10 mM compound stock solutions were prepared in DMSO.

### (4) Dilution of stock solution

For VCap cells, the compound stock solution was diluted with DMSO to give DMSO solutions 1-10 at concentrations of 0 µM, 80 µM, 16 µM, 3.2 µM, 1.5 µM, 0.5 µM, 0.15 µM, 0.05 µM, 0.025 µM, and 0.0005 µM.

For 22Rv1 cells and PC-3 cells, the compound stock solution was diluted with DMSO to give DMSO solutions 11-19 at concentrations of 0 µM, 500 µM, 100 µM, 20 µM, 4 µM, 0.8 µM, 0.16 µM, 0.032 µM, 0.0064 µM.

### (5) Mixing of compound with medium:

For Vcap cells, 2 µL of each of the above solutions 1-10 was added to 98 µL of Vcap medium to give mixed solutions 20-29 of the compound and the medium.

For 22Rv1 cells, 2 µL of each of the above solutions 11-19 was added to 98 µL of 22Rv1 medium to give mixed solutions 30-38 of the compound and the medium.

For PC-3 cells, 2 µL of each of the above solutions 11-19 was added to 98 µL of PC-3 medium to give mixed solutions 39-47 of the compound and the medium.

### (6) Addition of compound:

For Vcap cells, 10 µL of each of the above solutions 20-29 was added to the above cell seed solution to give a total volume of 100 µL (90 µL + 10 µL), where the concentrations of the compound in wells were 0 µM, 0.16 µM, 0.032 µM, 0.006 µM, 0.003 µM, 0.001 µM, 0.0003 µM, 0.0001 µM, 0.00005 µM, and 0.00001 µM. All concentrations were in triplicate.

For 22Rv1 and PC-3 cells, 10 µL of each of the above solutions 30-38 or 39-47 was added to the above cell seed solution to give a total volume of 100 µL (90 µL + 10 µL), where the concentrations of the compound in wells were 0 µM, 1.0 µM, 0.2 µM, 0.04 µM, 0.008 µM, 0.0016 µM, 0.00032 µM, 0.00006 µM, and 0.00001 µM. All concentrations were in triplicate.

### (7) Detection of anti-cell proliferation toxicity:

After the above-treated cells were cultured for 3 days, 10 µL of the CCK8 solution (DOJINDO, Cat. No. PF725) was added to each well. The plate was incubated in an incubator at 37 °C for 1-4 h and then detected on a microplate reader for the reading at 450 nm.

Tables 1-3 show the anti-proliferative toxicity data for compounds in Vcap, 22Rv1, and PC-3 cells, where IC₅₀ is the half maximal inhibitory concentration, and
A denotes an IC₅₀ less than or equal to 0.1 nM;
B denotes an IC₅₀ less than or equal to 10 nM and greater than 0.1 nM;
C denotes an IC₅₀ less than or equal to 1000 nM and greater than 10 nM;
D denotes an IC₅₀ greater than 1000 nM.

**Table 1. Anti-proliferative toxicity data in Vcap, 22Rv1, and PC-3 cells**

| Compound | VCaP | 22Rv1 | PC-3 |
|---|---|---|---|
| C1 | D | D | D |
| C2 | D | D | D |
| C3 | D | D | D |
| C4 | D | D | D |
| C5 | D | D | D |
| C6 | D | D | D |
| C7 | D | D | D |
| C8 | D | D | D |
| C9 | D | D | D |
| C10 | D | D | D |
| C11 | D | D | D |
| C12 | D | D | D |
| C13 | D | D | D |
| C14 | D | D | D |
| C15 | C | D | D |
| C16 | D | D | D |
| C17 | D | D | D |
| C18 | D | D | D |
| C19 | D | D | D |
| C20 | D | D | D |
| C21 | D | D | D |
| C22 | D | D | D |
| C23 | D | D | D |
| C24 | D | D | D |
| C25 | D | D | D |
| C26 | B | C | D |
| C27 | C | D | D |
| C28 | D | D | D |
| C29 | B | B | D |
| C30 | D | D | D |
| C31 | D | D | D |
| C32 | D | D | D |
| C33 | D | D | D |
| C34 | D | D | D |
| C35 | D | D | D |
| C36 | D | D | D |
| C37 | D | D | D |
| C38 | C | D | D |
| C39 | D | D | D |
| C40 | C | D | D |
| C41 | B | B | D |
| C42 | D | D | D |
| C43 | D | D | D |
| C44 | D | D | D |
| C45 | D | D | D |
| C46 | B | B | D |
| C47 | C | C | D |
| C48 | D | D | D |
| C49 | B | B | D |
| C50 | B | B | C |
| C51 | D | D | D |
| C52 | D | D | D |
| C53 | D | D | D |
| C54 | D | D | D |
| C55 | C | C | D |
| C56 | D | D | D |
| C57 | C | C | D |
| C58 | B | B | C |
| C59 | A | B | B |
| C60 | C | C | D |
| C61 | D | D | D |
| C62 | C | D | D |
| C63 | A | B | B |
| C64 | B | B | B |
| C65 | C | C | D |
| C66 | D | D | D |
| C67 | A | B | B |
| C68 | A | B | B |
| C69 | B | B | C |
| C70 | B | B | C |
| C71 | C | C | D |
| C72 | C | C | D |
| C73 | C | C | D |
| C74 | B | B | C |
| C75 | C | C | C |
| C76 | C | C | C |
| C77 | C | C | C |
| C78 | B | B | B |
| C79 | B | B | B |
| C80 | B | B | C |
| C81 | B | B | C |

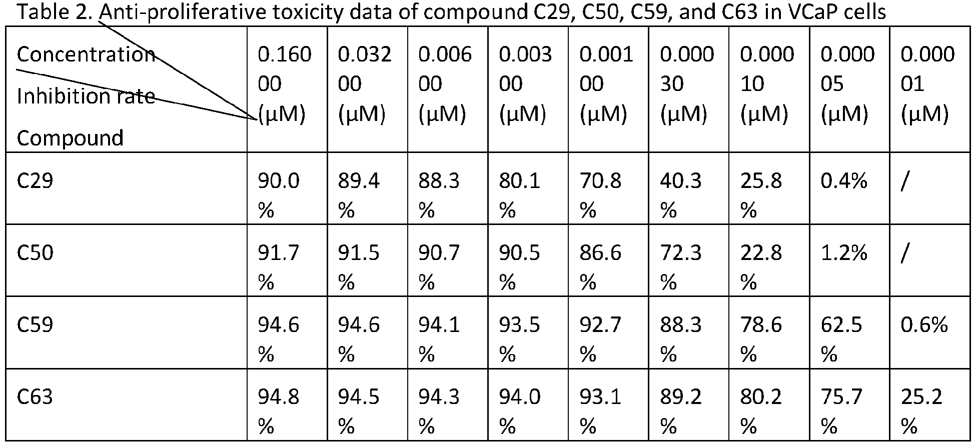

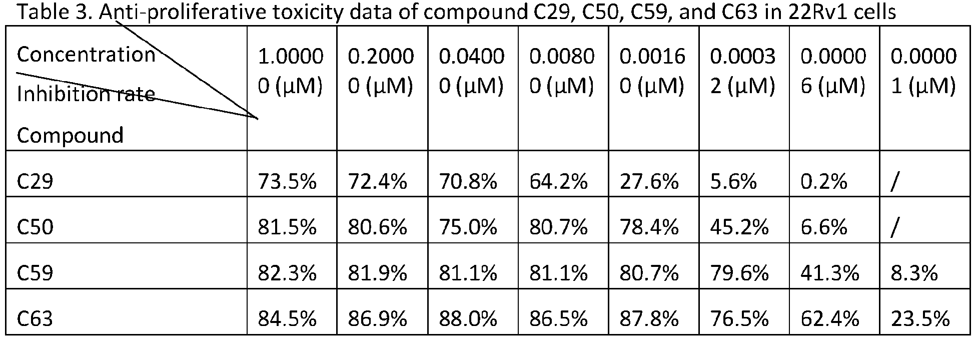

### Example 83: Detection of anti-proliferative toxicity of C63 in some tumor cell lines

### (1) Cell culture:

PC-3 cells (purchased from the Cell Bank of the Chinese Academy of Sciences, Cat. No. SCSP-532) were subcultured in a culture medium (F12K medium + 1% penicillin-streptomycin dual-antibiotic solution + 10% fetal bovine serum) in an incubator at 5% carbon dioxide and 37 °C.

DU 145 cells (purchased from the Cell Bank of the Chinese Academy of Sciences, Cat. No. SCSP-5024) were subcultured in a culture medium (MEM medium + 1% penicillin-streptomycin dual-antibiotic solution + 10% fetal bovine serum) in an incubator at 5% carbon dioxide and 37 °C.

AGS cells (purchased from the Cell Bank of the Chinese Academy of Sciences, Cat. No. TCHu232) were subcultured in a culture medium (F12K medium + 1% penicillin-streptomycin dual-antibiotic solution + 10% fetal bovine serum) in an incubator at 5% carbon dioxide and 37 °C.

HuH-7 cells (purchased from the Cell Bank of the Chinese Academy of Sciences, Cat. No. SCSP-526) were subcultured in a culture medium (DMEM medium + 1% penicillin-streptomycin dual-antibiotic solution + 10% fetal bovine serum) in an incubator at 5% carbon dioxide and 37 °C.

Ca Ski cells (purchased from the Cell Bank of the Chinese Academy of Sciences, Cat. No. TCHu137) were subcultured in a culture medium (RPMI-1640 medium + 1% penicillin-streptomycin dual-antibiotic solution + 10% fetal bovine serum) in an incubator at 5% carbon dioxide and 37 °C.

C-33 A cells (purchased from the Cell Bank of the Chinese Academy of Sciences, Cat. No. TCHu176) were subcultured in a culture medium (MEM medium + 1% penicillin-streptomycin dual-antibiotic solution + 10% fetal bovine serum) in an incubator at 5% carbon dioxide and 37 °C.

OVCAR-3 cells (purchased from the Cell Bank of the Chinese Academy of Sciences, Cat. No. TCHu228) were subcultured in a culture medium (RPMI-1640 medium + 1% penicillin-streptomycin dual-antibiotic solution + +20% fetal bovine serum + 0.01 mg/mL recombinant human insulin) in an incubator at 5% carbon dioxide and 37 °C.

PANC-1 cells (purchased from the Cell Bank of the Chinese Academy of Sciences, Cat. No. SCSP-535) were subcultured in a culture medium (DMEM medium + 1% penicillin-streptomycin dual-antibiotic solution + 10% fetal bovine serum) in an incubator at 5% carbon dioxide and 37 °C.

MCF-7 cells (purchased from the Cell Bank of the Chinese Academy of Sciences, Cat. No. SCSP-531) were subcultured in a culture medium (MEM medium + 1% penicillin-streptomycin dual-antibiotic solution +10% fetal bovine serum + 0.01 mg/mL recombinant human insulin) in an incubator at 5% carbon dioxide and 37 °C.

MDA-MB-468 cells (purchased from the Cell Bank of the Chinese Academy of Sciences, Cat. No. TCHu136) were subcultured in a culture medium (L15 medium + 1% penicillin-streptomycin dual-antibiotic solution + 10% fetal bovine serum) in an incubator at 37 °C.

MDA-MB-231 cells (purchased from the Cell Bank of the Chinese Academy of Sciences, Cat. No. TCHu227) were subcultured in a culture medium (L15 medium + 1% penicillin-streptomycin dual-antibiotic solution + 10% fetal bovine serum) in an incubator at 37 °C.

NCI-N87 cells (purchased from the Cell Bank of the Chinese Academy of Sciences, Cat. No. TCHu130) were subcultured in a culture medium (RPMI-1640 medium + 1% penicillin-streptomycin dual-antibiotic solution + 10% fetal bovine serum) in an incubator at 5% carbon dioxide and 37 °C.

BT-549 cells (purchased from the Cell Bank of the Chinese Academy of Sciences, Cat. No. TCHu93) were subcultured in a culture medium (RPMI-1640 medium + 1% penicillin-streptomycin dual-antibiotic solution + +10% fetal bovine serum + 0.01 mg/mL recombinant human insulin) in an incubator at 5% carbon dioxide and 37 °C.

BxPC-3 cells (purchased from the Cell Bank of the Chinese Academy of Sciences, Cat. No. TCHu12) were subcultured in a culture medium (RPMI-1640 medium + 1% penicillin-streptomycin dual-antibiotic solution + 10% fetal bovine serum) in an incubator at 5% carbon dioxide and 37 °C.

HGC-27 cells (purchased from the Cell Bank of the Chinese Academy of Sciences, Cat. No. TCHu22) were subcultured in a culture medium (RPMI-1640 medium + 1% penicillin-streptomycin dual-antibiotic solution + +20% fetal bovine serum) in an incubator at 5% carbon dioxide and 37 °C.

DLD-1 cells (purchased from the Cell Bank of the Chinese Academy of Sciences, Cat. No. TCHu134) were subcultured in a culture medium (RPMI-1640 medium + 1% penicillin-streptomycin dual-antibiotic solution + 10% fetal bovine serum) in an incubator at 5% carbon dioxide and 37 °C.

SNU-1 cells (purchased from the Cell Bank of the Chinese Academy of Sciences, Cat. No. TCHu230) were subcultured in a culture medium (RPMI-1640 medium + 1% penicillin-streptomycin dual-antibiotic solution + 10% fetal bovine serum) in an incubator at 5% carbon dioxide and 37 °C.

U251 cells (purchased from the Cell Bank of the Chinese Academy of Sciences, Cat. No. TCHu58) were subcultured in a culture medium (DMEM medium + 1% penicillin-streptomycin dual-antibiotic solution + 10% fetal bovine serum) in an incubator at 5% carbon dioxide and 37 °C.

KATOIII cells (purchased from the Cell Bank of the Chinese Academy of Sciences, Cat. No. TCHu229) were subcultured in a culture medium (IMDM medium + 1% penicillin-streptomycin dual-antibiotic solution + 10% fetal bovine serum) in an incubator at 5% carbon dioxide and 37 °C.

HepG2 cells (purchased from the Cell Bank of the Chinese Academy of Sciences, Cat. No. TCHu72) were subcultured in a culture medium (MEM medium + 1% penicillin-streptomycin dual-antibiotic solution + 10% fetal bovine serum) in an incubator at 5% carbon dioxide and 37 °C.

Hep3B2.1-7 cells (purchased from the Cell Bank of the Chinese Academy of Sciences, Cat. No. TCHu106) were subcultured in a culture medium (MEM medium + 1% penicillin-streptomycin dual-antibiotic solution + 10% fetal bovine serum) in an incubator at 5% carbon dioxide and 37 °C.

HL-60 cells (purchased from the Cell Bank of the Chinese Academy of Sciences, Cat. No. TCHu23) were subcultured in a culture medium (IMDM medium + 1% penicillin-streptomycin dual-antibiotic solution +20% fetal bovine serum) in an incubator at 5% carbon dioxide and 37 °C.

### (2) Cell seeding:

PC-3 cells were seeded at 4000 cells/well (90 µL) on a 96-well plate and cultured for 24 h.

DU 145 cells were seeded at 5000 cells/well (90 µL) on a 96-well plate and cultured for 24 h.

AGS cells were seeded at 2000 cells/well (90 µL) on a 96-well plate and cultured for 24 h.

HuH-7 cells were seeded at 5000 cells/well (90 µL) on a 96-well plate and cultured for 24 h.

Ca Ski cells were seeded at 5000 cells/well (90 µL) on a 96-well plate and cultured for 24 h.

C-33 A cells were seeded at 5000 cells/well (90 µL) on a 96-well plate and cultured for 24 h.

OVCAR-3 cells were seeded at 2000 cells/well (90 µL) on a 96-well plate and cultured for 24 h.

PANC-1 cells were seeded at 5000 cells/well (90 µL) on a 96-well plate and cultured for 24 h.

MCF-7 cells were seeded at 5000 cells/well (90 µL) on a 96-well plate and cultured for 24 h.

MDA-MB-468 cells were seeded at 5000 cells/well (90 µL) on a 96-well plate and cultured for 24 h.

MDA-MB-231 cells were seeded at 5000 cells/well (90 µL) on a 96-well plate and cultured for 24 h.

NCI-N87 cells were seeded at 8000 cells/well (90 µL) on a 96-well plate and cultured for 24 h.

BT-549 cells were seeded at 5000 cells/well (90 µL) on a 96-well plate and cultured for 24 h.

BxPC-3 cells were seeded at 3000 cells/well (90 µL) on a 96-well plate and cultured for 24 h.

HGC-27 cells were seeded at 3000 cells/well (90 µL) on a 96-well plate and cultured for 24 h.

DLD-1 cells were seeded at 2000 cells/well (90 µL) on a 96-well plate and cultured for 24 h.

SNU-1 cells were seeded at 5000 cells/well (90 µL) on a 96-well plate and cultured for 24 h.

U251 cells were seeded at 2000 cells/well (90 µL) on a 96-well plate and cultured for 24 h.

KATO III cells were seeded at 8000 cells/well (90 µL) on a 96-well plate and cultured for 24 h.

Hep G2 cells were seeded at 2000 cells/well (90 µL) on a 96-well plate and cultured for 24 h.

Hep 3B2.1-7 cells were seeded at 2000 cells/well (90 µL) on a 96-well plate and cultured for 24 h.

HL-60 cells were seeded at 20000 cells/well (90 µL) on a 96-well plate and cultured for 24 h.

### (3) Preparation of compound stock solution:

10 mM compound stock solutions were prepared in DMSO.

### (4)Dilution of stock solution:

The compound stock solution was diluted with DMSO to give DMSO solutions 1-9 at concentrations of 0 µM, 2500 µM, 500 µM, 100 µM, 20 µM, 4 µM, 0.8 µM, 0.16 µM, and 0.032 µM.

### (5) Mixing of compound with medium:

2 µL of each of solutions 1-9 was added to 98 µL of the medium to give mixed solutions 11-19 of the compound and the medium.

### (6) Addition of compound:

10 µL of each of solutions 11-19 was added to the above cell seed solution to give a total volume of 100 µL (90 µL + 10 µL).

The concentrations of the compound in the wells were 0 µM, 5.0 µM, 1.0 µM, 0.2 µM, 0.04 µM, 0.008 µM, 0.0016 µM, 0.00032 µM, and 0.000064 µM. All concentrations were in triplicate.

### (7) Detection of anti-cell proliferation toxicity:

After the above-treated cells were cultured for 3 days, 10 µL of the CCK8 solution (DOJINDO, Cat. No. PF725) was added to each well. The plate was incubated in an incubator at 37 °C for 1-4 h and then detected on a microplate reader for the reading at 450 nm.

| Concentrati on (µM) | 5.0000 00 | 1.0000 00 | 0.2000 00 | 0.0400 00 | 0.0080 00 | 0.0016 00 | 0.0003 20 | 0.0000 64 |
|---|---|---|---|---|---|---|---|---|
| Cell line | Inhibition rate | | | | | | | |
| PC-3 | 82.08% | 78.39% | 78.43% | 80.22% | 82.80% | 78.77% | 65.08% | 45.14% |
| DU 145 | 82.36% | 81.91% | 74.34% | 60.85% | 48.44% | 22.66% | 20.53% | 22.08% |
| AGS | 80.76% | 79.78% | 74.04% | 59.69% | 44.19% | 20.42% | 11.13% | 17.49% |
| HuH-7 | 73.06% | 75.09% | 78.29% | 78.26% | 79.34% | 77.14% | 59.93% | 13.07% |
| Ca Ski | 78.11% | 77.83% | 78.05% | 73.89% | 65.40% | 42.89% | 6.27% | 2.03% |
| C-33 A | 72.30% | 63.93% | 56.72% | 48.29% | 35.49% | 25.45% | 15.93% | 21.53% |
| OVCAR-3 | 84.95% | 83.67% | 80.03% | 74.36% | 61.43% | 42.65% | 16.38% | 17.92% |
| PANC-1 | 52.75% | 48.68% | 42.13% | 36.30% | 31.59% | 25.32% | 12.75% | 21.25% |
| MCF-7 | 61.44% | 57.60% | 54.85% | 50.21% | 45.95% | 36.81% | 14.51% | 6.61% |
| MDA-MB-468 | 74.03% | 70.16% | 65.47% | 64.97% | 64.56% | 55.09% | 30.48% | 4.42% |
| MDA-MB - 231 | 80.43% | 78.85% | 79.86% | 80.07% | 79.28% | 75.62% | 62.84% | 27.58% |
| NCI-N87 | 65.20% | 64.00% | 62.50% | 64.07% | 66.70% | 61.00% | 32.69% | 7.79% |
| BT-549 | 87.45% | 84.28% | 85.57% | 84.62% | 84.33% | 77.48% | 28.03% | 29.31% |
| BxPC-3 | 80.25% | 76.46% | 72.93% | 71.58% | 68.17% | 56.45% | 22.64% | 3.64% |
| HGC-27 | 97.68% | 98.16% | 98.29% | 97.12% | 94.21% | 80.02% | 9.52% | 7.74% |
| DLD-1 | 83.11% | 79.72% | 78.31% | 76.33% | 67.53% | 40.45% | 7.21% | 0.83% |
| SNU-1 | 79.94% | 80.87% | 82.00% | 82.66% | 75.15% | 65.11% | 28.91% | 10.16% |
| U251 | 72.17% | 72.17% | 71.71% | 69.00% | 67.78% | 53.38% | 21.73% | 22.00% |
| KATO III | 72.29% | 74.70% | 74.83% | 67.92% | 51.55% | 15.86% | 2.17% | 1.09% |
| Hep G2 | 81.08% | 85.69% | 87.31% | 87.10% | 79.34% | 66.93% | 14.11% | 1.97% |
| Hep 3B2.1-7 | 88.84% | 90.54% | 89.56% | 90.73% | 90.41% | 90.16% | 90.60% | 77.54% |
| HL-60 | 87.49% | 91.61% | 94.28% | 92.41% | 90.68% | 89.53% | 80.21% | 73.49% |

### Example 83: 22Rv1 mouse xenograft tumor study for compounds C29, C59, and C63

| Species/strain: BALB/c nude mouse | |
|---|---|
| Sex: | Male |
| Body weight: | 19.9-23.6 g |
| Age: | 7-8 weeks |
| Animal grade: | SPF |

Housing conditions: The animals were provided with free access to food (cobalt 60 irradiated diet) and water (SPF purified water). The animals were housed at 6 per cage in IVC systems in a shielded environment. The animals were housed at 20-26 °C, 40-70% relative humidity, and in 12-h light/12-h dark cycles.

Cell culture: 22RV1 (human prostate cancer cell line) culture medium: RPMI1640 (Gibco, Cat. No. C11875500CP); 10% fetal bovine serum (cellmax, Cat. No. SA211.02); 100 U/mL penicillin and 100 µg/mL streptomycin (Hyclone, Cat. No. SV30010); 2 mM L-Glutamine (Gibco, Cat. No. 25030-081). The cells were digested with 0.25% trypsin and passaged at 1:3 once every other day. When the cells were expanded to the required cell count and the cells were in the logarithmic growth phase, the cells were collected, counted, and adjusted to 5 × 10⁷ cells/mL. The cell suspension was mixed with Matrigel (Corning, Cat. No. 354234) in a volume ratio of 1:1, and the mixture was uniformly mixed on ice and then preserved on ice for later use (cell concentration: 2.5 × 10⁷ cells/mL).

Tumor cell grafting: 0.2 mL of cell suspension (5 × 10⁶ 22RV1 cells) was given to each mouse at the right back subcutaneously.

Tumor-bearing mouse selection: 13 days after the grafting of 22RV1 cells, mice with a tumor volume of 80 mm³-150 mm³ were selected and randomized by the tumor volume and body weight into groups of 6 tumor-bearing mice.

Preparation of test compound: DMSO denotes dimethyl sulfoxide, and Captisol denotes sulfobutyl-β-cyclodextrin.

15 mg of C29 was dissolved in 0.375 mL of DMSO solution, before 7.125 mL of 20% Captisol solution was added to give a 2 mg/mL C29 working solution of 7.5 mL.

15 mg of C59 was dissolved in 0.375 mL of DMSO solution, before 7.125 mL of 20% Captisol solution was added to give a 2 mg/mL C59 working solution of 7.5 mL.

7.5 mg of C59 was dissolved in 0.375 mL of DMSO solution, before 7.125 mL of 20% Captisol solution was added to give a 1 mg/mL C59 working solution of 7.5 mL.

15 mg of C63 was dissolved in 0.375 mL of DMSO solution, before 7.125 mL of 20% Captisol solution was added to give a 2 mg/mL C63 working solution of 7.5 mL.

7.5 mg of C63 was dissolved in 0.375 mL of DMSO solution, before 7.125 mL of 20% Captisol solution was added to give a 1 mg/mL C63 working solution of 7.5 mL.

3.75 mg of C63 was dissolved in 0.375 mL of DMSO solution, before 7.125 mL of 20% Captisol solution was added to give a 0.5 mg/mL C63 working solution of 7.5 mL.

Treatment: The compounds were administered by intraperitoneal (IP) injection or oral gavage (PO). The administration frequency was once every day for 18-21 days.

Body weight measurement: The body weight was determined twice every week.

Tumor volume measurement: The tumor volume was measured twice every week, where the long and short diameters of the tumors were measured using a vernier caliper. Tumor volume (TV) = 0.5 × a × b², where a and b denote the tumor's long diameter and short diameter, respectively.

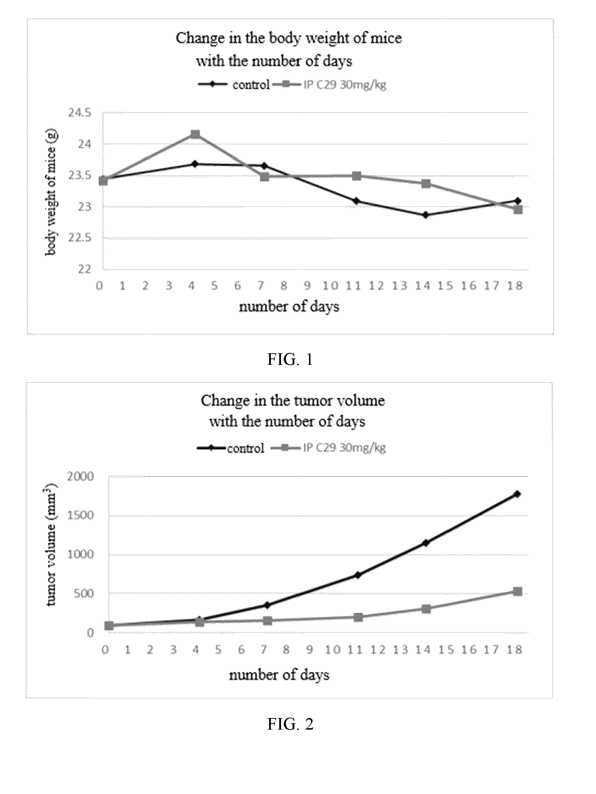

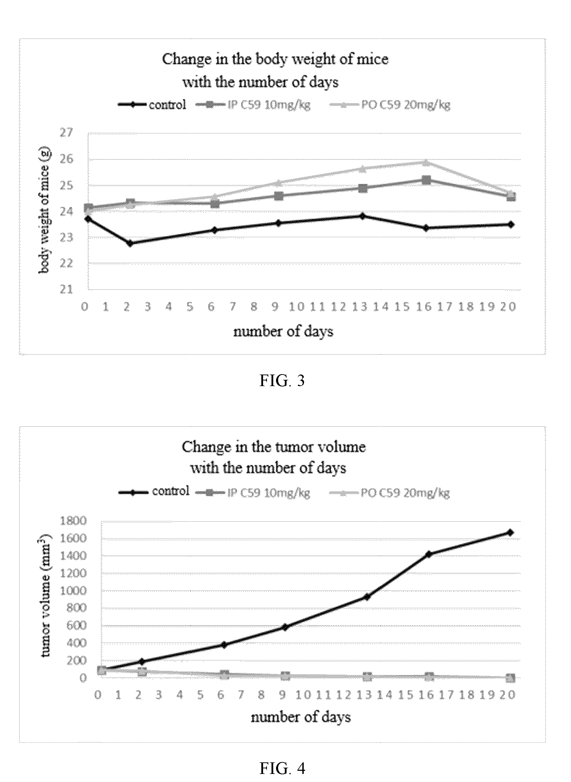

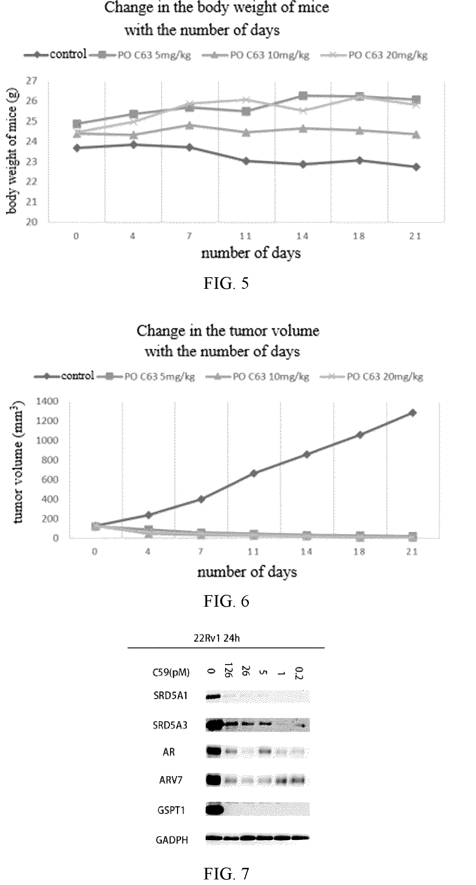

The results are shown below:
1. The body weight and tumor volume of mice receiving C29 intraperitoneal (IP) injection are shown in FIGs. 1 and 2
2. The body weight and tumor volume of mice receiving C59 intraperitoneal (IP) and oral gavage (PO) doses are shown in FIGs. 3 and 4
3. The body weight and tumor volume of mice receiving C63 oral gavage (PO) doses are shown in FIGs. 5 and 6

### Example 84: Western blot assay of compound C59 in 22Rv1, PC-3, and HepG2 cells

(1) Materials: 22Rv1, PC-3, and HepG2 cell, anti-SRD5A1 antibody (ab167606), anti-SRD5A2 antibody (ab124877), anti-SRD5A3 antibody (PA5-107029), anti-androgen receptor (AR) antibody (CST, No. 5153), anti-GSPT1 antibody (Abcam, ab234433), anti-GADPH antibody (CST, No. 5174), anti-c-Myc antibody (CST, No. 8583), anti-α-tublin antibody (CST, No. 2125), anti-rabbit IgG, HRP-linked antibody (CST, No. 7074), electrophoresis device and membrane transfer device (LIUYI, BEIJING; DYCZ-24DN)
(2) Procedures:
   Cell seeding: 22Rv1 (Cell Bank of the Chinese Academy of Sciences, TCHu100), PC-3 (Cell Bank of the Chinese Academy of Sciences, SCSP-532) and HepG2 cells (Cell Bank of the Chinese Academy of Sciences, TCHu72) were subcultured and trypsinized to prepare 6 × 10⁵ cells/mL suspensions, seeded on 6-well plates (3 mL/well), and cultured overnight in a cell incubator at 5% CO₂ and 37 °C.

Addition of compounds and post-treatment: The compounds at different concentrations were added, and the cells were cultured for 24 h. After the cells were washed twice with icy PBS buffer, 800 µL of RIPA lysis buffer was added to each well, and the cells were lysed on ice for 30 min. The lysate was collected in a centrifuge tube and centrifuged at 12000 rpm for 10 min at 4 °C. The supernatant was transferred to a new centrifuge tube.

Protein quantification: The protein was quantified using a BCA protein quantification kit. The proteins were adjusted to the same concentration and added to a sample loading buffer. The mixture was mixed, and the proteins were denatured at 100 °C for 5 min and preserved at -80 °C or directly used in Western-Blot assay.

Western immunoblot assay (or Western blot, WB): SDS-PAGE gel at a proper concentration was prepared according to the molecular weight of the target protein: for example, the concentration of the separation gel was 8% and the concentration of the stacking gel was 5%. The loading volume was adjusted according to the protein quantification results. The voltage for the protein sample was 90 V in the stacking gel, and was adjusted to 130 V after the protein sample entered the separation gel. The electrophoresis was stopped when the bromophenol blue completely ran out of the PAGE gel. After a membrane transfer sandwich (wet transfer) was made, the membrane transfer device was placed in an ice-water bath, and the membrane transfer was conducted for 1.2 h at a constant current of 180 mA. The PVDF membrane was taken out and immersed in a TBST solution containing 5% of skim milk for blocking on a shaker at room temperature for 1 h. The blocked PVDF membrane was washed 5 times with a TBST buffer, and a primary antibody diluted in a suitable ratio was added. The membrane was incubated overnight at 4 °C and then washed 5 times with a TBST buffer for 6 min each time. The secondary antibody diluted in a certain factor was added, and the membrane was incubated at room temperature for 2 h and washed 5 times with a TBST buffer for 6 min each time. The membrane was exposed on a gel imaging system.

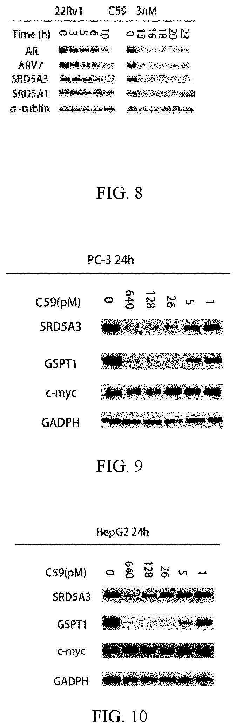

The results are shown in the following:
1. The downregulation of SRD5A1, SRD5A3, AR, ARV7, GSPT1 proteins in 22Rv1 cells by C59 are shown in FIGs. 7 and 8.
2. The downregulation of SRD5A3 and GSPT1 (eRF3a) proteins in PC-3 and HepG2 cells by C59 are shown in FIGs. 9 and 10.

## Claims

1. A compound having a structure of: TPB-L-E3B,
wherein TPB is a target protein-binding moiety, L is a linker, and E3B is an E3 ligase-binding moiety,
TPB is shown in a structure (I), 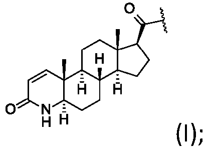
L is selected from a structure (IIA) or (IIB), 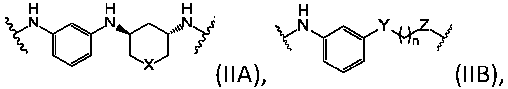
X is CH₂, O, NH, or CF₂, n is an integer of 2-3, Y is NH or O, and Z is NH or O;
E3B is selected from a structure (IIIA), (IIIB), or (IIIC), 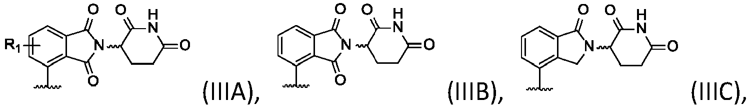 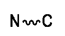
denotes that C may have R configuration, S configuration, or a mixture of R and S configurations, and R₁ denotes that the H at any one of three positions of the benzene ring is substituted by F.

2. The compound according to claim 1, wherein L is selected from a structure (IIA) or (IIB), 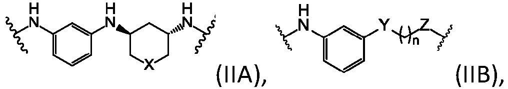
X is CH₂, O, NH, or CF₂, n is an integer of 2-3, Y is NH or O, and Z is NH;
E3B is selected from a structure (IIIB) or (IIIC), 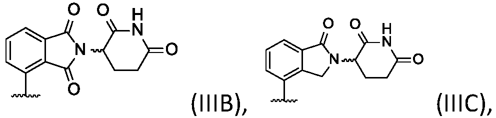
and 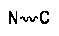 denotes that C may have R configuration, S configuration, or a mixture of R and S configurations.

3. The compound according to claim 2, wherein L is selected from a structure (IIA) or (IIB), 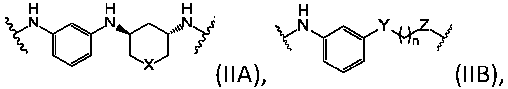
X is CH₂, n is 3, Y is NH, and Z is NH;
E3B is shown in a structure (IIIB), 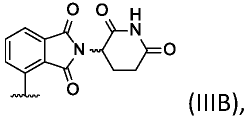
and 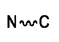 denotes that C may be have R configuration, S configuration, or a mixture of R and S configurations.

4. The compound according to claim 3, wherein the compound is selected from a structure of: 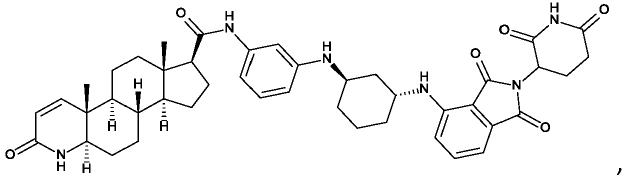 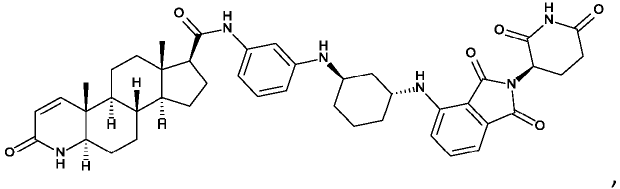 or

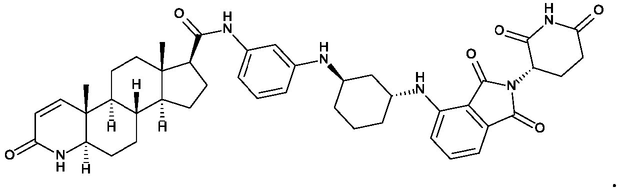

5. Use of any one or more of the compounds according to claims 1-4 in preparing a medicament for treating a human tumor disease.

6. The use according to claim 5, wherein the human tumor disease is prostate cancer, ovarian cancer, liver cancer, breast cancer, pancreatic cancer, cerebral glioma, cervical cancer, leukemia, gastric cancer, or colon cancer.

7. The use according to claim 6, wherein the human tumor disease is prostate cancer.
